# EUROPEAN PATENT SPECIFICATION

(11) **EP 4 442 093 B1**
(45) Date of publication and mention of the grant of the patent: **25.11.2026**
(21) Application number: 22823452.2
(22) Date of filing: 30.11.2022
(51) Int. Cl.: C07D 239/26, C07D 251/24, H10K 85/60, H10K 50/18

(54) **COMPOUNDS HAVING FLUORENE STRUCTURES**
VERBINDUNGEN MIT FLUORENSTRUKTUREN
COMPOSÉS AYANT DES STRUCTURES DE FLUORÈNE

(30) Priority: 30.11.2021 EP 21211490
(43) Date of publication of application: 09.10.2024
(73) Proprietor: Merck Patent GmbH, 64293 Darmstadt (DE)
(72) Inventor: GANSS, Stephanie Marie, 64293 Darmstadt (DE)
(74) Representative: Merck Patent Association
(86) International application number: PCT/EP2022/083807
(87) International publication number: WO 2023/099543

(56) References cited:
- EP-A1- 3 127 901
- EP-A1- 3 127 988

## Description

The present invention describes fluorene derivatives substituted by electron-transporting groups, especially for use in electronic devices. The invention further relates to a process for preparing the compounds of the invention and to electronic devices comprising these compounds.

The structure of organic electroluminescent devices (OLEDs) in which organic semiconductors are used as functional materials is described, for example, in US 4539507, US 5151629, EP 0676461 and WO 98/27136. Emitting materials used are frequently organometallic complexes which exhibit phosphorescence. For quantum-mechanical reasons, up to four times the energy efficiency and power efficiency is possible using organometallic compounds as phosphorescent emitters. In general terms, there is still a need for improvement in OLEDs, especially also in OLEDs which exhibit phosphorescence, for example with regard to efficiency, operating voltage and lifetime.

The properties of phosphorescent OLEDs are not just determined by the triplet emitters used. More particularly, the other materials used, for example matrix materials, are also of particular significance here. Improvements to these materials can thus also lead to distinct improvements in the OLED properties.

Frequently used according to the prior art as matrix materials for phosphorescent compounds and as electron transport materials are heteroaromatic compounds, for example triazine derivatives or benzimidazole derivatives. Suitable matrix materials for phosphorescent compounds are also carbazole derivatives. Known derivatives for this function are, for example, spirobifluorene derivatives substituted in the 2 position by triazine groups, as disclosed in WO 2010/015306 and WO 2010/072300. In the case of these compounds, there is still need for improvement both in the case of fluorescent and in the case of phosphorescent OLEDs, especially with regard to efficiency, lifetime and operating voltage on use in an organic electroluminescent device.

Moreover, JP 2014183315A discloses heterocyclic compounds having fluorene structures. Similar compounds are additionally known from EP 2842954 A1, EP3127988 A1, and EP3127901 A1.

In general terms, in the case of these materials, e. g. for use as matrix materials or as electron-transport materials, there is still need for improvement, particularly in relation to the efficiency, operating voltage of the device, lower refractive index (RI) and increased external quantum efficiency (EQE), but also in relation to lifetime.

It is an object of the present invention to provide compounds suitable for use in an electronic device, especially an organic electroluminescent device, preferably a phosphorescent or fluorescent OLED, especially as matrix material and/or as electron-transport material. More particularly, it is an object of the present invention to provide matrix materials which are suitable for red-, yellow- and green-phosphorescing OLEDs and possibly also for blue-phosphorescing OLEDs, and which lead to long lifetime, good efficiency, low operating voltage, lower refractive index (RI) and increased external quantum efficiency (EQE). Particularly the properties of the electron-transport materials and of the matrix materials, too, have an essential influence on the lifetime and efficiency of the organic electroluminescent device

Moreover, the compounds should be processible in a very simple manner, and especially exhibit good solubility and film formation. For example, the compounds should exhibit elevated oxidation stability and an improved glass transition temperature.

A further object can be considered that of providing electronic devices having excellent performance very inexpensively and in constant quality.

Furthermore, it should be possible to use or adapt the electronic devices for many purposes. More particularly, the performance of the electronic devices should be maintained over a broad temperature range.

It has been found that, surprisingly, devices containing compounds comprising structures of the following Formula (A), preferably Formula (I) and/or Formula (II) have improvements over the prior art, especially when used as hole blocking material, as electron injection material and/or as electron transport material.

The present invention therefore provides a compound comprising structures of the Formula (A), preferably compound according to Formula (A), 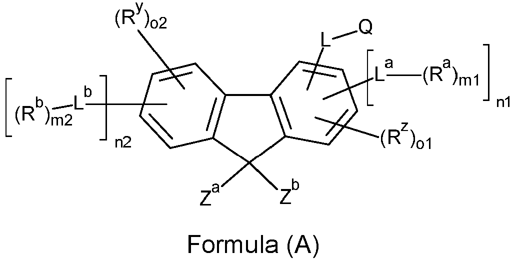 where the symbols used are as follows:
- Z^{a}: is Ar^{a} or R^{x}, wherein Ar^{a} is an aromatic or heteroaromatic ring system which has 5 to 40 aromatic ring atoms, each of which may be substituted by one or more R^{x} radicals, wherein the groups Z^{a} and Z^{b} may form a mono- or polycyclic aliphatic, heteroaliphatic, aromatic or heteroaromatic ring system with one another;
- Z^{b}: is Ar^{b} or R^{w}, wherein Ar^{b} is an aromatic or heteroaromatic ring system which has 5 to 40 aromatic ring atoms, each of which may be substituted by one or more R^{w} radicals, wherein the groups Z^{a} and Z^{b} may form a mono- or polycyclic aliphatic, heteroaliphatic, aromatic or heteroaromatic ring system with one another;
- Q: is an electron transport group;
- L, L^{a}, L^{b}: is the same or different at each instance and is a single bond, C(=O) or an aromatic or heteroaromatic ring system having 5 to 24 aromatic ring atoms, which may be substituted by one or more radicals R;
- R, R^{w}, R^{x}, R^{y}, R^{z}: is the same or different at each instance and is H, D, F, Cl, Br, I, CN, Si(R¹)₃, a straight-chain alkyl, alkoxy or thioalkyl group having 1 to 40 C atoms or a branched or cyclic alkyl, alkoxy or thio-alkyl¬ group having 3 to 40 C atoms, each of which may be substituted by one or more radi¬cals R¹, where in each case one or more non-adjacent CH₂ groups may be replaced by -R¹C=CR¹-, - C≡C-, Si(R')₂, C=O, C=S, C=NR¹, -C(=O)O-, -C(=O)NR¹-, NR¹, P(=O)(R¹), -O-, -S-, SO or SO₂ and where one or more H atoms may be replaced by D, F, Cl, Br, I, CN or NO₂, or an aromatic or heteroaromatic ring system having 5 to 40 aromatic ring atoms, which may in each case be substituted by one or more radicals R¹, or an aryloxy or heteroaryloxy group having 5 to 40 aromatic ring atoms, which may be substituted by one or more radicals R¹, or an aralkyl group having 5 to 40 aromatic ring atoms, which may in each case be substituted by one or more radicals R¹, or a combination of these systems; at the same time two or more adjacent substituents R, R^{w}, R^{x}, R^{y}, R^{z} may also form a ring system, preferably a mono- or polycyclic, aliphatic or aromatic ring system with one another, which may be substituted by one or more radicals R¹;
- R¹: is the same or different at each instance and is H, D, a straight-chain alkyl, alkoxy or thioalkoxy group having 1 to 40 carbon atoms or a branched or cyclic alkyl, alkoxy or thioalkoxy group having 3 to 40 carbon atoms, each of which may be substituted by one or more R² radicals, where one or more non-adjacent CH₂ groups may be replaced by -R²C=CR²-, -C≡C-, Si(R²)₂, C=O, C=S, C=NR², - C(=O)O-, -C(=O)NR²-, NR², P(=O)(R²), -O-, -S-, SO or SO₂ and where one or more hydrogen atoms may be replaced by D, F, Cl, Br, I, CN or NO₂, or an aromatic or heteroaromatic ring system which has 5 to 40 aromatic ring atoms, each of which may be substituted by one or more R² radicals, or an aryloxy or heteroaryloxy group which has 5 to 40 aromatic ring atoms, each of which may be substituted by one or more R² radicals, or a combination of these systems; at the same time, two or more adjacent R¹ substituents may also form a ring system, preferably a mono- or polycyclic aliphatic or aromatic ring system with one another;
- R²: is the same or different at each instance and is H, D, F or an aliphatic hydrocarbyl radical having 1 to 20 carbon atoms, in which one or more hydrogen atoms may be replaced by D or F, or an aromatic or heteroaromatic ring system having 5 to 30 carbon atoms, in which one or more hydrogen atoms may be replaced by D or F; at the same time, two or more adjacent R² substituents together may also form a ring system, preferably a mono- or polycyclic, aliphatic or aromatic ring system;
- R^{a}, R^{b}: is the same or different at each instance and selected from (R^{a}-1) to (R²-33): 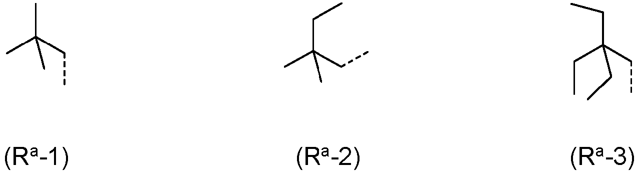 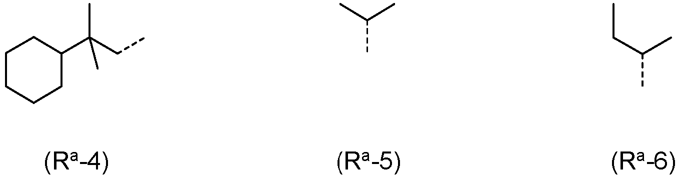 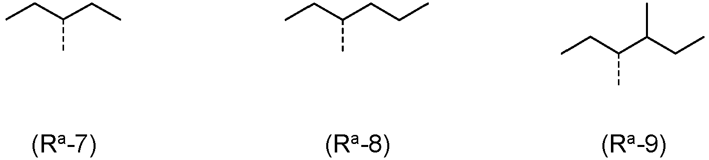 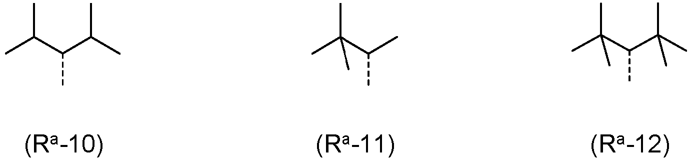 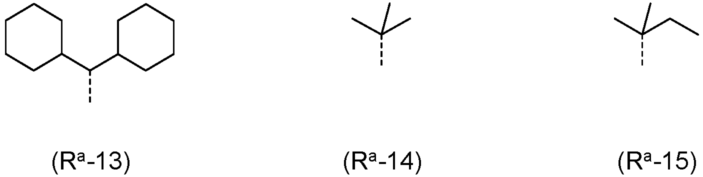 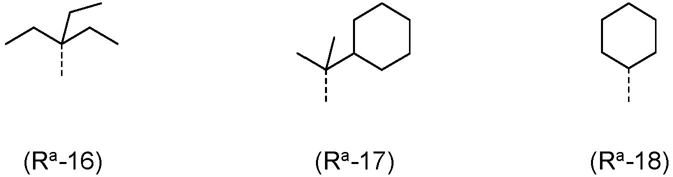 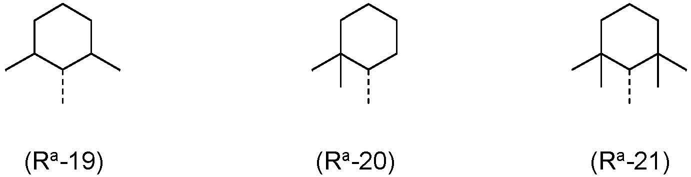 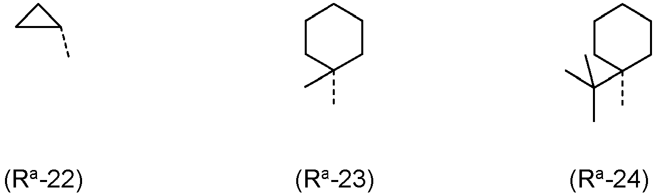 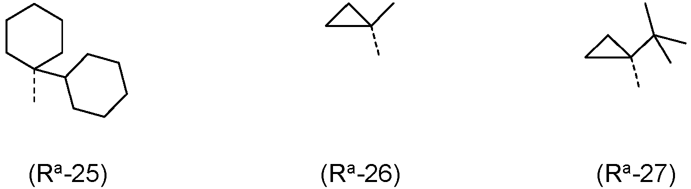 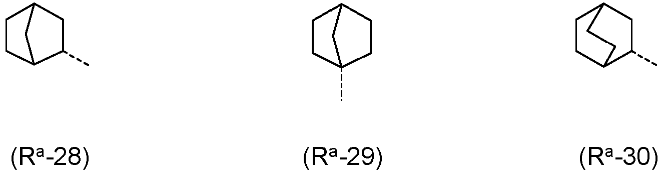 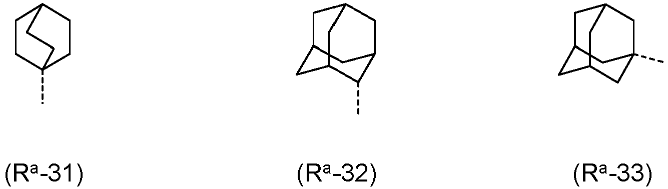 wherein one or more H atoms may be replaced by D and the dotted line denotes the bond to the corresponding group;
- m1, m2: is the same or different at each instance and is 0, 1, 2, 3 or 4, preferably 1, 2, 3 or 4, more preferably 1 or 2;
- n2, o2: is the same or different at each instance and is 0, 1, 2, 3 or 4, preferably 0, 1 or 2;
- n1, o1: is the same or different at each instance and is 0, 1, 2 or 3, preferably 0, 1 or 2;

the sum of n1 + o1 is 0, 1, 2 or 3, preferably 0, 1 or 2;
the sum of n2 + o2 is 0, 1, 2, 3 or 4, preferably 0, 1 or 2; and
wherein the compound comprises two, three or four, structure element of formulae (R^{a}-1) to (R^{a}-33).

Ar^{a}, Ar^{b} is the same or different at each instance and is an aromatic or heteroaromatic ring system having 5 to 40 aromatic ring atoms, preferably 6 to 18 aromatic ring atoms, and is more preferably an aromatic ring system having 6 to 12 aromatic ring atoms or a heteroaromatic ring system having 5 to 12 aromatic ring atoms, each of which may be substituted by one or more R^{w}, R^{x} radicals, respectively, where R^{w}, R^{x} may have the definition given above, especially in Formulae (A). Examples of suitable Ar^{a}, Ar^{b} groups are selected from the group consisting of phenyl, ortho-, meta- or para-biphenyl, terphenyl, especially branched terphenyl, quaterphenyl, especially branched quaterphenyl, 1-, 2-, 3- or 4-fluorenyl, 1-, 2-, 3- or 4-spirobifluorenyl, pyridyl, pyrimidinyl, 1-, 2-, 3- or 4-dibenzofuranyl, 1-, 2-, 3- or 4-dibenzothienyl and 1-, 2-, 3- or 4-carbazolyl, each of which may be substituted by one or more R^{w}, R^{x} radicals.

Preferably, the symbol Ar^{a}, Ar^{b} represents an aryl or heteroaryl radical, such that an aromatic or heteroaromatic group of an aromatic or heteroaromatic ring system is bonded directly, i.e. via an atom of the aromatic or heteroaromatic group, to the respective atom of the further group, for example the carbon, nitrogen or phosphorus atom of the R^{w}, R^{x} groups.

Preferably, the present invention therefore provides a compound comprising structures of the Formula (I) or Formula (II), more preferably a compound according to Formula (I) or Formula (II), 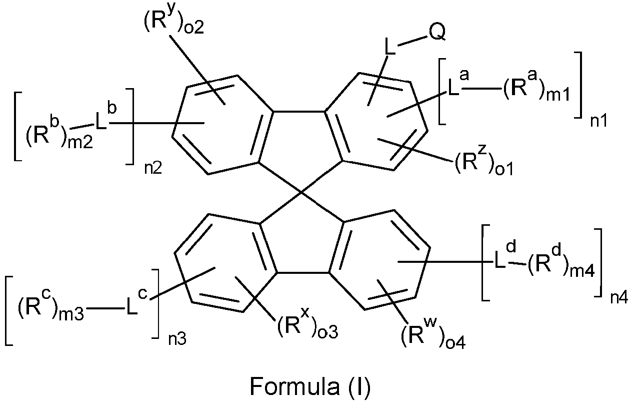 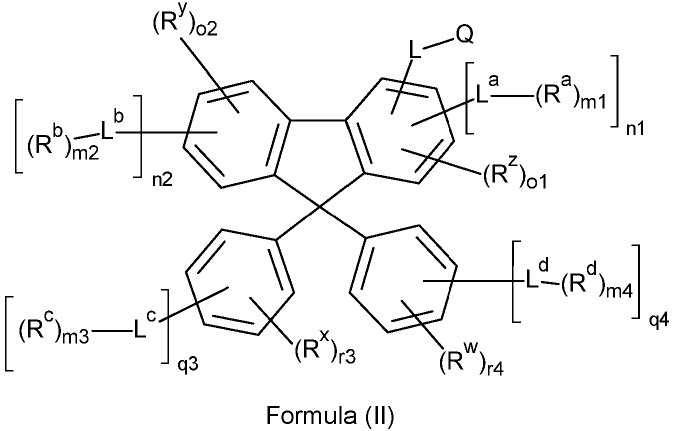 in which
the symbols Q, L, L^{a}, L^{b}, R, R^{w}, R^{x}, R^{y}, R^{z}, R^{a}, R^{b}, m1, m2, n1, n2, o1 and o2 have the definition given above, especially for Formula (A) and
L^{c}, L^{d} is the same or different at each instance and is a single bond, C(=O) or an aromatic or heteroaromatic ring system having 5 to 24 aromatic ring atoms, which may be substituted by one or more radicals R;
R^{c}, R^{d} is the same or different at each instance and selected from (R^{a}-1) to (R^{a}-33) as given above, especially for Formula (A);
m3, m4 is the same or different at each instance and is 0, 1, 2, 3 or 4, preferably 1, 2, 3 or 4, more preferably 1 or 2;
n3, n4, o3, o4 is the same or different at each instance and is 0, 1, 2, 3 or 4, preferably 0, 1 or 2;
q3, q4, r3, r4 is the same or different at each instance and is 0, 1, 2, 3, 4 or 5, preferably 0, 1 or 2;
the sum of n3 + o3 is 0, 1, 2, 3 or 4, preferably, 0, 1 or 2;
the sum of n4 + o4 is 0, 1, 2, 3 or 4, preferably, 0, 1 or 2;
the sum of q3 + r3 is 0, 1, 2, 3, 4 or 5, preferably, 0, 1 or 2;
the sum of q4 + r4 is 0, 1, 2, 3, 4 or 5, preferably, 0, 1 or 2; and
wherein the compound comprises two, three or four, structure element of formulae (R^{a}-1) to (R^{a}-33).

The indices m1, m2, m3, m4 may be 0. In this case the corresponding residues R^{a}, R^{b}, R^{c}, R^{d} are H or D, preferably H.

Adjacent carbon atoms in the context of the present invention are carbon atoms bonded directly to one another. In addition, "adjacent radicals" in the definition of the radicals means that these radicals are bonded to the same carbon atom or to adjacent carbon atoms. These definitions apply correspondingly, inter alia, to the terms "adjacent groups" and "adjacent substituents".

The wording that two or more radicals together may form a ring, in the context of the present description, shall be understood to mean, inter alia, that the two radicals are joined to one another by a chemical bond with formal elimination of two hydrogen atoms. This is illustrated by the following scheme:

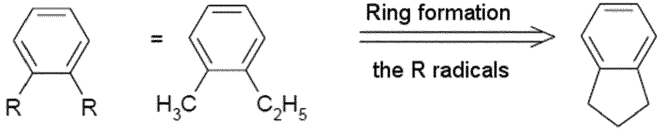

In addition, however, the abovementioned wording shall also be understood to mean that, if one of the two radicals is hydrogen, the second radical binds to the position to which the hydrogen atom was bonded, forming a ring. This shall be illustrated by the following scheme:

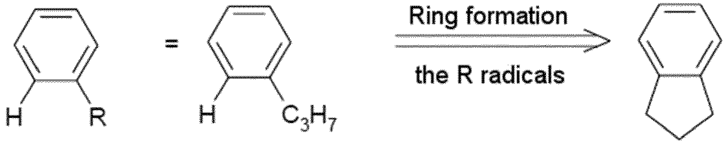

A fused aryl group in the context of the present invention is a group in which two or more aromatic groups are fused, i.e. annealed, to one another along a common edge, such that, for example, two carbon atoms belong to the at least two aromatic or heteroaromatic rings, as, for example, in naphthalene. By contrast, for example, fluorene is not a fused aryl group in the context of the present invention, since the two aromatic groups in fluorene do not have a common edge.

An aryl group in the context of this invention contains 6 to 40 carbon atoms, preferably 6 to 24 C atoms; a heteroaryl group in the context of this invention contains 2 to 40 carbon atoms, preferably 2 to 24 C atoms and at least one heteroatom, with the proviso that the total sum of carbon atoms and heteroatoms is at least 5. The heteroatoms are preferably selected from N, O and/or S. An aryl group or heteroaryl group is understood here to mean either a simple aromatic cycle, i.e. benzene, or a simple heteroaromatic cycle, for example pyridine, pyrimidine, thiophene, etc., or a fused aryl or heteroaryl group, for example naphthalene, anthracene, phenanthrene, quinoline, isoquinoline, etc.

An aromatic ring system in the context of this invention contains 6 to 40 carbon atoms in the ring system. A heteroaromatic ring system in the context of this invention contains 1 to 40 carbon atoms and at least one heteroatom in the ring system, with the proviso that the total sum of carbon atoms and heteroatoms is at least 5. The heteroatoms are preferably selected from N, O and/or S. An aromatic or heteroaromatic ring system in the context of this invention shall be understood to mean a system which does not necessarily contain only aryl or heteroaryl groups, but in which it is also possible for two or more aryl or heteroaryl groups to be interrupted by a nonaromatic unit (preferably less than 10% of the atoms other than H), for example a carbon, nitrogen or oxygen atom or a carbonyl group. For example, systems such as 9,9'-spirobifluorene, 9,9-diarylfluorene, triarylamine, diaryl ethers, stilbene, etc. shall also be regarded as aromatic ring systems in the context of this invention, and likewise systems in which two or more aryl groups are interrupted, for example, by a linear or cyclic alkyl group or by a silyl group. In addition, systems in which two or more aryl or heteroaryl groups are bonded directly to one another, for example biphenyl, terphenyl, quaterphenyl or bipyridine, shall likewise be regarded as an aromatic or heteroaromatic ring system.

A cyclic alkyl, alkoxy or thioalkoxy group in the context of this invention is understood to mean a monocyclic, bicyclic or polycyclic group.

In the context of the present invention, a C₁- to C₂₀-alkyl group in which individual hydrogen atoms or CH₂ groups may also be substituted by the abovementioned groups is understood to mean, for example, the methyl, ethyl, n-propyl, i-propyl, cyclopropyl, n-butyl, i-butyl, s-butyl, t-butyl, cyclobutyl, 2-methylbutyl, n-pentyl, s-pentyl, t-pentyl, 2-pentyl, neopentyl, cyclopentyl, n-hexyl, s-hexyl, t-hexyl, 2-hexyl, 3-hexyl, neohexyl, cyclohexyl, 1-methylcyclopentyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2.2.2]octyl, 2-bicyclo[2.2.2]octyl, 2-(2,6-dimethyl)octyl, 3-(3,7-dimethyl)octyl, adamantyl, trifluoromethyl, pentafluoroethyl, 2,2,2-trifluoroethyl, 1,1-dimethyl-n-hex-1-yl, 1,1-dimethyl-n-hept-1-yl, 1,1-dimethyl-n-oct-1-yl, 1,1-dimethyl-n-dec-1-yl, 1,1-dimethyl-n-dodec-1-yl, 1,1-dimethyl-n-tetradec-1-yl, 1,1-dimethyl-n-hexadec-1-yl, 1,1-dimethyl-n-octadec-1-yl, 1,1-diethyl-n-hex-1-yl, 1,1-diethyl-n-hept-1-yl, 1,1-diethyl-n-oct-1-yl, 1,1-diethyl-n-dec-1-yl, 1,1-diethyl-n-dodec-1-yl, 1,1-diethyl-n-tetradec-1-yl, 1,1-diethyl-n-hexadec-1-yl, 1,1-diethyl-n-octadec-1-yl, 1-(n-propyl)cyclohex-1-yl, 1-(n-butyl)cyclohex-1-yl, 1-(n-hexyl)cyclohex-1-yl, 1-(n-octyl)cyclohex-1-yl and 1-(n-decyl)cyclohex-1-yl radicals. An alkenyl group is understood to mean, for example, ethenyl, propenyl, butenyl, pentenyl, cyclopentenyl, hexenyl, cyclohexenyl, heptenyl, cycloheptenyl, octenyl, cyclooctenyl or cyclooctadienyl. An alkynyl group is understood to mean, for example, ethynyl, propynyl, butynyl, pentynyl, hexynyl, heptynyl or octynyl. A C₁- to C₄₀-alkoxy group is understood to mean, for example, methoxy, trifluoromethoxy, ethoxy, n-propoxy, i-propoxy, n-butoxy, i-butoxy, s-butoxy, t-butoxy or 2-methylbutoxy.

An aromatic or heteroaromatic ring system which has 5-40 aromatic ring atoms and may also be substituted in each case by the abovementioned radicals and which may be joined to the aromatic or heteroaromatic system via any desired positions is understood to mean, for example, groups derived from benzene, naphthalene, anthracene, benzanthracene, phenanthrene, benzophenanthrene, pyrene, chrysene, perylene, fluoranthene, benzofluoranthene, naphthacene, pentacene, benzopyrene, biphenyl, biphenylene, terphenyl, terphenylene, fluorene, spirobifluorene, dihydrophenanthrene, dihydropyrene, tetrahydropyrene, cis- or trans-indenofluorene, cis- or trans-monobenzoindenofluorene, cis- or trans-dibenzoindenofluorene, truxene, isotruxene, spirotruxene, spiroisotruxene, furan, benzofuran, isobenzofuran, dibenzofuran, thiophene, benzothiophene, isobenzothiophene, dibenzothiophene, pyrrole, indole, isoindole, carbazole, indolocarbazole, indenocarbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

In a preferred embodiment, the compounds of the invention comprise at least one of the structures of the Formulae (Ia), (Ib), (Ic), (Id), (IIa), (IIb), (IIc) and/or (IId), preferably the compounds of the invention are a compound according to Formulae (Ia), (Ib), (Ic), (Id), (IIa), (IIb), (IIc) and/or (IId), 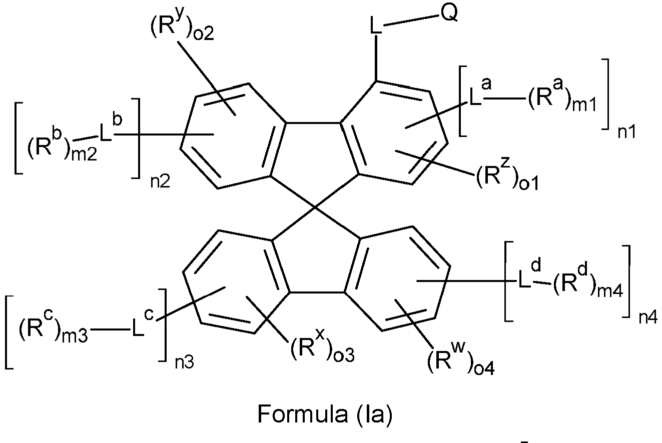 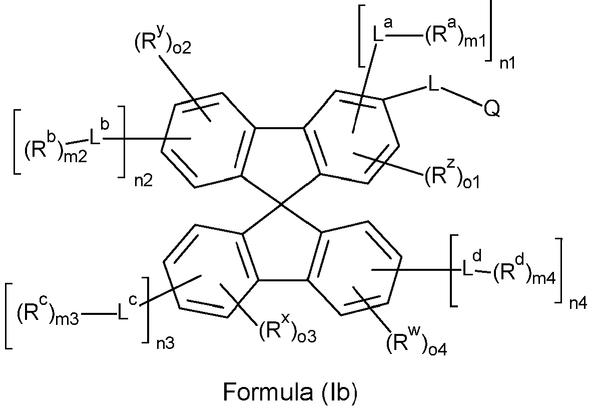 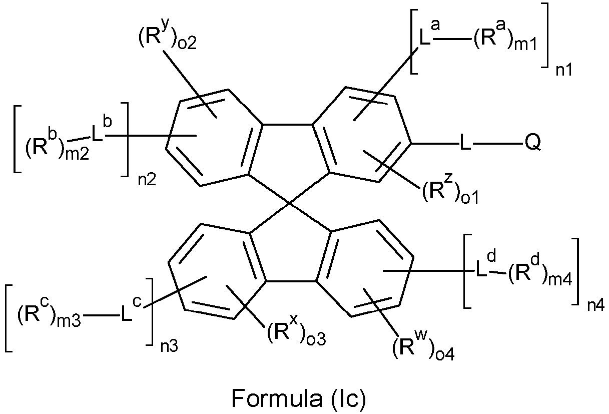 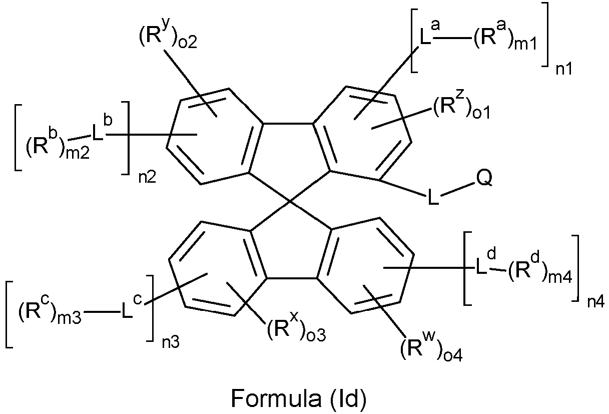 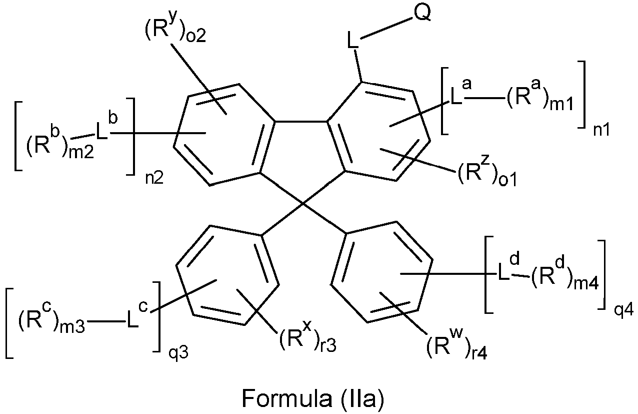 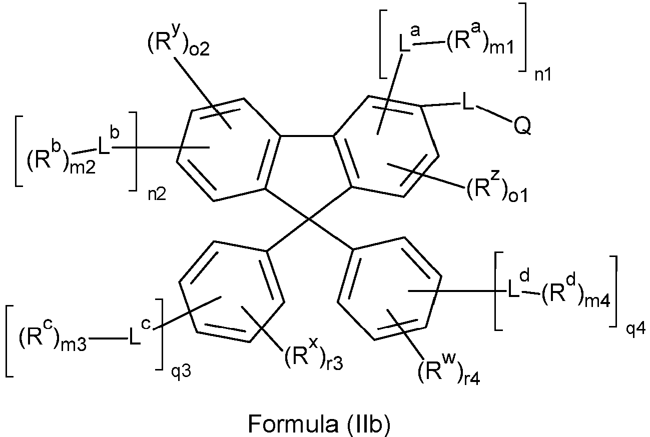 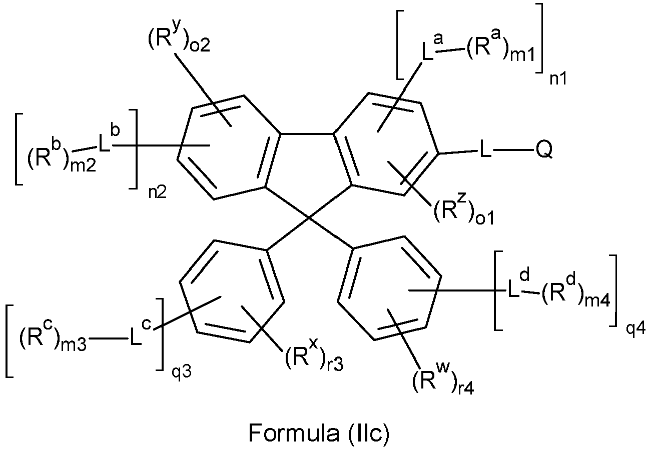 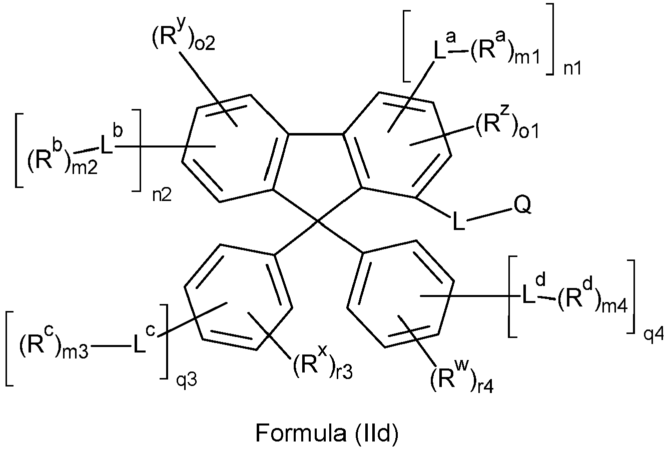 in which the symbols Q, L, L^{a}, L^{b}, L^{c}, L^{d}, R^{w}, R^{x}, R^{y}, R^{z}, R^{a}, R^{b}, R^{c}, R^{d}, m1, m2, m3, m4, n1, n2, n3, n4, o1, o2, o3, o4, q3, q4, r3 and r4 have the definition set out above, especially for Formulae (A), (I) and/or (II). The Structures (Ia) and (Ic) are especially preferred.

Furthermore, it can be provided, preferably in Formulae (A), (I), (II), (Ia) to (Id) and/or (IIa) to (IId), that the sum of the indices n1, n2, n3, n4, o1, o2, o3, o4, q3, q4, r3 and r4 is in the range of 1 to 8, preferably 1 to 6, more preferably 2 to 4.

Additionally, it can be provided, preferably in Formulae (A), (I), (II), (Ia) to (Id) and/or (IIa) to (IId), that the sum of the indices m1, m2, m3 and m4 is in the range of 1 to 6, preferably 1 to 4, more preferably 2 or 3.

In the case that a ring system is formed, e. g. by the radicals R, R^{w}, R^{x}, R^{y}, R^{z}, the ring system could be a mono- or polycyclic, aliphatic, heteroaliphatic, aromatic or heteroaromatic ring system, preferably a mono- or polycyclic, aliphatic or aromatic ring system.

It may further be the case that the radicals R, R^{w}, R^{x}, R^{y}, R^{z} do not form a fused ring system with the ring atoms of the fluorene structure. This includes the formation of a fused ring system with possible R¹, R² substituents which may be bonded to the radicals R, R^{w}, R^{x}, R^{y}, R^{z}. It may preferably be the case that the radicals R, R^{w}, R^{x}, R^{y}, R^{z} do not form a ring system with the ring atoms of the fluorene structure. This includes the formation of a ring system with possible R¹, R² substituents which may be bonded to the radicals R, R^{w}, R^{x}, R^{y}, R^{z}.

In a further preferred embodiment, the compounds of the invention comprise at least one of the structures of the Formulae (1-1), (I-2), (I-3), (I-4), (I-5), (I-6), (II-1), (II-2), (II-3), (II-4), (II-5) and/or (II-6), preferably the compounds of the invention are a compound according to Formulae (I-1), (I-2), (I-3), (I-4), (I-5), (I-6), (II-1), (II-2), (II-3), (II-4), (II-5) and/or (II-6), 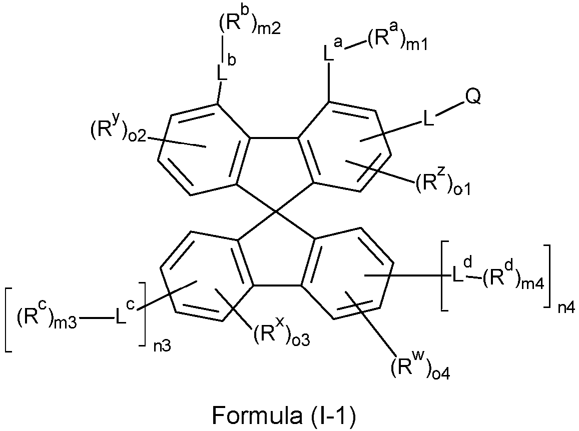 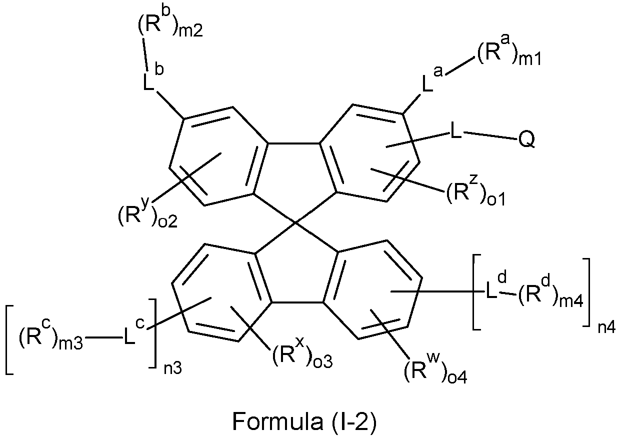 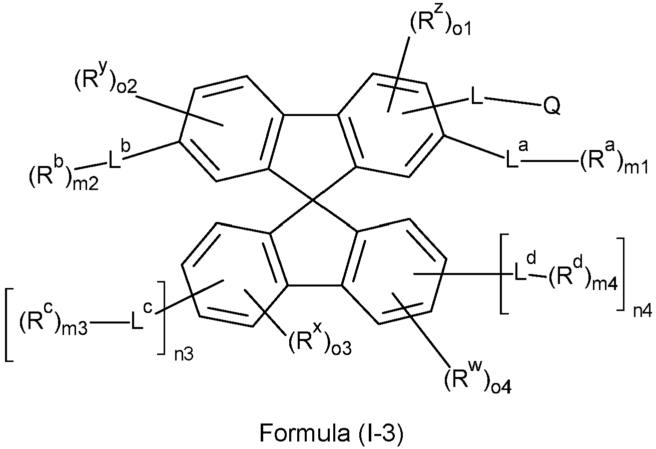 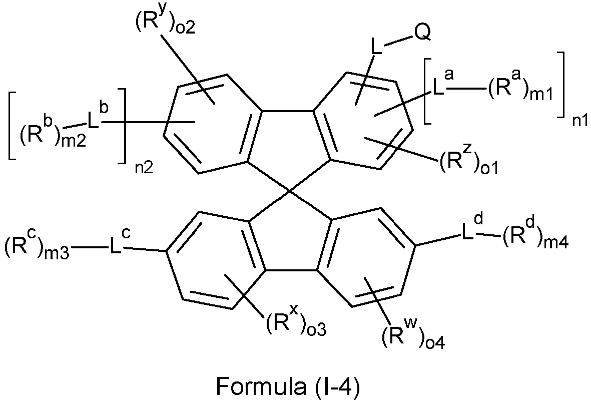 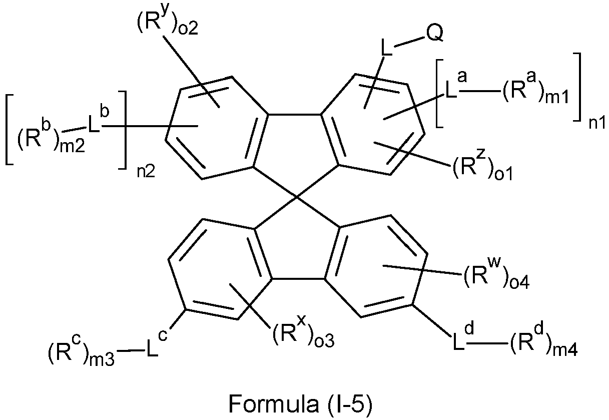 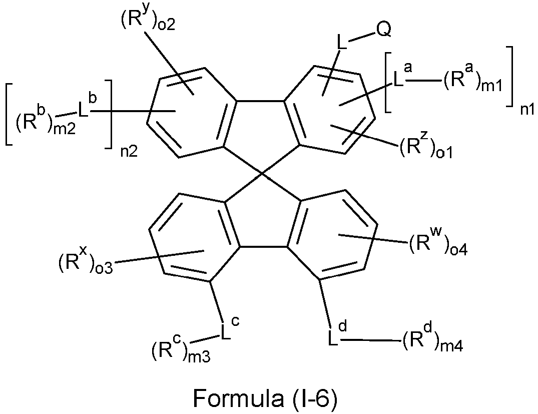 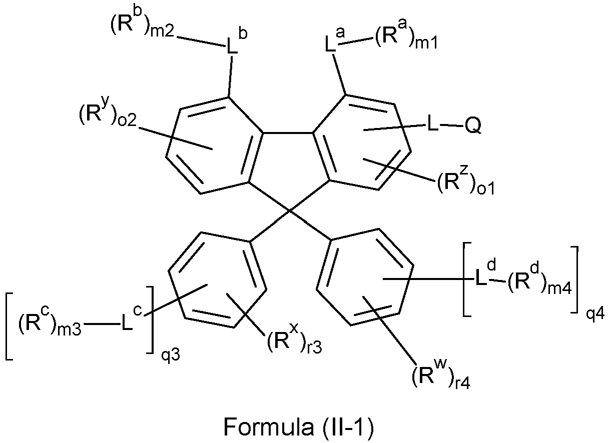 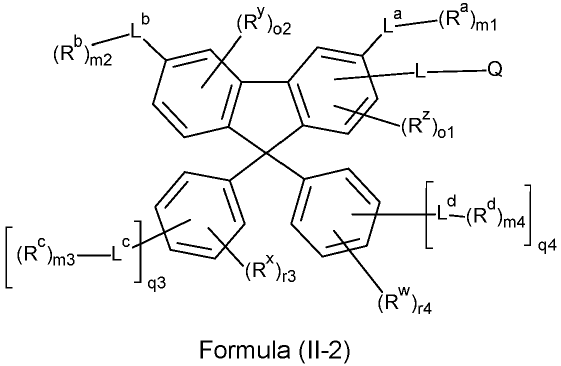 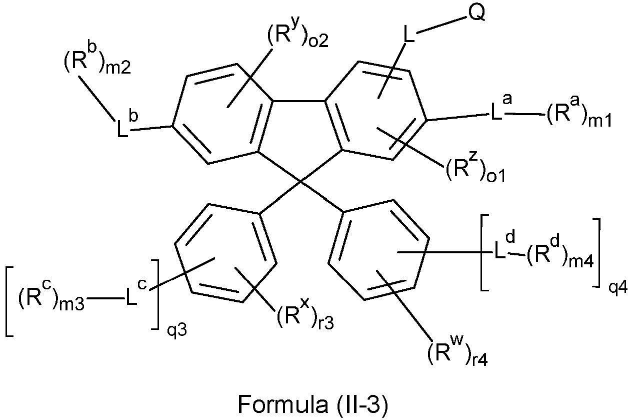 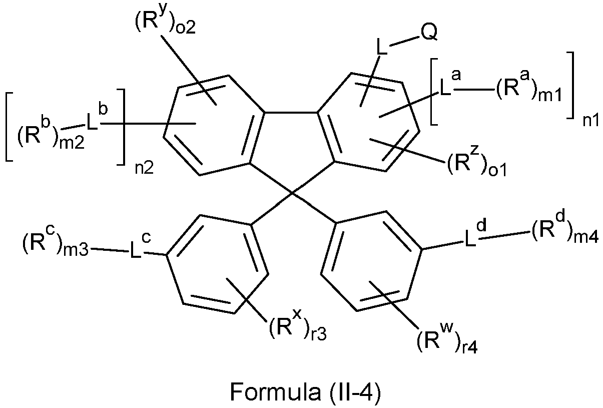 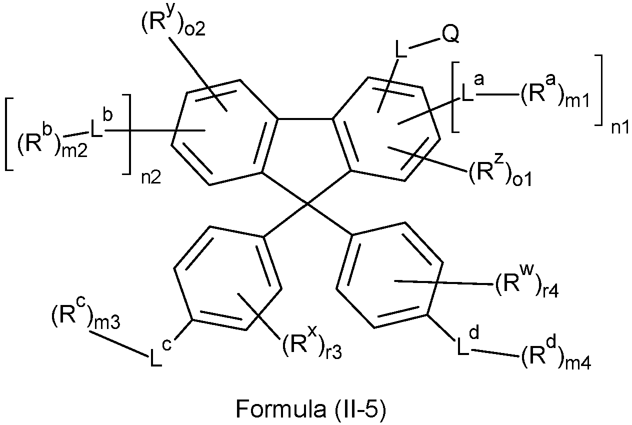 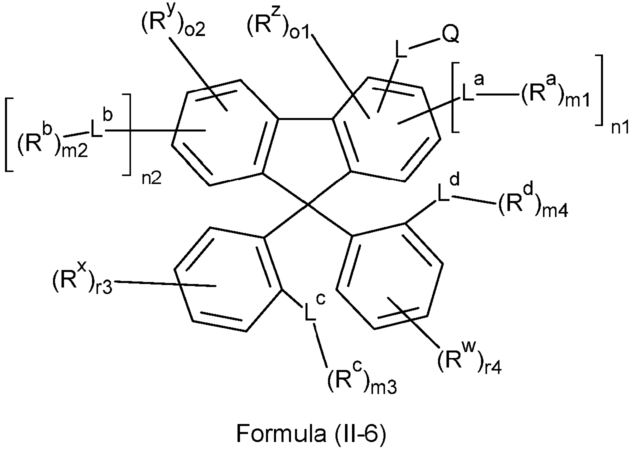 in which the symbols Q, L, L^{a}, L^{b}, L^{c}, L^{d}, R^{w}, R^{x}, R^{y}, R^{z}, R^{a}, R^{b}, R^{c}, R^{d}, m1, m2, m3, m4, n1, n2, n3, n4, o1, o2, o3, o4, q3, q4, r3 and r4 have the definition set out above, especially for Formulae (A), (I) and/or (II). The Structures (I-3) and (I-4) are especially preferred.

In a further preferred embodiment, the compounds of the invention comprise at least one of the structures of the Formulae (la-3), (la-4), (Ib-4), (Ic-4) and/or (Id-4), preferably the compounds of the invention are a compound according to Formulae (la-3), (la-4), (Ib-4), (Ic-4) and/or (Id-4), 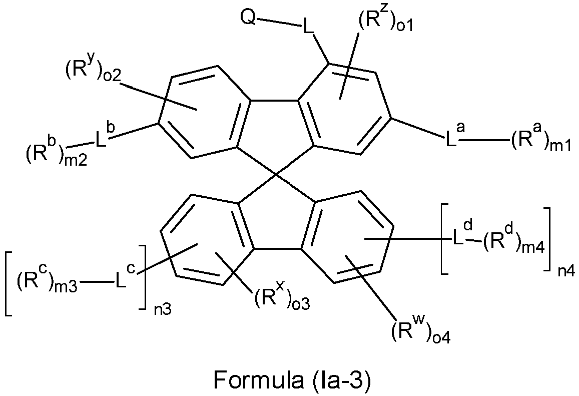 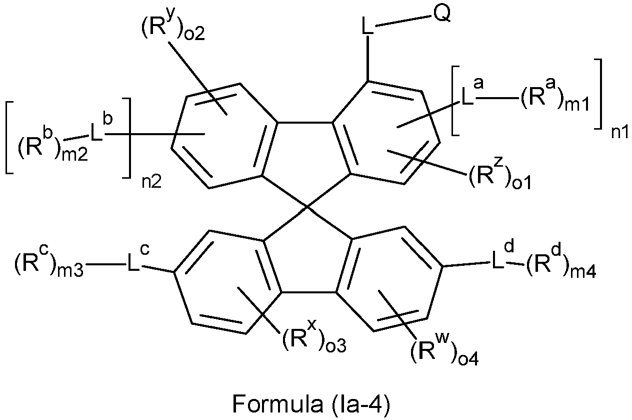 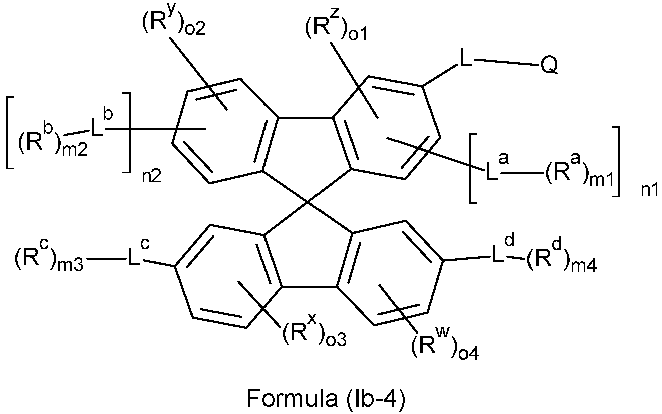 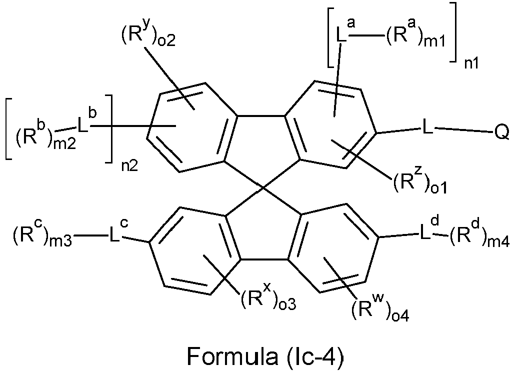 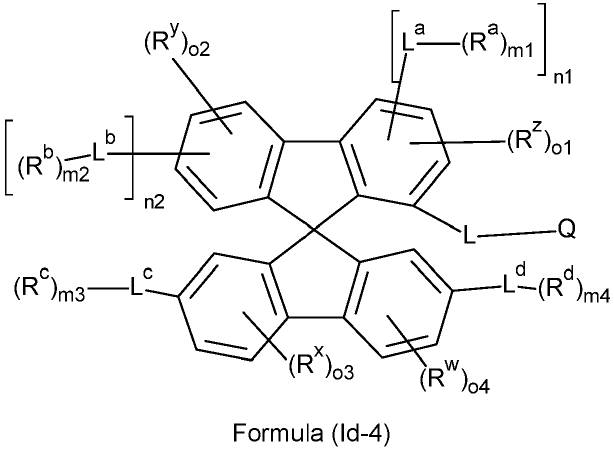 in which the symbols Q, L, L^{a}, L^{b}, L^{c}, L^{d}, R^{w}, R^{x}, R^{y}, R^{z}, R^{a}, R^{b}, R^{c}, R^{d}, m1, m2, m3, m4, n1, n2, n3, n4, o1, o2, o3 and o4 have the definition set out above, especially for Formulae (A), (I) and/or (II).

Furthermore, it can be provided, preferably in Formulae (1-1), (I-2), (I-3), (I-4), (I-5), (I-6), (II-1), (II-2), (II-3), (II-4), (II-5), (II-6), (la-3), (la-4), (Ib-4), (Ic-4) and/or (Id-4) that the sum of the indices n1, n2, n3, n4, o1, o2, o3, o4, q3, q4, r3 and r4 is in the range of 1 to 8, preferably 1 to 6, more preferably 2 to 4.

Additionally, it can be provided, preferably in Formulae (1-1), (I-2), (I-3), (I-4), (I-5), (I-6), (II-1), (II-2), (II-3), (II-4), (II-5), (II-6), (la-3), (la-4), (Ib-4), (Ic-4) and/or (Id-4) that the sum of the indices m1, m2, m3 and m4 is in the range of 1 to 6, preferably 1 to 4, more preferably 2 or 3.

In a preferred configuration, compounds comprising structures of Formulae (A), (I), (II), (Ia) to (Id), (IIa) to (IId), (1-1), (I-2), (I-3), (I-4), (I-5), (I-6), (II-1), (II-2), (II-3), (II-4), (II-5), (II-6), (la-3), (la-4), (Ib-4), (Ic-4) and/or (Id-4) can be represented by structures of Formulae (A), (I), (II), (Ia) to (Id), (IIa) to (IId), (1-1), (I-2), (I-3), (I-4), (I-5), (I-6), (II-1), (II-2), (II-3), (II-4), (II-5), (II-6), (Ia-3), (la-4), (Ib-4), (Ic-4) and/or (Id-4). Preferably, compounds comprising structures of Formulae (A), (I), (II), (Ia) to (Id), (IIa) to (IId), (1-1), (I-2), (I-3), (I-4), (I-5), (I-6), (II-1), (II-2), (II-3), (II-4), (II-5), (II-6), (la-3), (la-4), (Ib-4), (Ic-4) and/or (Id-4) have a molecular weight of not more than 5000 g/mol, preferably not more than 4000 g/mol, particularly preferably not more than 3000 g/mol, especially preferably not more than 2000 g/mol and most preferably not more than 1200 g/mol.

In addition, it is a feature of preferred compounds of the invention that they are sublimable. These compounds generally have a molar mass of less than about 1200 g/mol.

The Q group is an electron-transporting group. Electron-transporting groups are widely known in the technical field and promote the ability of compounds to transport and/or conduct electrons.

Moreover, the Q group comprises at least one structure selected from the group of pyridines, pyrimidines, pyrazines, pyridazines, triazines, quinazolines, quinoxalines, quinolines, isoquinolines, imidazoles and/or benzimidazoles. Preferably, the Q group is a pyridine group, a pyrimidine group, or a triazine group, more preferably a triazine group, which may be sub¬stituted by one or more radicals R. This is especially true with regard to compounds comprising structures of Formulae (A), (I), (II), (Ia) to (Id), (IIa) to (IId), (1-1), (I-2), (I-3), (I-4), (I-5), (I-6), (II-1), (II-2), (II-3), (II-4), (II-5), (II-6), (la-3), (la-4), (Ib-4), (Ic-4) and/or (Id-4).

More preferably, the Q group is selected from the structures of the Formulae (Q-1), (Q-2), (Q-3), (Q-4), (Q-5), (Q-6), (Q-7) and/or (Q-8) 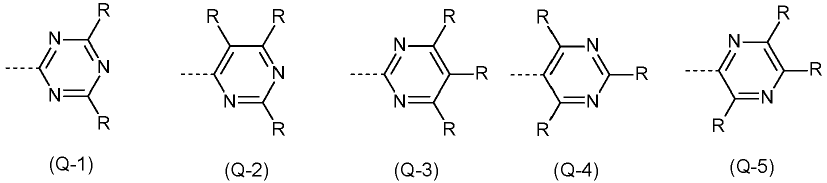 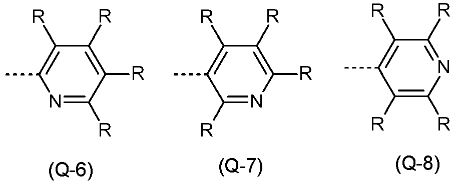 where the symbol R has the definition given above, especially for Formulae (A), the dotted bond marks the attachment position. The structures (Q-1) to (Q-5) are preferred and the structure (Q-1) is especially preferred.

Even more preferably, the Q group is selected from the structures of the Formulae (Q-1a), (Q-1b), (Q-1c), (Q-1d), (Q-1e), (Q-1f), (Q-1g), (Q-1h), (Q-1i) and/or (Q-1j) 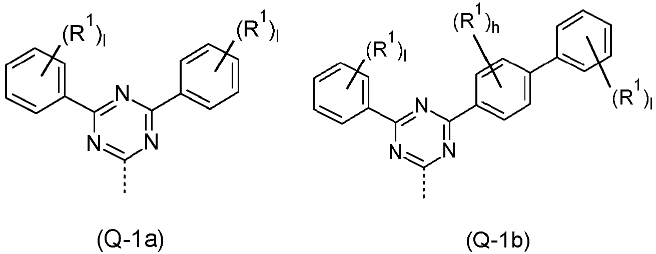 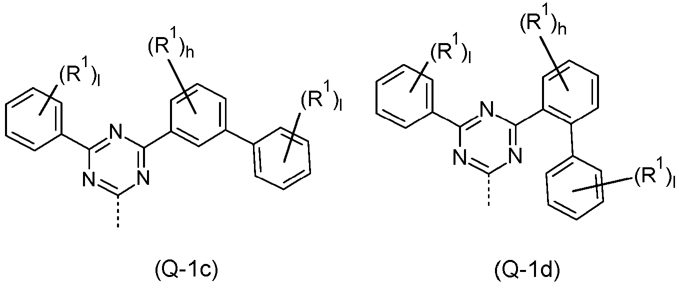 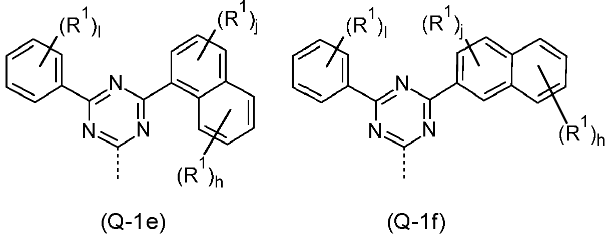 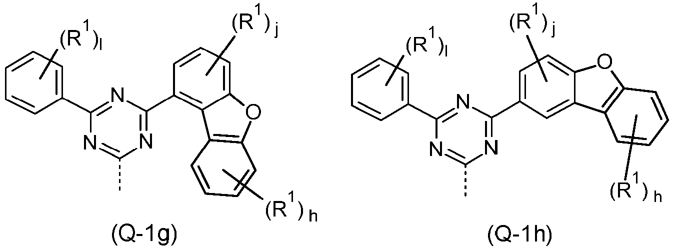 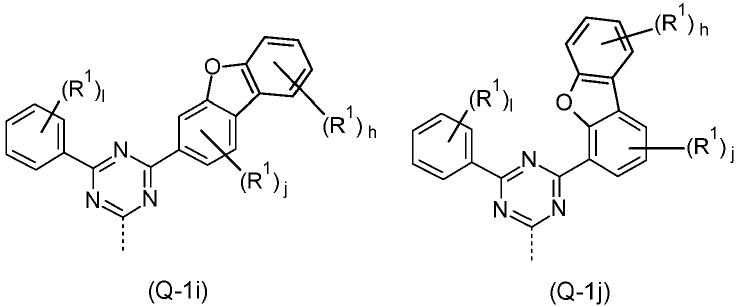 where the symbol R¹ has the definition given above, especially for Formulae (A), (I) and/or (II), the dotted bond marks the attachment position and the index I is the same or different at each instance and is 0, 1, 2, 3, 4 or 5, preferably 0, 1, 2 or 3, more preferably 0 or 1, the index h is the same or different at each instance and is 0, 1, 2, 3 or 4, preferably 0, 1 or 2, the index j is the same or different at each instance and is 0, 1, 2 or 3, preferably 0, 1 or 2. The structure (Q-1a) is preferred.

Preferably, the group **L,** L^{a}, L^{b}, L^{c}, L^{d} may form through-conjugation with the electron-transporting Q group and the fluorene structure of the Formulae (A), (I), (II) and/or preferred embodiments thereof. Through-conjugation of the aromatic or heteroaromatic systems is formed as soon as direct bonds are formed between adjacent aromatic or heteroaromatic rings. A further bond between the aforementioned conjugated groups, for example via a sulphur, nitrogen or oxygen atom or a carbonyl group, is not detrimental to conjugation. In the case of a fluorene system, the two aromatic rings are bonded directly, where the sp³-hybridized carbon atom in position 9 does prevent fusion of these rings, but conjugation is possible, since this sp³-hybridized carbon atom in position 9 does not necessarily lie between the electron-transporting Q group and the fluorene structure. In contrast, in the case of a spirobifluorene structure, through-conjugation can be formed if the bond between the electron-transporting Q group and the fluorene structure of the Formulae (A), (I), (II) and/or preferred embodiments thereof is via the same phenyl group in the spirobifluorene structure or via phenyl groups in the spirobifluorene structure that are bonded directly to one another and are in one plane. If the bond between the electron-transporting Q group and the fluorene structure of the Formulae (A), (I), (II) and/or preferred embodiments thereof is via different phenyl groups of the spirobifluorene structure which are bonded via the sp³-hybridized carbon atom in position 9, the conjugation is interrupted.

In a further preferred embodiment of the invention, L, L^{a}, L^{b}, L^{c}, L^{d} is the same or different at each instance and is a single bond or an aromatic or heteroaromatic ring system which has 5 to 24 aromatic ring atoms and may be substituted by one or more R radicals. More preferably, L, L^{a}, L^{b}, L^{c}, L^{d} is the same or different at each instance and is a single bond or an aromatic ring system having 6 to 12 aromatic ring atoms or a heteroaromatic ring system having 5 to 12 aromatic ring atoms, each of which may be substituted by one or more R radicals, but is preferably unsubstituted, where R may have the definition given above, especially for Formula (A), (I) and/or (II). Further preferably, the symbol group **L,** L^{a}, L^{b}, L^{c}, L^{d} is the same or different at each instance and is a single bond or an aryl or heteroaryl radical, such that an aromatic or heteroaromatic group of an aromatic or heteroaromatic ring system is bonded directly, i.e. via an atom in the aromatic or heteroaromatic group, to the respective atom in the other group. More preferably, group L, L^{a}, L^{b}, L^{c}, L^{d} is a bond or an aryl group having 6 to 12 aromatic ring atoms, which may be substituted by one or more radicals R, more preferably a phenylene group, a biphenylene group or a naphthylene group, even more preferably a phenylene group or a naphthylene group, which may be sub¬stituted by one or more radicals R. Even more preferably, group L, L^{a}, L^{b}, L^{c}, L^{d} is a single bond. Examples of suitable aromatic or heteroaromatic ring systems group L, L^{a}, L^{b}, L^{c}, L^{d} are selected from the group consisting of ortho-, meta- or para-phenylene, biphenylene, fluorene, pyridine, pyrimidine, triazine, dibenzofuran and dibenzothiophene, each of which may be substituted by one or more R radicals, but are preferably unsubstituted.

Preference is given to compounds, preferably compounds comprising structures of the formulae (A), (I), (II), (Ia) to (IId), (la-3) to (Id-4) and/or (Ia-3a), to (Ic-4a), (IVb) in which the group L, L^{a}, L^{b}, L^{c}, L^{d} is a bond or a group selected from the formulae (L-1) to (L-9) 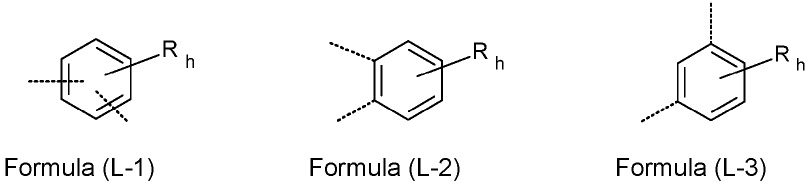 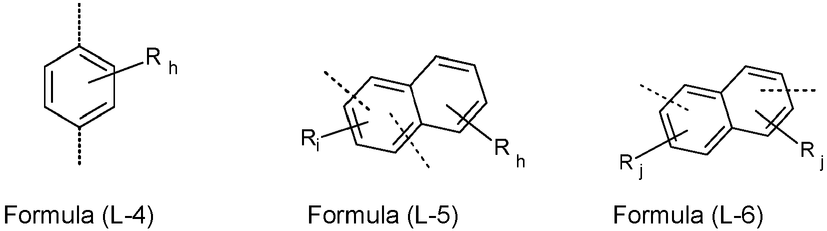 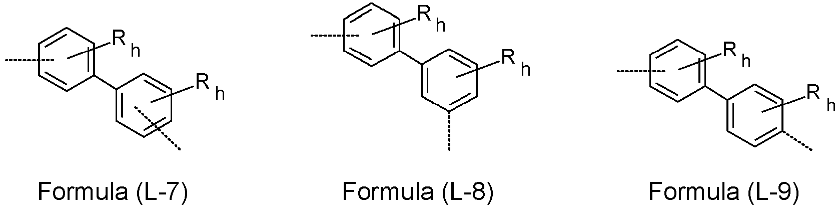 where the symbol R has the definition given above, especially for Formulae (A), (I) and/or (II), the dotted bond marks the attachment positions, the index h is the same or different at each instance and is 0, 1, 2, 3 or 4, preferably 0, 1 or 2, the index j is the same or different at each instance and is 0, 1, 2 or 3, preferably 0, 1 or 2; the index i is the same or different at each instance and 0, 1 or 2, preferably 0 or 1; wherein the structures (L-1) to (L-4) are preferred and the structures (L-3) and (L-4) are more preferred.

It may preferably be the case that the total sum of the indices h, i and j in the structures of the formula (L-1) to (L-9) is at most 3 in each case, preferably at most 2 and more preferably at most 1.

In an especially preferred embodiment, the compounds of the invention comprise at least one of the structures of the Formulae (la-3a), (la-4a), (Ib-4a) and/or (Ic-4a), preferably the compounds of the invention are a compound according to Formulae (la-3a), (la-4a), (Ib-4a) and/or (Ic-4a), 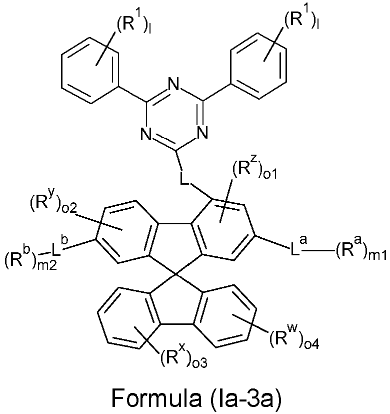 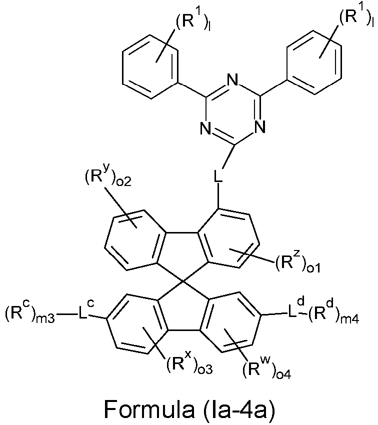 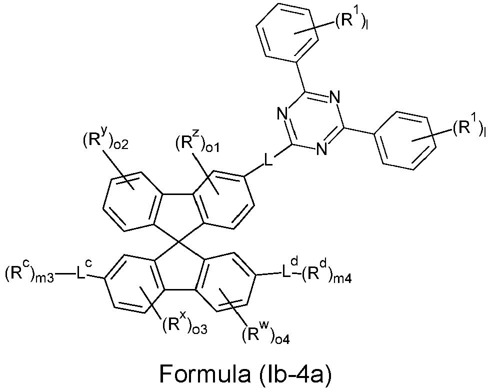 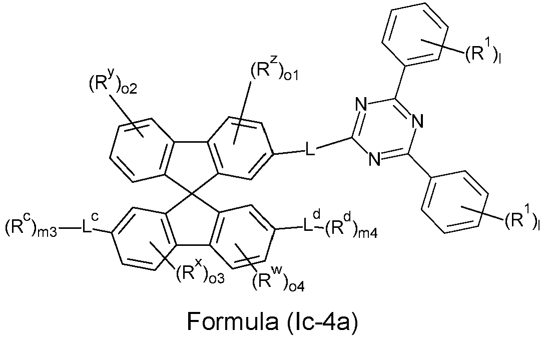 in which the symbols L, L^{a}, L^{b}, L^{c}, L^{d}, R¹, R^{w}, R^{x}, R^{y}, R^{z}, Ra, R^{b}, R^{c}, R^{d}, I, m1, m2, m3, m4, o1, o2, o3 and o4 have the definition set out above, especially for Formulae (A), (I), (II) and/or (Q-1a).

Furthermore, it can be provided, preferably in Formulae (la-3a), (la-4a), (Ib-4a) and/or (Ic-4a), that the sum of the indices I, o1, o2, o3 and o4 is in the range of 0 to 4, preferably 0 to 2, more preferably 0.

Additionally, it can be provided, preferably in Formulae (la-3a), (la-4a), (Ib-4a) and/or (Ic-4a), that the sum of the indices m1, m2, m3 and m4 is in the range of 1 to 4, preferably 1 to 3, more preferably 2.

Regarding the symbols L, L^{a}, L^{b}, L^{c}, L^{d}, R¹, R^{w}, R^{x}, R^{y}, R^{z}, R^{a}, R^{b}, R^{c}, R^{d}, 1, m1, m2, m3, m4, o1, o2, o3 and o4 in Formulae (la-3a), (la-4a), (Ib-4a) and/or (Ic-4a), the preferences mentioned above with regard to Formulae (A), (I) and/or (II).

Preferably it can be provided in Formulae (la-3a), (la-4a), (Ib-4a) and/or (Ic-4a), that the symbol L, L^{a}, L^{b}, L^{c}, L^{d} is a bond or is selected from the structures of the formulae (L-1) to (L-9) as mentioned above, preferably the symbol L, L^{a}, L^{b}, L^{c}, L^{d} is a bond or is selected from the structures of the formulae (L-3) or (L-4) as mentioned above.

Preferably it can be provided in Formulae (A), (I), (II) and/or preferred embodiments thereof that the symbol R^{a}, R^{b}, R^{c}, R^{d} is a structure according to Formulae (R^{a}-14) (t-butyl), (R^{a}-32) or (R^{a}-33) (adamantyl).

When the aromatic and/or heteroaromatic groups are substituted by R, R^{w}, R^{x}, R^{y}, R^{z} substituents, these R, R^{w}, R^{x}, R^{y}, R^{z} substituents are preferably selected from the group consisting of H, D, F, CN, a straight-chain alkyl or alkoxy group having 1 to 10 carbon atoms or a branched or cyclic alkyl or alkoxy group having 3 to 10 carbon atoms or an alkenyl group having 2 to 10 carbon atoms, each of which may be substituted by one or more R¹ radicals, where one or more non-adjacent CH₂ groups may be replaced by O and where one or more hydrogen atoms may be replaced by D or F, an aromatic or heteroaromatic ring system which has 5 to 24 aromatic ring atoms, each of which may be substituted by one or more R¹ radicals, but is preferably unsubstituted, or an aralkyl or heteroaralkyl group which has 5 to 25 aromatic ring atoms and may be substituted by one or more R¹ radicals, at the same time, it is optionally possible for two R, R^{w}, R^{x}, R^{y}, R^{z} substituents bonded to the same carbon atom or adjacent carbon atoms to form a monocyclic or polycyclic, aliphatic, aromatic or heteroaromatic ring system with one another, which may be substituted by one or more R¹ radicals.

More preferably, these R, R^{w}, R^{x}, R^{y}, R^{z} substituents are selected from the group consisting of H, D, F, CN, a straight-chain alkyl group having 1 to 8 carbon atoms, preferably having 1, 2, 3 or 4 carbon atoms, or a branched or cyclic alkyl group having 3 to 8 carbon atoms, preferably having 3 or 4 carbon atoms, or an alkenyl group having 2 to 8 carbon atoms, preferably having 2, 3 or 4 carbon atoms, each of which may be substituted by one or more R¹ radicals, but is preferably unsubstituted, or an aromatic or heteroaromatic ring system having 6 to 24 aromatic ring atoms, preferably having 6 to 18 aromatic ring atoms, more preferably having 5 to 12 aromatic ring atoms, each of which may be substituted by one or more nonaromatic R, R^{w}, R^{x}, R^{y}, R^{z} radicals, but is preferably unsubstituted; at the same time, it is optionally possible for two R, R^{w}, R^{x}, R^{y}, R^{z} substituents bonded to the same carbon atom or two adjacent carbon atoms to form a monocyclic or polycyclic aliphatic ring system with one another, which may be substituted by one or more R¹ radicals, but is preferably unsubstituted.

Most preferably, the R, R^{w}, R^{x}, R^{y}, R^{z} substituents are selected from the group consisting of H and an aromatic or heteroaromatic ring system having 6 to 18 aromatic ring atoms, preferably having 5 to 12 aromatic ring atoms, each of which may be substituted by one or more nonaromatic R² radicals, but is preferably unsubstituted. Examples of suitable R, R^{w}, R^{x}, R^{y}, R^{z} substituents are selected from the group consisting of phenyl, ortho-, meta- or para-biphenyl, terphenyl, especially branched terphenyl, quaterphenyl, especially branched quaterphenyl, 1-, 2-, 3- or 4-fluorenyl, 1-, 2-, 3- or 4-spirobifluorenyl, pyridyl, pyrimidinyl, 1-, 2-, 3- or 4-dibenzofuranyl, 1-, 2-, 3- or 4-dibenzothienyl and 1-, 2-, 3- or 4-carbazolyl, each of which may be substituted by one or more R¹ radicals, but are preferably unsubstituted.

It may additionally be the case that, in a structure of Formulae (A), (I), (II) and/or preferred embodiments thereof, at least one R, R^{w}, R^{x}, R^{y} and/or R^{z} radical is a group selected from the formulae (R-1) to (R-43) 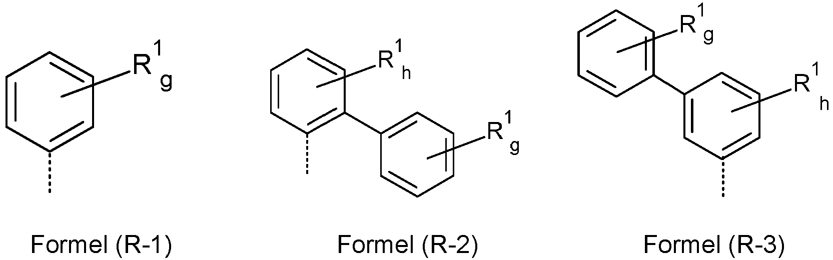 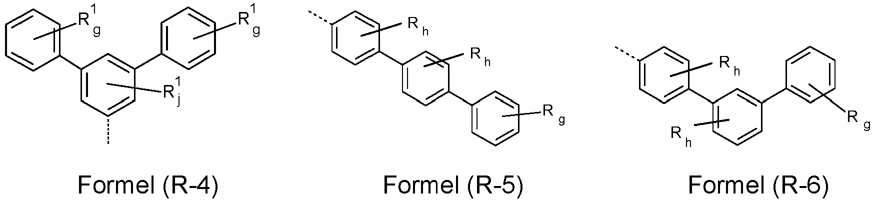 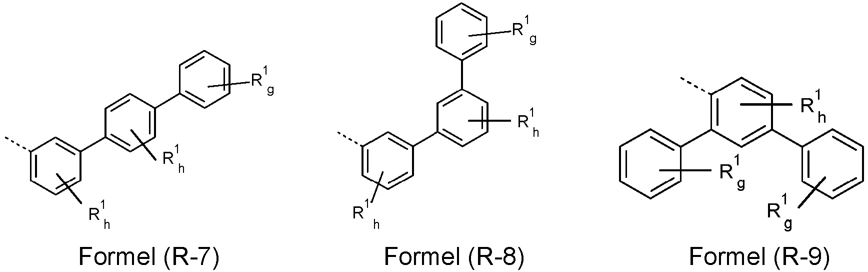 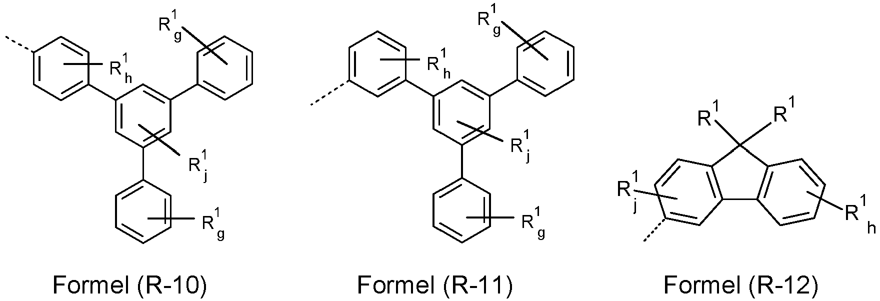 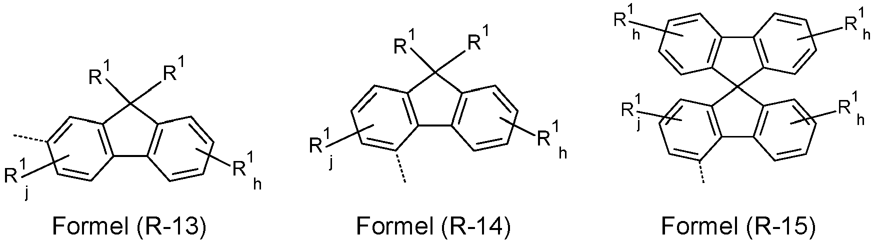 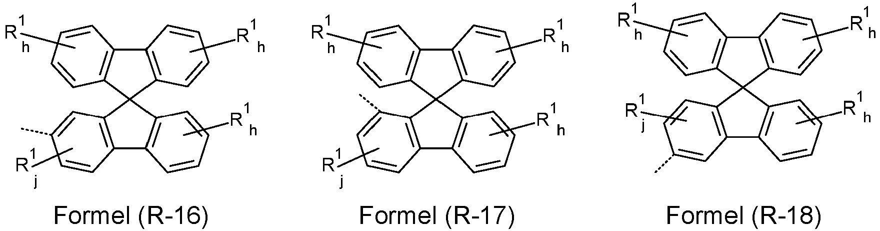 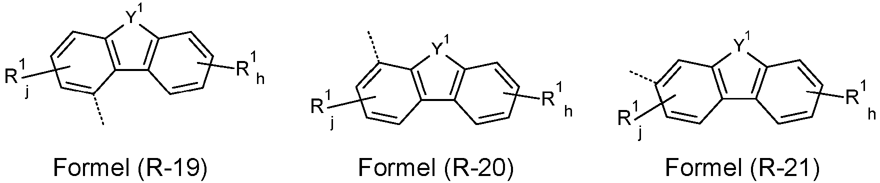 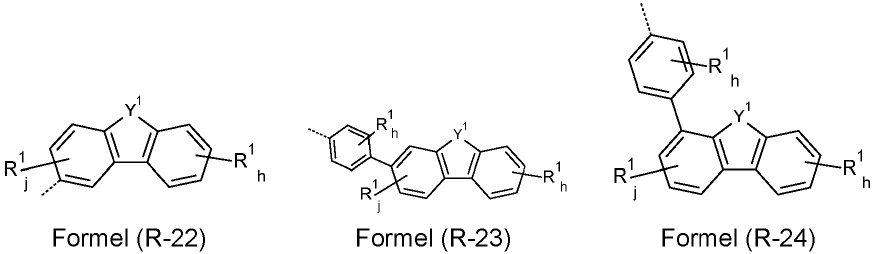 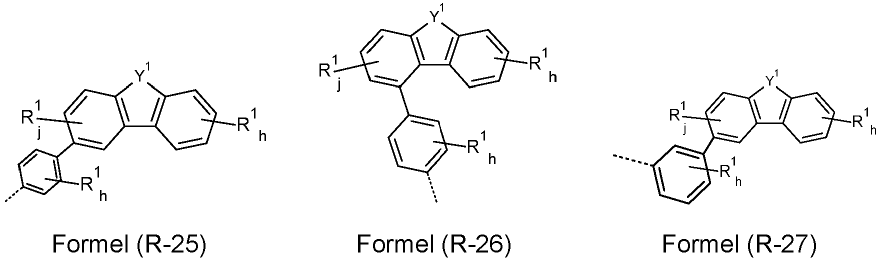 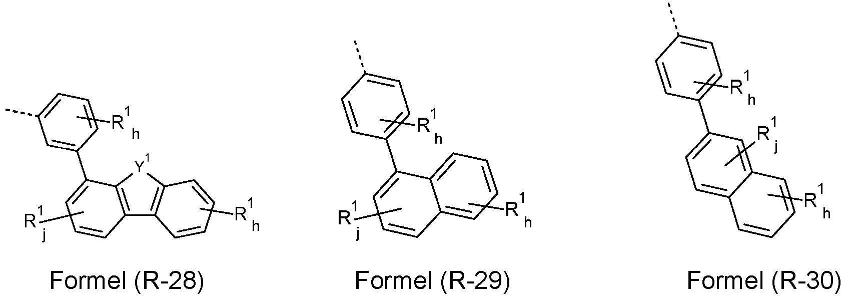 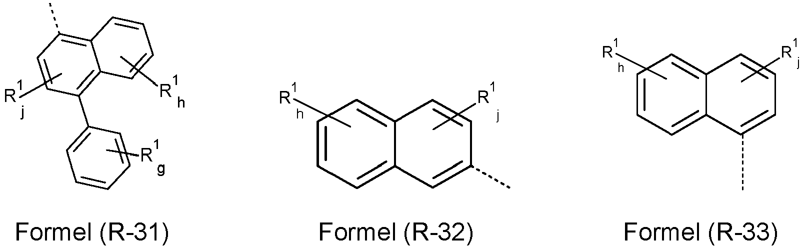 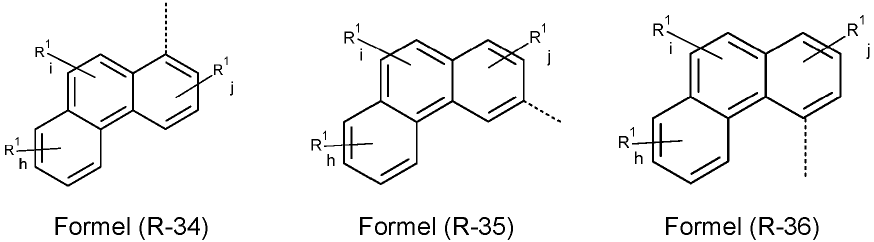 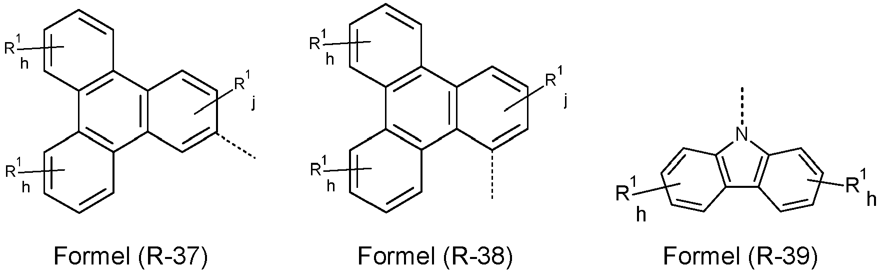 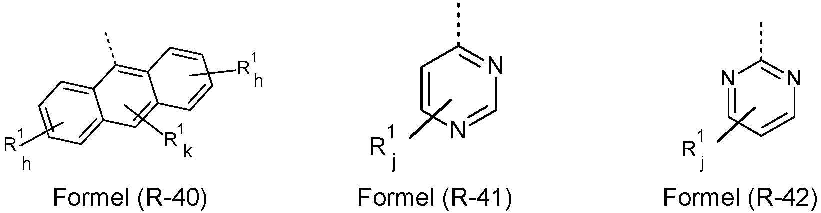 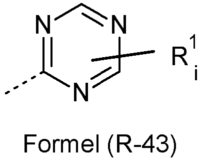 where the symbols used are as follows:
- Y¹: is O, S or NR¹, preferably O or S;
- i: at each instance is independently 0, 1 or 2;
- j: independently at each instance is 0, 1, 2 or 3;
- h: independently at each instance is 0, 1, 2, 3 or 4;
- g: independently at each instance is 0, 1, 2, 3, 4 or 5;
- R¹: may have the definition given above, especially for Formula (A), (I) and/or (II), and
the dotted bond marks the attachment position.

The groups of formulae R-1 to R-28 are preferred and the groups of formulae R-1, R-3, R-4, R-10, R-11, R-12, R-13, R-14, R-16, R-17, R-18, R-19, R-20, R-21 and/or R-22 are especially preferred.

It may preferably be the case that the total sum of the indices i, j, h and g in the structures of the formula (R-1) to (R-43) is not more than 3 in each case, preferably not more than 2 and more preferably not more than 1.

In an embodiment, it may be the case that a compound of the invention comprising at least one structure of Formulae (A), (I), (II) and/or preferred embodiments thereof comprises two or more electron transport groups.

**In** a preferred embodiment a compound of the invention comprising at least one structure of Formulae (A), (I), (II) and/or preferred embodiments thereof comprises exactly one triazine group, more preferably exactly one electron transport groups.

Advantageously, it may be the case that a compound of the invention comprising at least one structure of Formulae (A), (I), (II) and/or preferred embodiments thereof does not comprise any carbazole and/or triarylamine group. More preferably, a compound of the invention does not comprise any hole-transporting group. Hole-transporting groups are known in the specialist field, these groups in many cases being carbazole, indenocarbazole, indolocarbazole, arylamine or diarylamine structures.

**In** a further configuration, it may be the case that a compound of the invention comprising at least one structure of Formulae (A), (I), (II) and/or preferred embodiments thereof comprises at least one hole-transporting group, preferably a carbazole and/or triarylamine group. **In** addition, hole-transporting groups provided may also be indenocarbazole, indenocarbazole, arylamine or diarylamine group.

**In** a further preferred embodiment of the invention, R¹, for example in a structure of Formula (A), (I), (II) and/or preferred embodiments of these structures or the structures where reference is made to these formulae, is the same or different at each instance and is selected from the group consisting of H, D, an aliphatic hydrocarbyl radical having 1 to 10 carbon atoms, preferably having 1, 2, 3 or 4 carbon atoms, or an aromatic or heteroaromatic ring system having 5 to 30 aromatic ring atoms, preferably having 5 to 24 aromatic ring atoms, more preferably having 5 to 13 aromatic ring atoms, which may be substituted by one or more alkyl groups each having 1 to 4 carbon atoms, but is preferably unsubstituted.

In a further preferred embodiment of the invention, R², for example in a structure of Formula (A), (I), (II) and/or preferred embodiments of these structures or the structures where reference is made to these formulae, is the same or different at each instance and is selected from the group consisting of H, D, F, CN, an aliphatic hydrocarbyl radical having 1 to 10 carbon atoms, preferably having 1, 2, 3 or 4 carbon atoms, or an aromatic or heteroaromatic ring system having 5 to 30 aromatic ring atoms, preferably having 5 to 24 aromatic ring atoms, more preferably having 5 to 13 aromatic ring atoms, which may be substituted by one or more alkyl groups each having 1 to 4 carbon atoms, but is preferably unsubstituted.

When the compound of the invention is substituted by aromatic or heteroaromatic R, R^{w}, R^{x}, R^{y}, R^{z}, R¹ or R² groups, it is preferable when these do not have any aryl or heteroaryl groups having more than two aromatic six-membered rings fused directly to one another. More preferably, the substituents do not have any aryl or heteroaryl groups having six-membered rings fused directly to one another at all. The reason for this preference is the low triplet energy of such structures. Fused aryl groups which have more than two aromatic six-membered rings fused directly to one another but are nevertheless also suitable in accordance with the invention are phenanthrene and triphenylene, since these also have a high triplet level.

Examples of suitable compounds of the invention are the structures shown below of the following formulae 1-47, 52-108, 113-116. Compounds of the following formulae 48-51, 109-112 are reference compounds not forming part of the present invention.

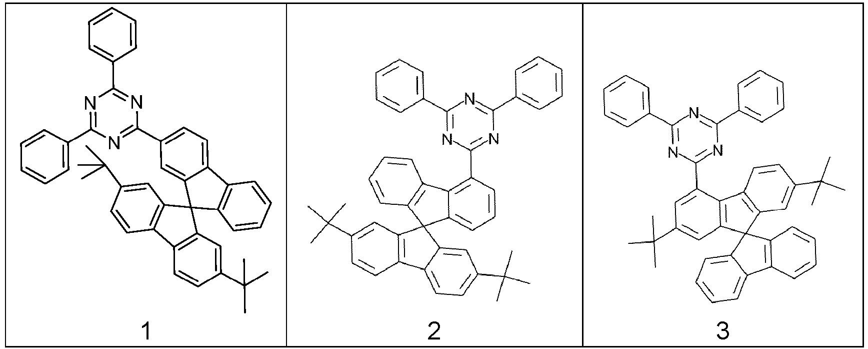

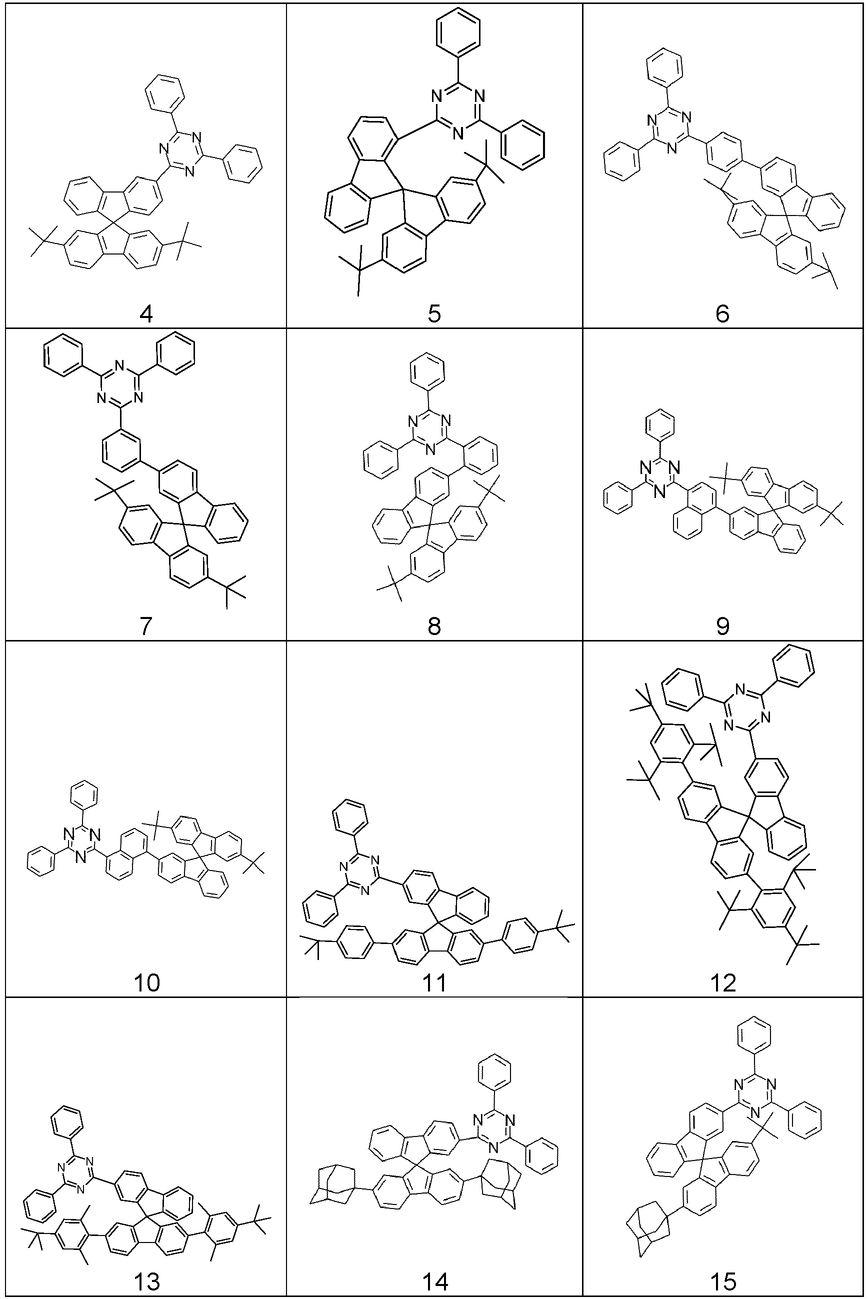

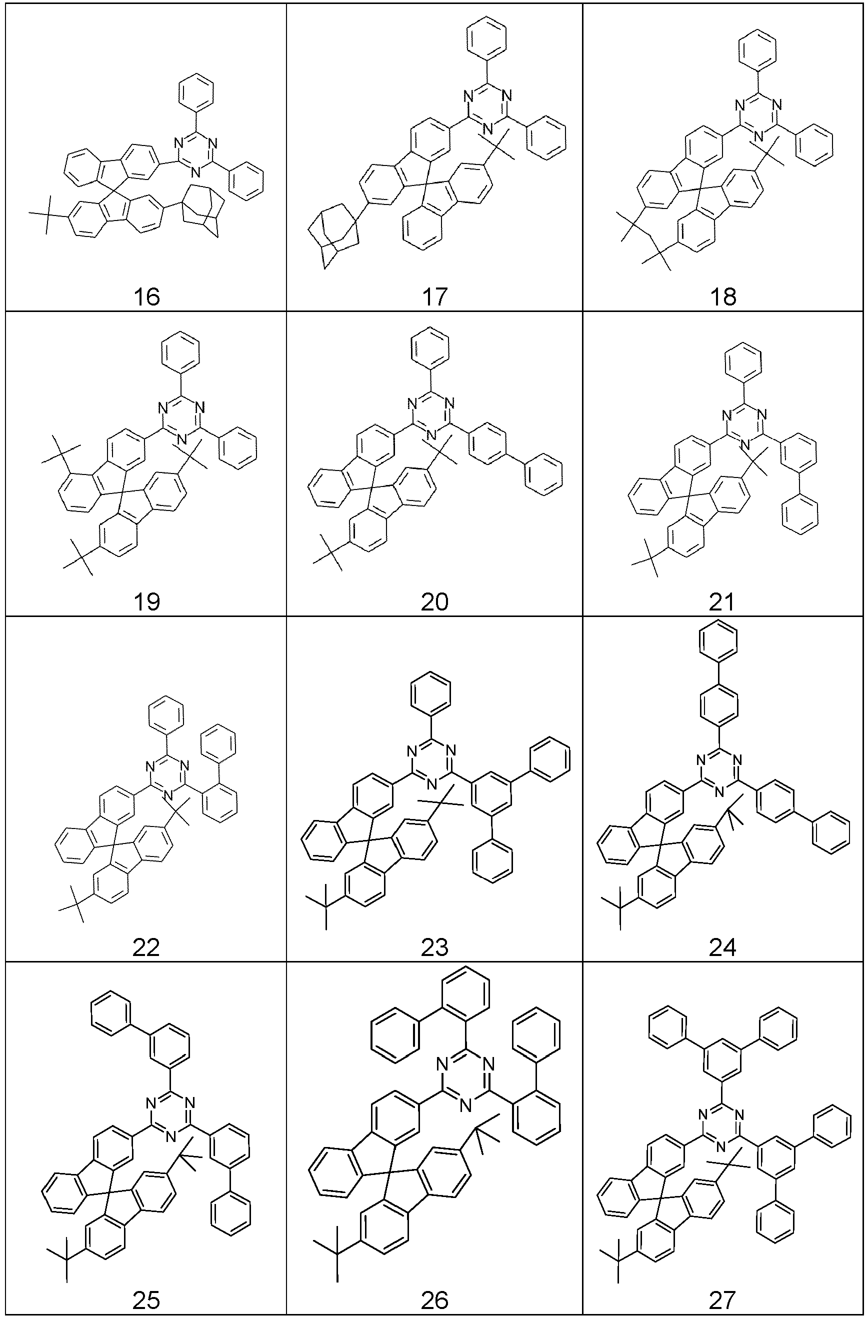

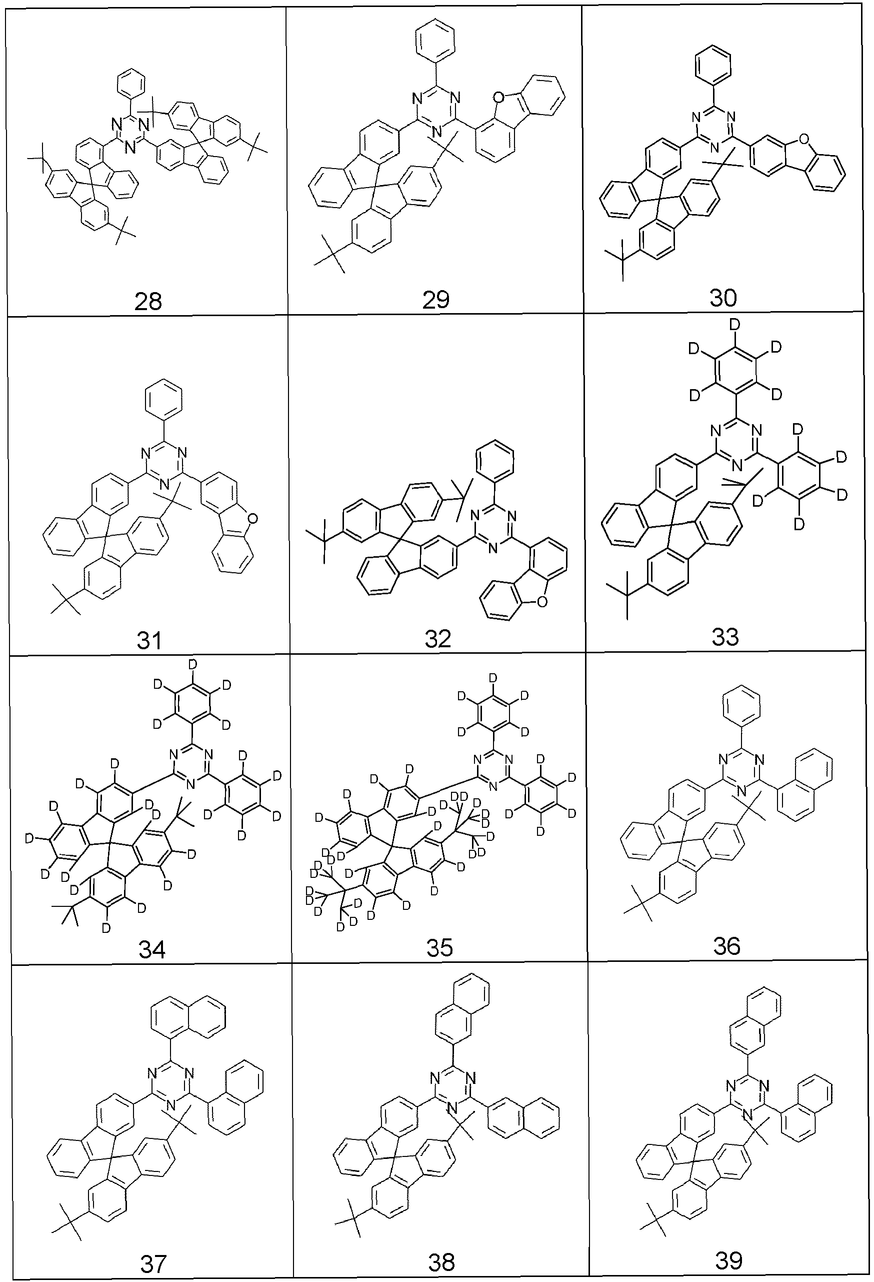

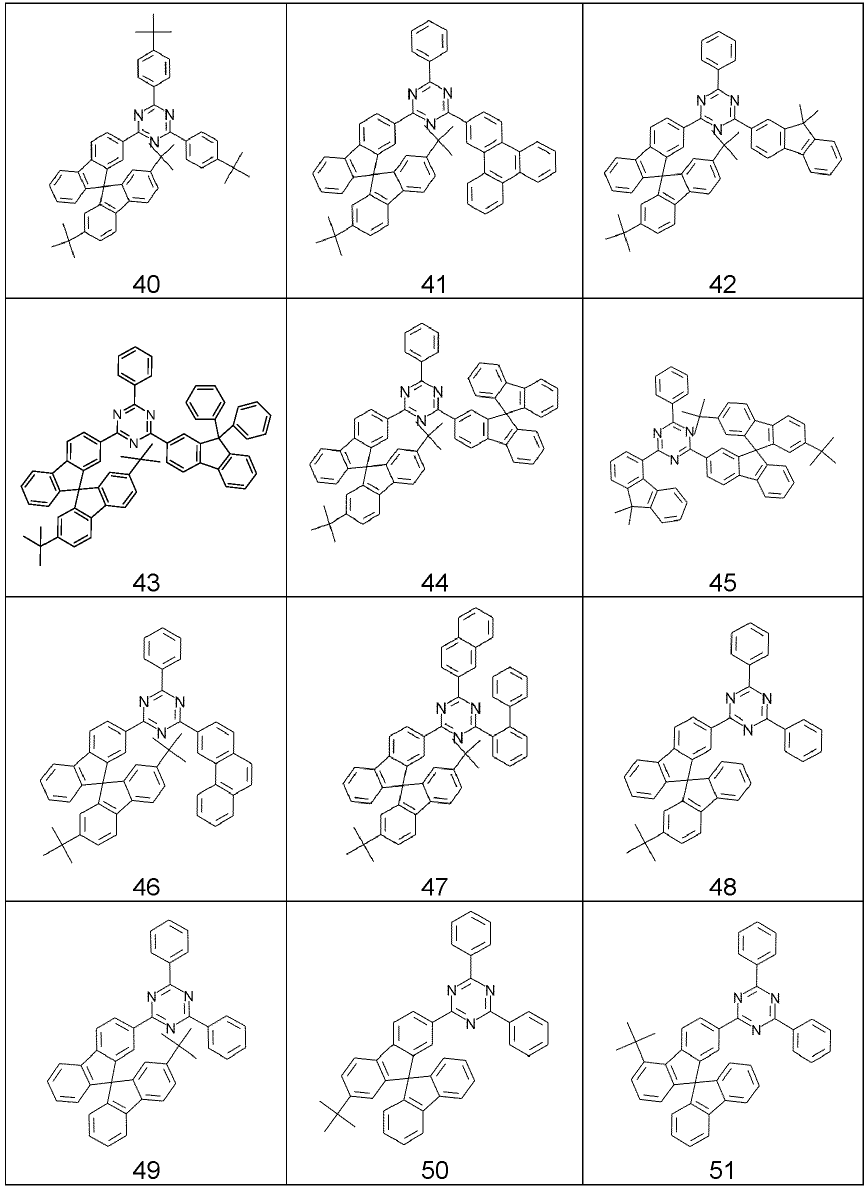

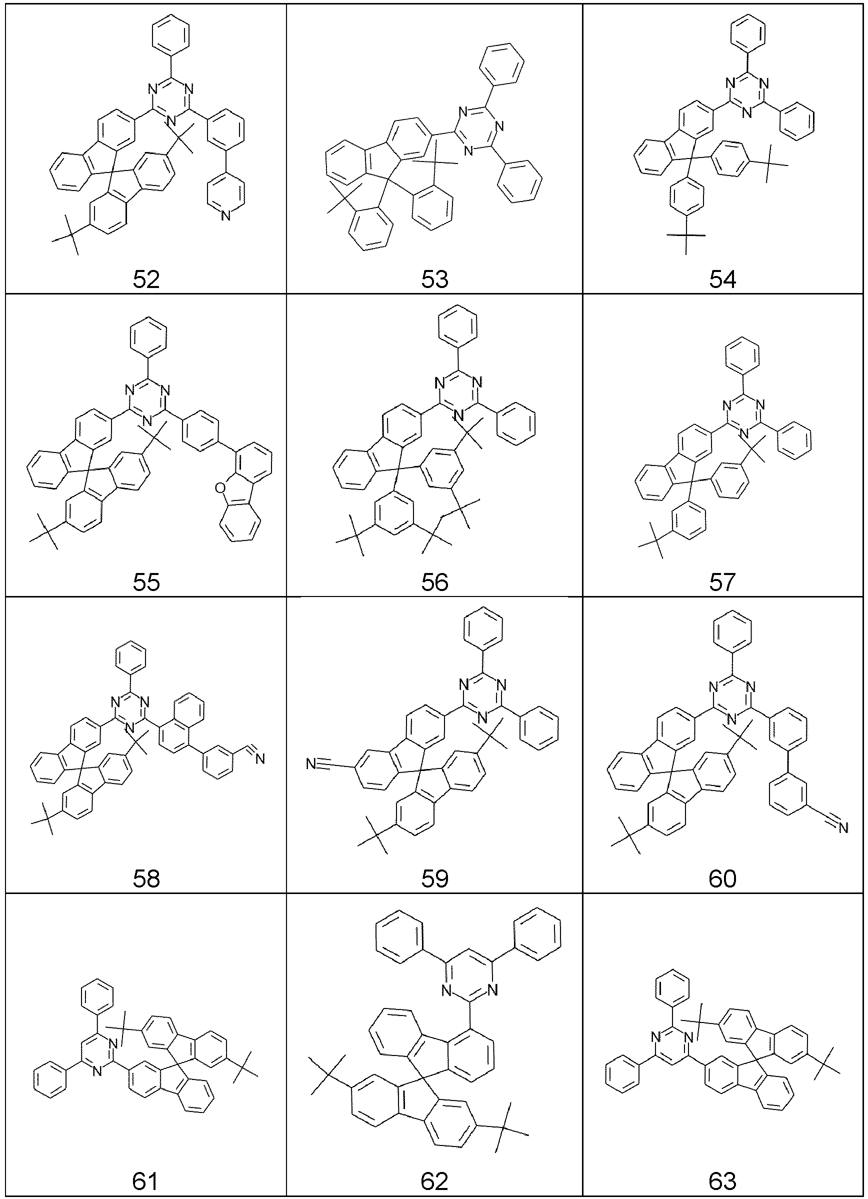

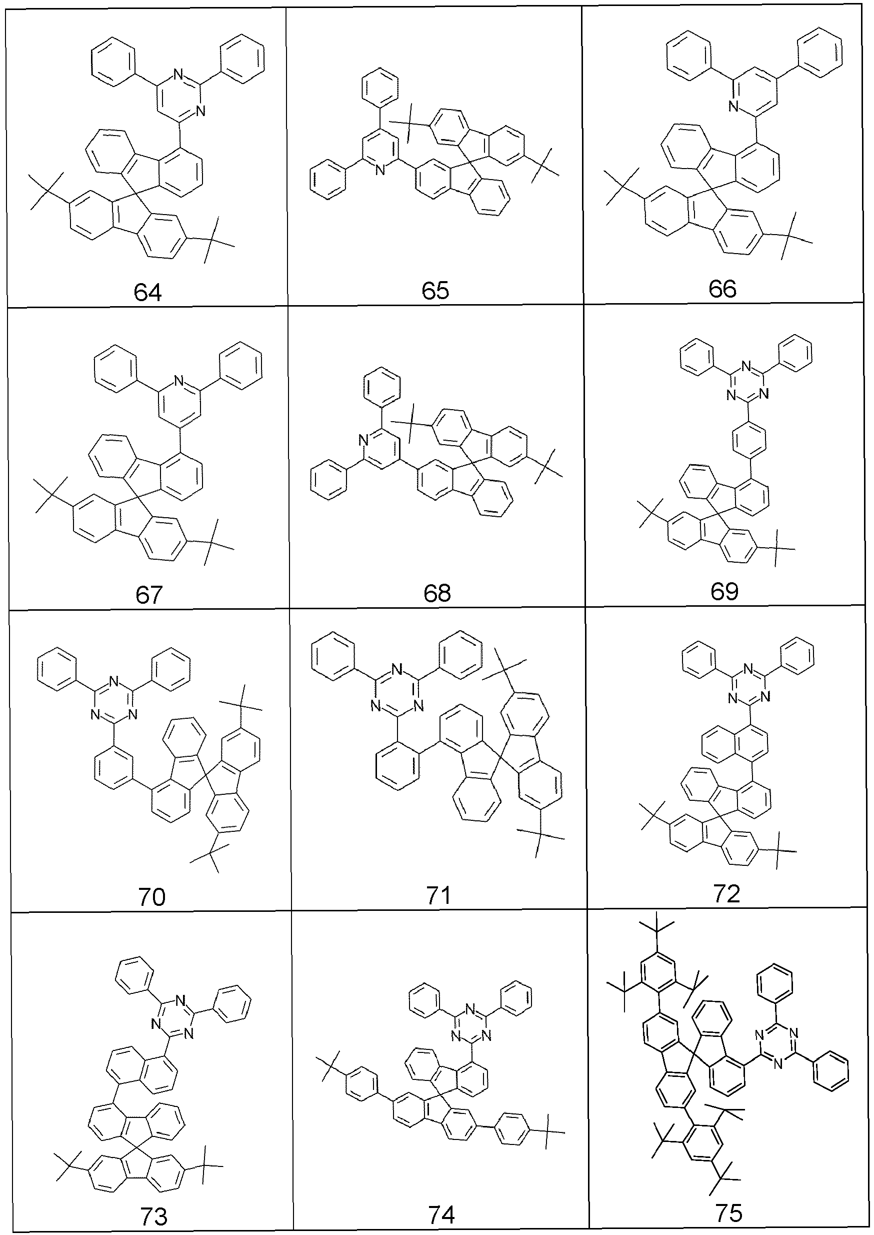

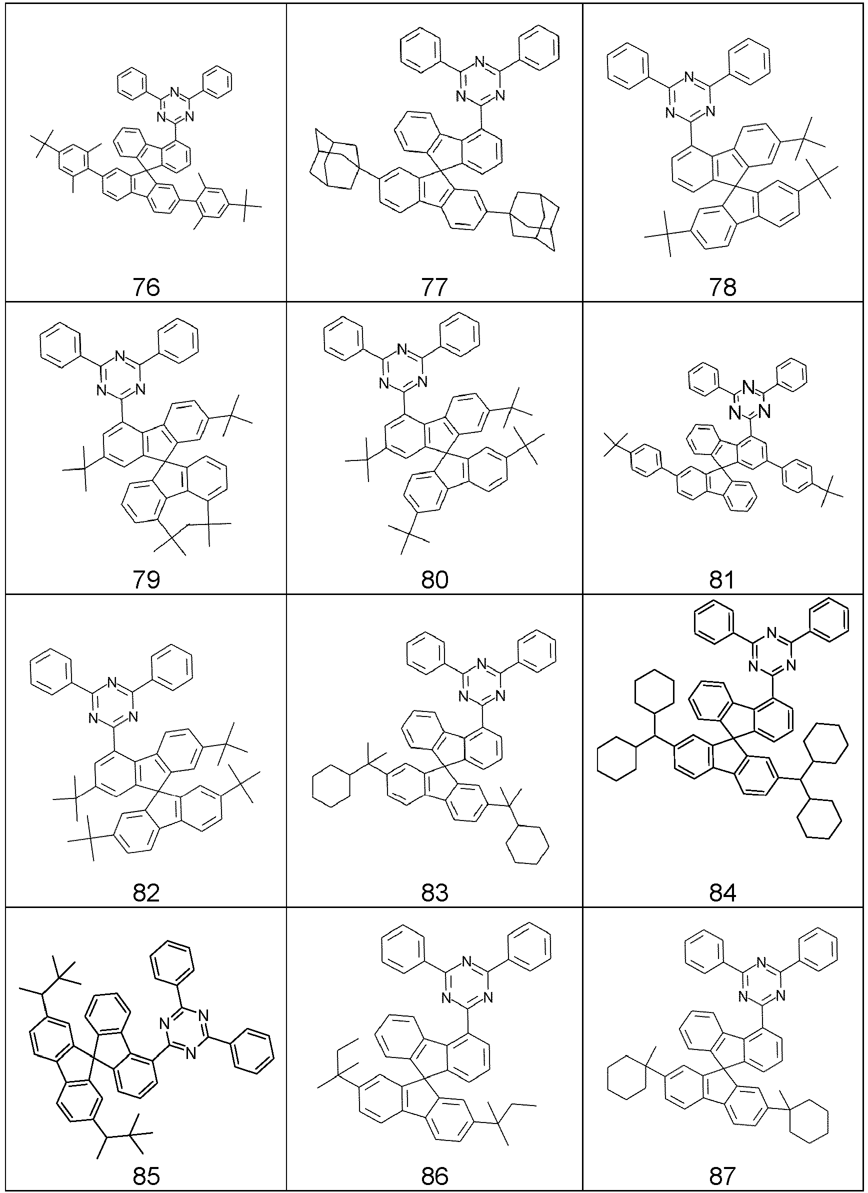

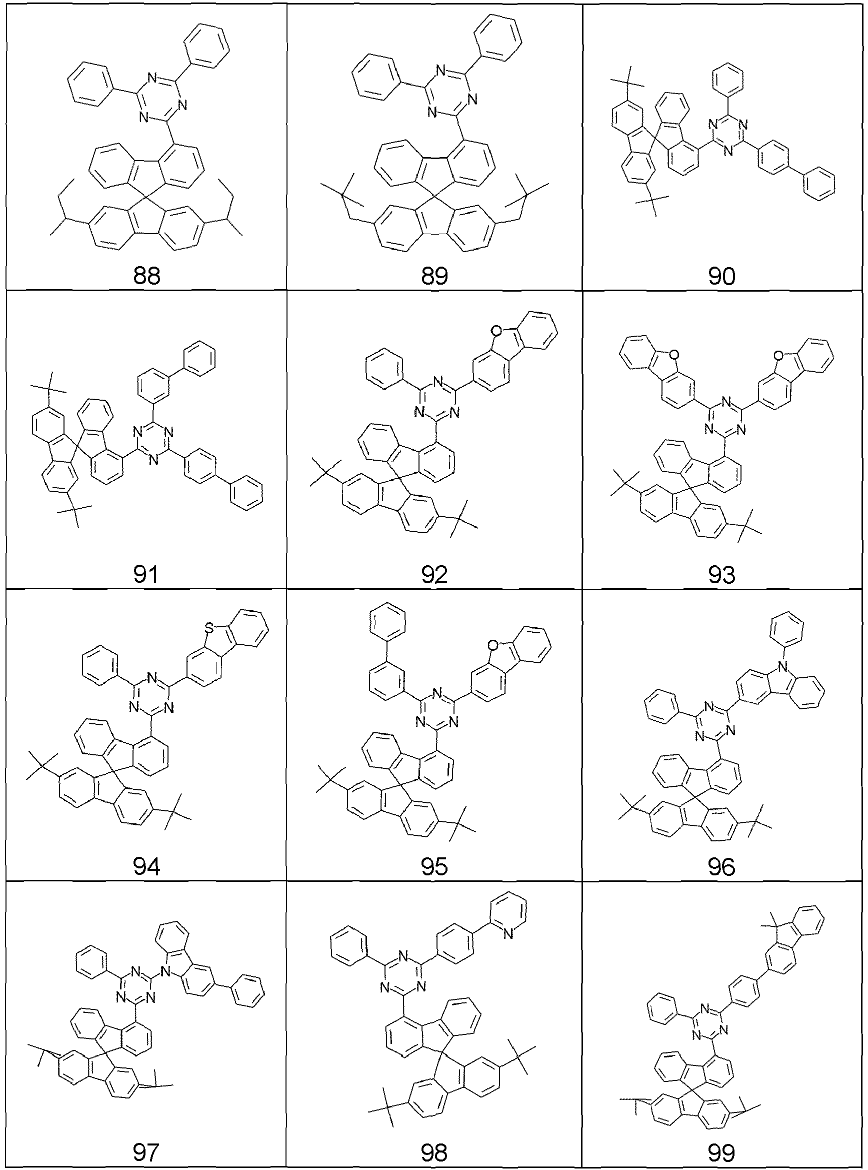

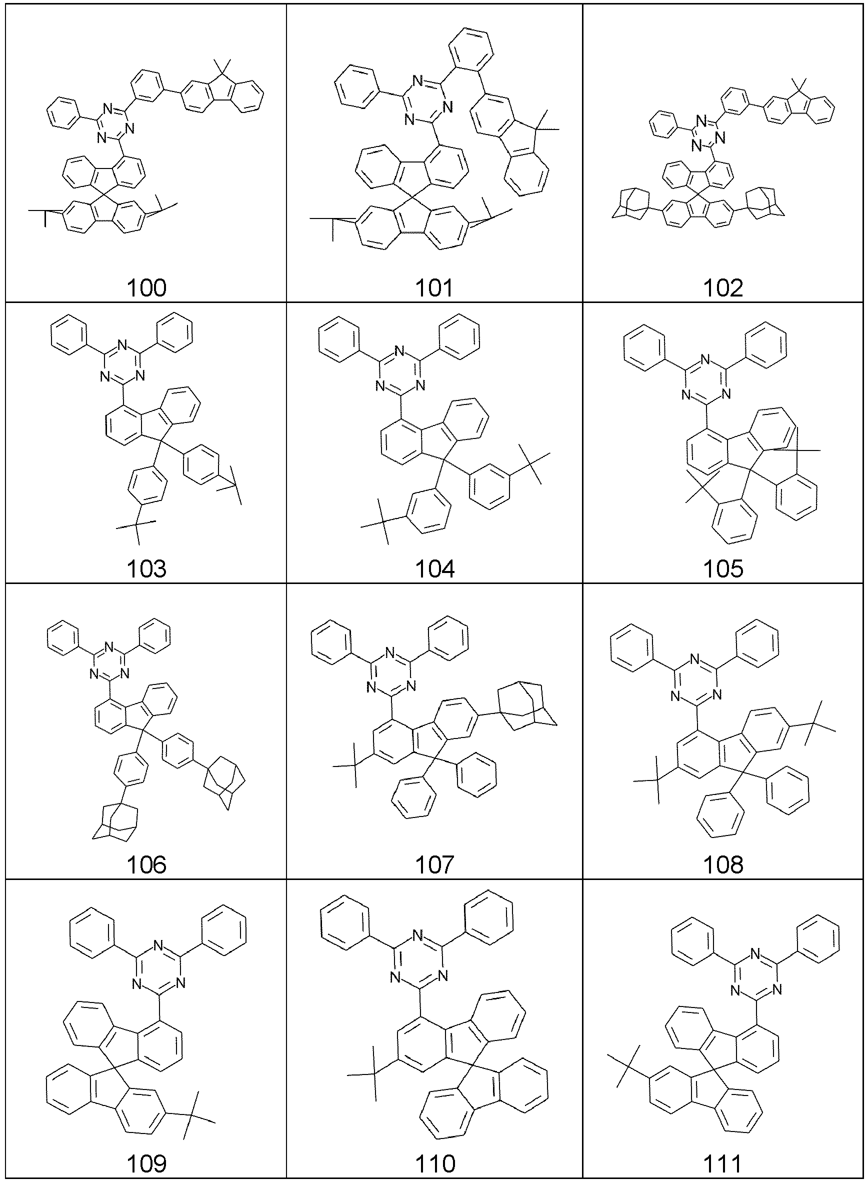

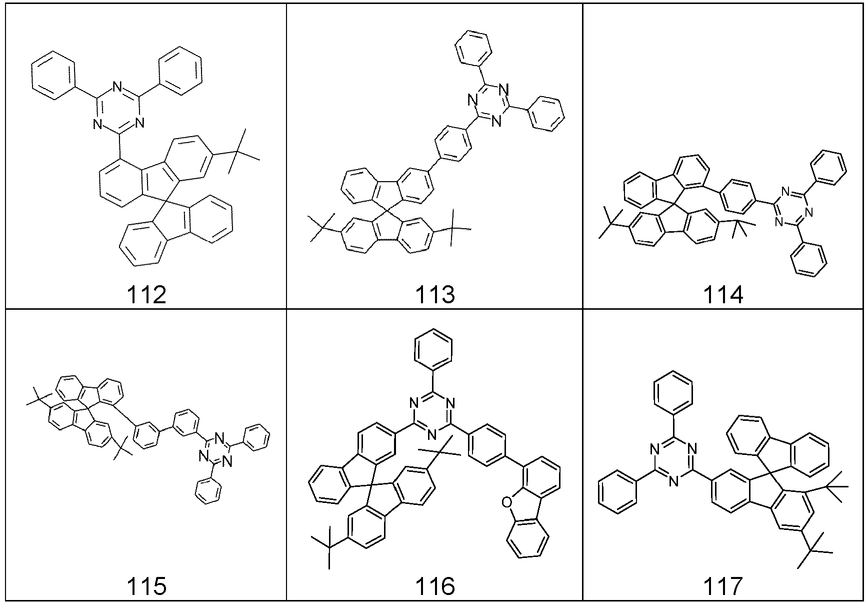

Preferred embodiments of compounds of the invention are detailed specifically in the examples, these compounds being usable alone or in combination with further compounds for all purposes of the invention.

Provided that the conditions specified in Claim 1 are complied with, the abovementioned preferred embodiments can be combined with one another as desired. In a particularly preferred embodiment of the invention, the abovementioned preferred embodiments apply simultaneously.

The compounds of the invention are preparable in principle by various processes. However, the processes described hereinafter have been found to be particularly suitable.

Therefore, the present invention further provides a process for preparing the compounds comprising structures of Formula (A), (I) and/or (II) in which, in a coupling reaction, a compound comprising at least one electron-transporting group is reacted with a compound comprising at least one fluorene or spirobifluorene radical.

Suitable compounds having an electron-transporting group are in many cases commercially available, and the starting compounds detailed in the examples are obtainable by known processes, and so reference is made thereto.

These compounds can be reacted with further aryl compounds by known coupling reactions, the necessary conditions for this purpose being known to the person skilled in the art, and detailed specifications in the examples give support to the person skilled in the art in conducting these reactions.

Particularly suitable and preferred coupling reactions which all lead to C-C bond formation and/or C-N bond formation are those according to BUCHWALD, SUZUKI, YAMAMOTO, STILLE, HECK, NEGISHI, SONOGASHIRA and HIYAMA. These reactions are widely known, and the examples will provide the person skilled in the art with further pointers.

In all the synthesis schemes which follow, the compounds are shown with a small number of substituents to simplify the structures. This does not rule out the presence of any desired further substituents in the processes.

An illustrative implementation is given by the schemes which follow, without any intention that these should impose a restriction. The component steps of the individual schemes may be combined with one another as desired.

The processes shown for synthesis of the compounds of the invention should be understood by way of example. The person skilled in the art will be able to develop alternative synthesis routes within the scope of his common knowledge in the art.

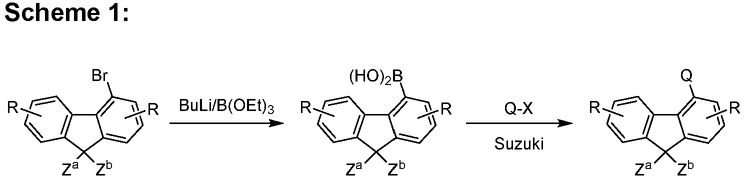

The Q group is an electron transport group, and X is a leaving group, for example halogen.

A preferred synthesis of spirobifluorene which is substituted by Q in the 1-position is shown in Scheme 2. The synthesis of spirobifluorene which is substituted by Q in the 4-position is shown in Scheme 3. Corresponding compounds being substituted at 2-position or at 3-position can be achieved by using appropriate starting compounds.

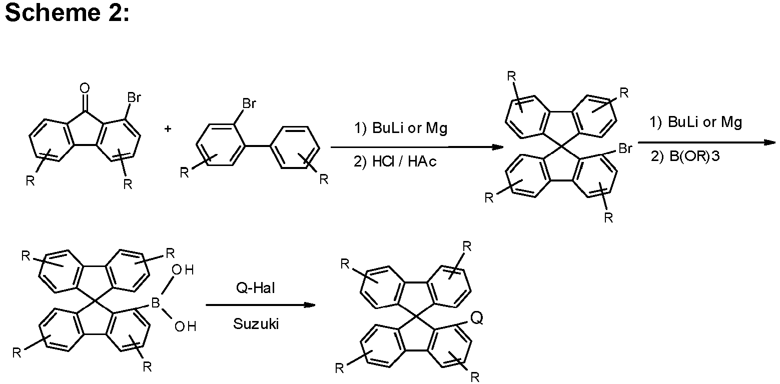

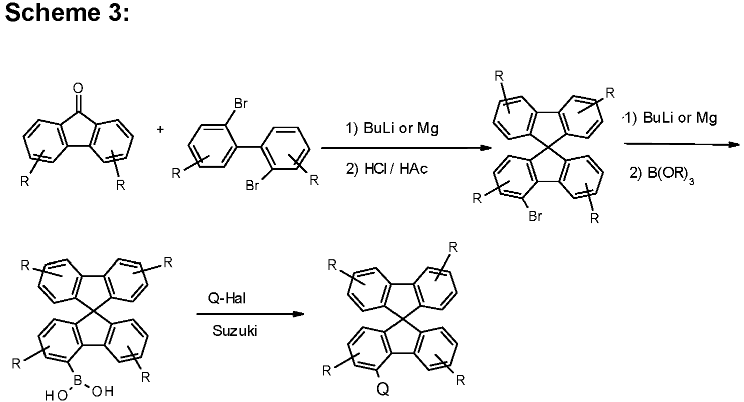

Corresponding compounds in which the group Q is not bonded directly to the spirobifluorene, but instead via a group L which does not stand for a single bond can likewise also be synthesised entirely analogously by employing a corresponding compound Q-L-Hal instead of a halogenated aromatic compound Ar-Hal. Hal here preferably stands for Cl, Br or I, in particular for Br.

The halogenated spirobifluorene derivatives which are coupled to a boronic acid derivative of the group -L-Ar can likewise be employed entirely analogously.

The definitions of the symbols used in Schemes 1, 2 and 3 corresponds essentially to those defined for Formula (A), (I), (II) and preferred embodiments of these structures, respectively, although for reasons of clarity numbering and a complete representation of all symbols have been omitted. These indications are therefore to be understood by way of example, the skilled person being able to transfer the syntheses set forth previously and hereinafter, in particular in the examples.

The fundamentals of the preparation processes set forth previously are, in principle, known from the literature for similar compounds and can be readily adapted by those skilled in the art to prepare the compounds of the invention. Further information can be obtained from the examples.

The principles of the preparation processes detailed above are known in principle from the literature for similar compounds and can be adapted easily by the person skilled in the art to the preparation of the compounds of the invention. Further information can be found in the examples.

It is possible by these processes, if necessary followed by purification, for example recrystallization or sublimation, to obtain the compounds of the invention comprising structures of the Formula (A), (I), (II) and preferred embodiments of these structures in high purity, preferably more than 99% (determined by means of ¹H NMR and/or HPLC).

The compounds of the invention may also have suitable substituents, for example by relatively long alkyl groups (about 4 to 20 carbon atoms), especially branched alkyl groups, or optionally substituted aryl groups, for example xylyl, mesityl or branched terphenyl or quaterphenyl groups, which bring about solubility in standard organic solvents, for example toluene or xylene, at room temperature in a sufficient concentration soluble, in order to be able to process the compounds from solution. These soluble compounds are of particularly good suitability for processing from solution, for example by printing methods. In addition, it should be emphasized that the compounds of the invention comprising at least one structure of the formula (I) and/or formula (II) already have enhanced solubility in these solvents.

The compounds of the invention may also be mixed with a polymer. It is likewise possible to incorporate these compounds covalently into a polymer. This is especially possible with compounds substituted by reactive leaving groups such as bromine, iodine, chlorine, boronic acid or boronic ester, or by reactive polymerizable groups such as olefins or oxetanes. These may find use as monomers for production of corresponding oligomers, dendrimers or polymers. The oligomerization or polymerization is preferably effected via the halogen functionality or the boronic acid functionality or via the polymerizable group. It is additionally possible to crosslink the polymers via groups of this kind. The compounds of the invention and polymers may be used in the form of a crosslinked or uncrosslinked layer.

The invention therefore further provides oligomers, polymers or dendrimers containing one or more of the above-detailed structures of the Formula (A), (I), (II) and preferred embodiments of these structures or compounds of the invention, wherein one or more bonds in the compounds of the invention or in the structures of the Formula (A), (I), (II) and preferred embodiments of these structures to the polymer, oligomer or dendrimer are present. According to the linkage of the structures of the Formula (A), (I), (II) and preferred embodiments of these structures or of the compounds, these therefore form a side chain of the oligomer or polymer or are bonded within the main chain. The polymers, oligomers or dendrimers may be conjugated, partly conjugated or nonconjugated. The oligomers or polymers may be linear, branched or dendritic. For the repeat units of the compounds of the invention in oligomers, dendrimers and polymers, the same preferences apply as described above.

For preparation of the oligomers or polymers, the monomers of the invention are homopolymerized or copolymerized with further monomers. Preference is given to copolymers wherein the units of Formula (A), (I) and/or (II) or the preferred embodiments recited above and hereinafter are present to an extent of 0.01 to 99.9 mol%, preferably 5 to 90 mol%, more preferably 20 to 80 mol%. Suitable and preferred comonomers which form the polymer base skeleton are chosen from fluorenes (for example according to EP 842208 or WO 2000/022026), spirobifluorenes (for example according to EP 707020, EP 894107 or WO 2006/061181), paraphenylenes (for example according to WO 92/18552), carbazoles (for example according to WO 2004/070772 or WO 2004/113468), thiophenes (for example according to EP 1028136), dihydrophenanthrenes (for example according to WO 2005/014689), cis- and trans-indenofluorenes (for example according to WO 2004/041901 or WO 2004/113412), ketones (for example according to WO 2005/040302), phenanthrenes (for example according to WO 2005/104264 or WO 2007/017066) or else a plurality of these units. The polymers, oligomers and dendrimers may contain still further units, for example hole transport units, especially those based on triarylamines, and/or electron transport units.

Additionally of particular interest are compounds of the invention which feature a high glass transition temperature. In this connection, preference is given especially to compounds of the invention comprising structures of the general Formula (A), (I) and/or (II) or the preferred embodiments recited above and hereinafter which have a glass transition temperature of at least 70°C, more preferably of at least 110°C, even more preferably of at least 125°C, still even more preferably of at least 140°C, and especially preferably of at least 150°C, determined in accordance with DIN EN ISO 11357-2 (2014).

For the processing of the compounds of the invention from the liquid phase, for example by spin-coating or by printing methods, formulations of the compounds of the invention are required. These formulations may, for example, be solutions, dispersions or emulsions. For this purpose, it may be preferable to use mixtures of two or more solvents. Suitable and preferred solvents are, for example, toluene, anisole, o-, m- or p-xylene, methyl benzoate, mesitylene, tetralin, veratrole, THF, methyl-THF, THP, chlorobenzene, dioxane, phenoxytoluene, especially 3-phenoxytoluene, (-)-fenchone, 1,2,3,5-tetramethylbenzene, 1,2,4,5-tetramethylbenzene, 1-methylnaphthalene, 2-methylbenzothiazole, 2-phenoxyethanol, 2-pyrrolidinone, 3-methylanisole, 4-methylanisole, 3,4-dimethylanisole, 3,5-dimethylanisole, acetophenone, α-terpineol, benzothiazole, butyl benzoate, cumene, cyclohexanol, cyclohexanone, cyclohexylbenzene, decalin, dodecylbenzene, ethyl benzoate, indane, methyl benzoate, NMP, p-cymene, phenetole, 1,4-diisopropylbenzene, dibenzyl ether, diethylene glycol butyl methyl ether, triethylene glycol butyl methyl ether, diethylene glycol dibutyl ether, triethylene glycol dimethyl ether, diethylene glycol monobutyl ether, tripropylene glycol dimethyl ether, tetraethylene glycol dimethyl ether, 2-isopropylnaphthalene, pentylbenzene, hexylbenzene, heptylbenzene, octylbenzene, 1,1-bis(3,4-dimethylphenyl)ethane, hexamethylindane or mixtures of these solvents.

The present invention therefore further provides a formulation comprising a compound of the invention and at least one further compound. The further compound may, for example, be a solvent, especially one of the abovementioned solvents or a mixture of these solvents. The further compound may alternatively be at least one further organic or inorganic compound which is likewise used in the electronic device, for example an emitting compound, especially a phosphorescent dopant, and/or a further matrix material. This further compound may also be polymeric.

The present invention therefore still further provides a composition comprising a compound of the invention and at least one further organically functional material. Functional materials are generally the organic or inorganic materials introduced between the anode and cathode. Preferably, the organic functional material is selected from the group consisting of fluorescent emitters, phosphorescent emitters, TADF emitters, host materials, matrix materials, electron transport materials, electron injection materials, hole conductor materials, hole injection materials, n-dopants, wide band gap materials, electron blocking materials and hole blocking materials.

The present invention therefore also relates to a composition comprising at least one compound comprising structures of Formula (A), (I) and/or (II) or the preferred embodiments recited above and hereinafter and at least one further matrix material. According to a particular aspect of the present invention, the further matrix material has hole-transporting properties.

The present invention further provides a composition comprising at least one compound comprising at least one structure of Formula (A), (I) and/or (II) or the preferred embodiments recited above and hereinafter and at least one wide band gap material, a wide band gap material being understood to mean a material in the sense of the disclosure of US 7,294,849. These systems exhibit particularly advantageous performance data in electroluminescent devices.

Preferably, the additional compound may have a band gap of 2.5 eV or more, preferably 3.0 eV or more, very preferably of 3.5 eV or more. One way of calculating the band gap is via the energy levels of the highest occupied molecular orbital (HOMO) and the lowest unoccupied molecular orbital (LUMO).

Molecular orbitals, especially also the highest occupied molecular orbital (HOMO) and the lowest unoccupied molecular orbital (LUMO), the energy levels thereof and the energy of the lowest triplet state T₁ and that of the lowest excited singlet state S₁ of the materials are determined via quantum-chemical calculations. For calculation of organic substances without metals, an optimization of geometry is first conducted by the "Ground State/Semi-empirical/Default Spin/AM1/Charge 0/Spin Singlet" method. Subsequently, an energy calculation is effected on the basis of the optimized geometry. This is done using the "TD-SCF/DFT/Default Spin/B3PW91" method with the "6-31G(d)" basis set (charge 0, spin singlet). For metal-containing compounds, the geometry is optimized via the "Ground State/ Hartree-Fock/Default Spin/LanL2MB/Charge 0/Spin Singlet" method. The energy calculation is effected analogously to the above-described method for the organic substances, except that the "LanL2DZ" basis set is used for the metal atom and the "6-31G(d)" basis set for the ligands. The HOMO energy level HEh or LUMO energy level LEh is obtained from the energy calculation in Hartree units. This is used to determine the HOMO and LUMO energy levels in electron volts, calibrated by cyclic voltammetry measurements, as follows: $HOMO \left(eV\right) = \left(\left(HEh*27.212\right)-0.9899\right) / 1.1206$ $LUMO \left(eV\right) = \left(\left(LEh*27.212\right)-2.0041\right) / 1.385$

These values are to be regarded as HOMO and LUMO energy levels of the materials in the context of this application.

The lowest triplet state T₁ is defined as the energy of the triplet state having the lowest energy, which is apparent from the quantum-chemical calculation described.

The lowest excited singlet state S₁ is defined as the energy of the excited singlet state having the lowest energy, which is apparent from the quantum-chemical calculation described.

The method described herein is independent of the software package used and always gives the same results. Examples of frequently utilized programs for this purpose are "Gaussian09W' (Gaussian Inc.) and Q-Chem 4.1 (Q-Chem, Inc.).

The present invention also relates to a composition comprising at least one compound comprising structures of Formula (A), (I) and/or (II) or the preferred embodiments recited above and hereinafter and at least one phosphorescent emitter, the term "phosphorescent emitter" also being understood to mean phosphorescent dopants.

A dopant in a system comprising a matrix material and a dopant is understood to mean that component having the smaller proportion in the mixture. Correspondingly, a matrix material in a system comprising a matrix material and a dopant is understood to mean that component having the greater proportion in the mixture.

Preferred phosphorescent dopants for use in matrix systems, preferably mixed matrix systems, are the preferred phosphorescent dopants specified hereinafter.

The term "phosphorescent dopants" typically encompasses compounds where the emission of light is effected through a spin-forbidden transition, for example a transition from an excited triplet state or a state having a higher spin quantum number, for example a quintet state.

Suitable phosphorescent compounds (= triplet emitters) are especially compounds which, when suitably excited, emit light, preferably in the visible region, and also contain at least one atom of atomic number greater than 20, preferably greater than 38 and less than 84, more preferably greater than 56 and less than 80, especially a metal having this atomic number. Preferred phosphorescence emitters used are compounds containing copper, molybdenum, tungsten, rhenium, ruthenium, osmium, rhodium, iridium, palladium, platinum, silver, gold or europium, especially compounds containing iridium or platinum. In the context of the present invention, all luminescent compounds containing the abovementioned metals are regarded as phosphorescent compounds.

Examples of the above-described emitters can be found in the applications WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960 and the as yet unpublished applications EP 13004411.8, EP 14000345.0, EP 14000417.7 and EP 14002623.8. In general, all phosphorescent complexes as used for phosphorescent OLEDs according to the prior art and as known to those skilled in the art in the field of organic electroluminescence are suitable, and the person skilled in the art will be able to use further phosphorescent complexes without exercising inventive skill.

Explicit examples of phosphorescent dopants are adduced in the following table:

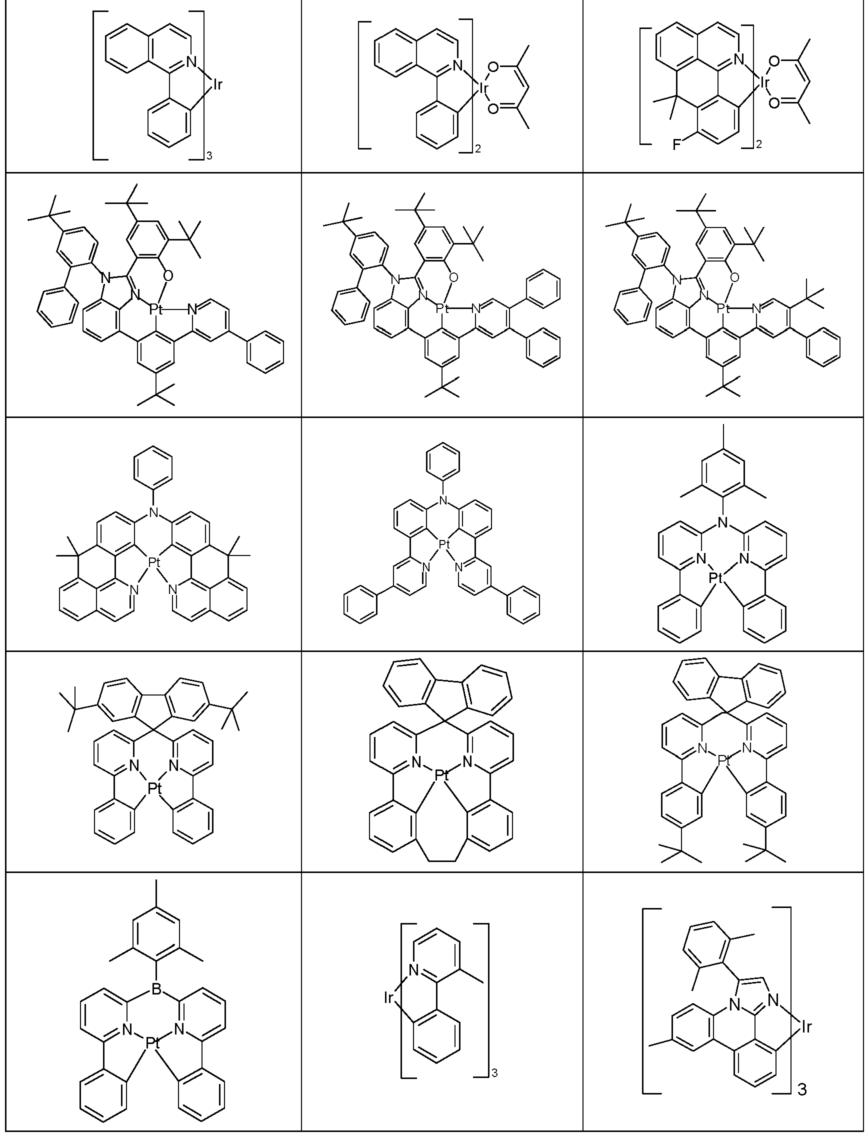

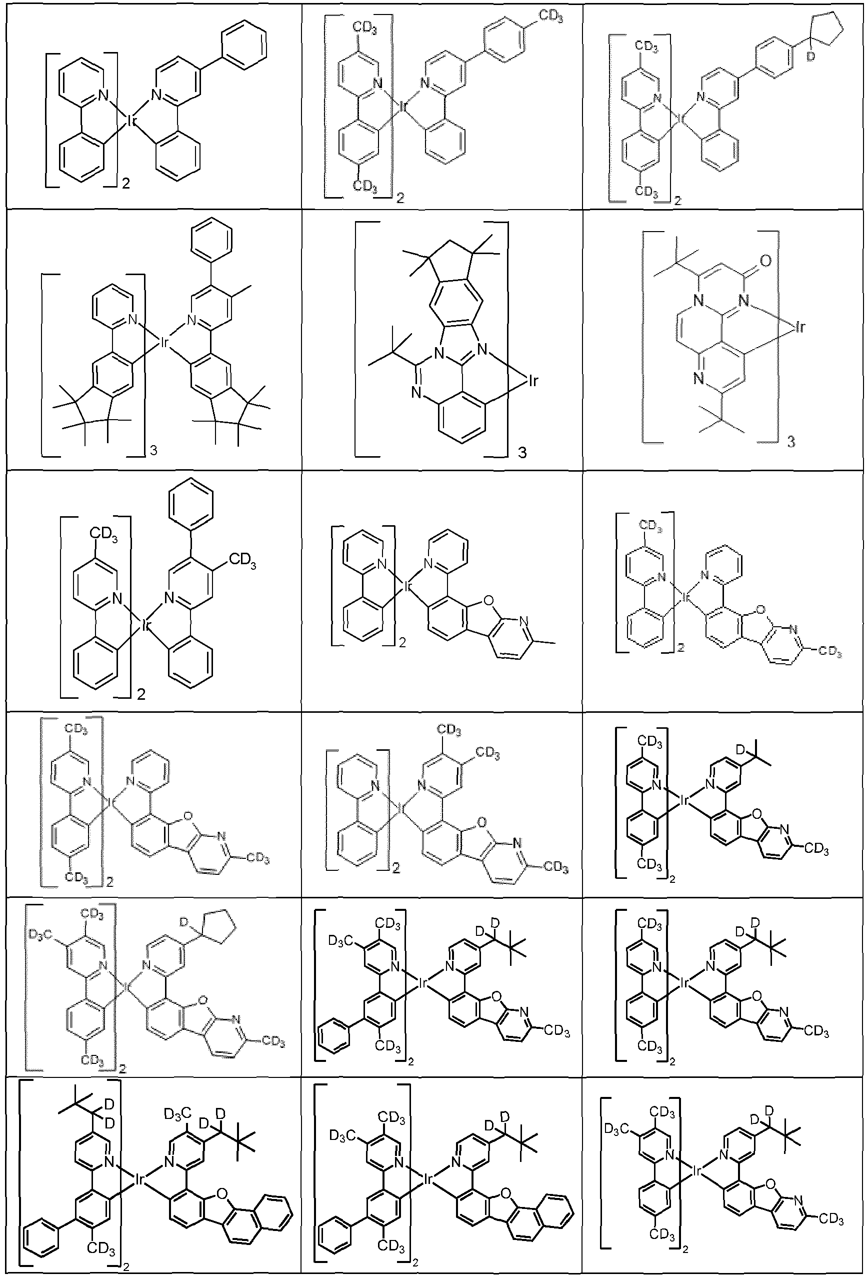

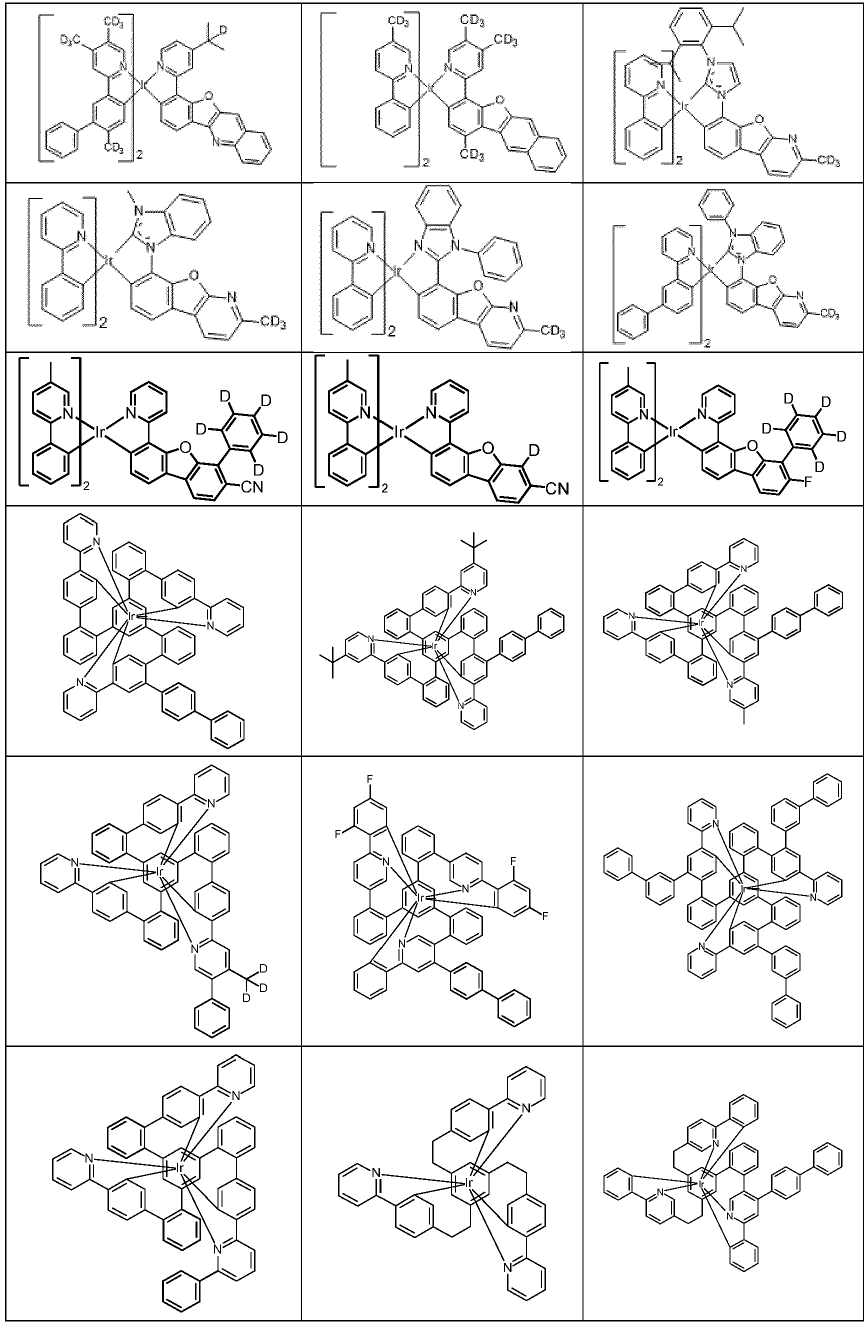

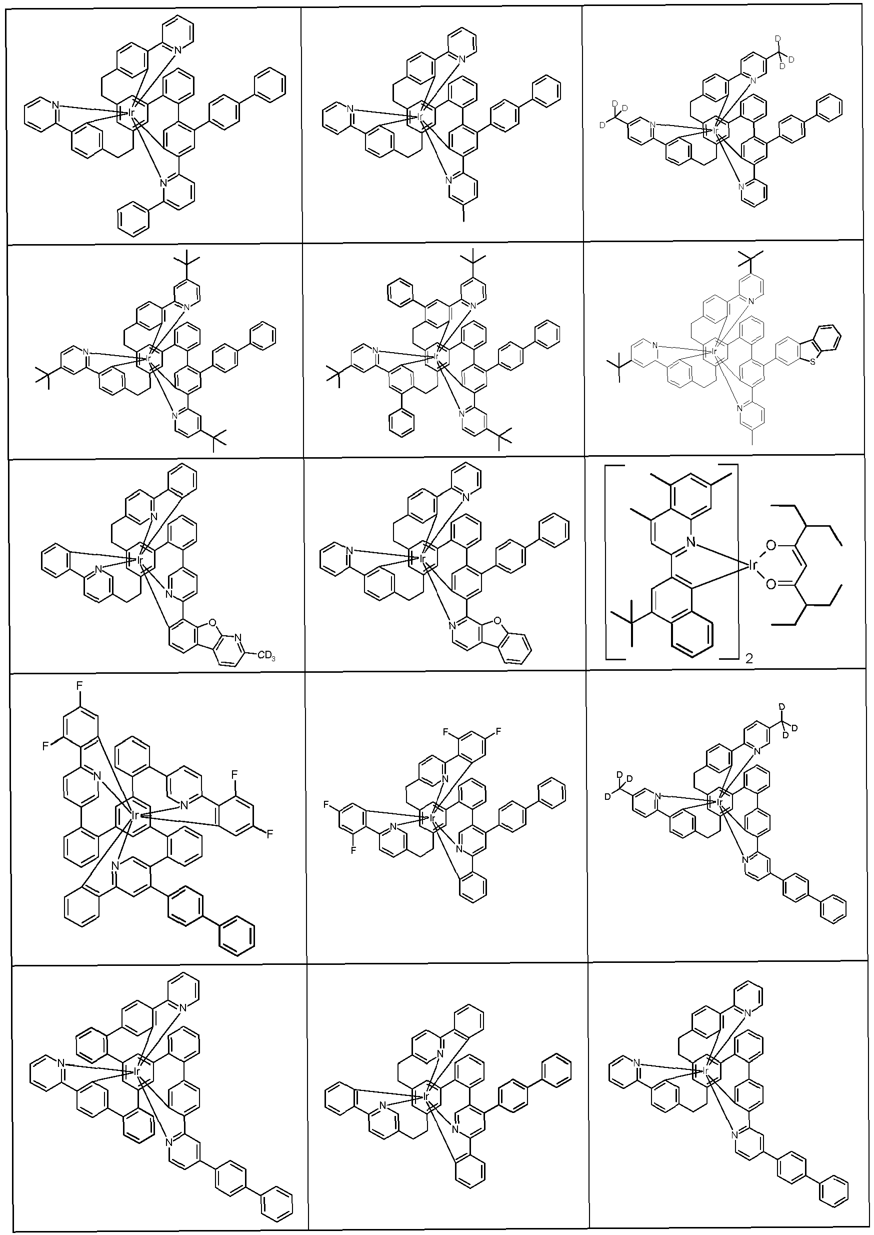

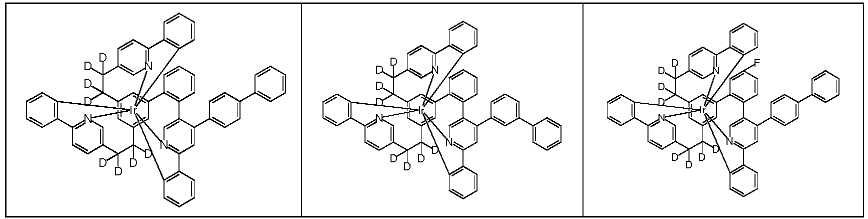

The above-described compound comprising structures of the Formula (A), (I) and/or (II) or the above-detailed preferred embodiments can preferably be used as active component in an electronic device. An electronic device is understood to mean any device comprising anode, cathode and at least one layer between anode and cathode, said layer comprising at least one organic or organometallic compound. The electronic device of the invention thus comprises anode, cathode and at least one layer in between containing at least one compound comprising structures of the Formula (A), (I) and/or (II). Preferred electronic devices here are selected from the group consisting of organic electroluminescent devices (OLEDs, PLEDs), organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), organic electrical sensors, light-emitting electrochemical cells (LECs), organic laser diodes (O-lasers) and organic plasmon emitting devices (D. M. Koller et al., Nature Photonics 2008, 1-4), preferably organic electroluminescent devices (OLEDs, PLEDs), especially phosphorescent OLEDs, containing at least one compound comprising structures of the Formula (A), (I) and/or (II) in at least one layer. Particular preference is given to organic electroluminescent devices. Active components are generally the organic or inorganic materials introduced between the anode and cathode, for example charge injection, charge transport or charge blocking materials, but especially emission materials and matrix materials.

The present invention further provides a use of a compound of the invention in an electronic device, preferably as a matrix material, a hole blocking material, an electron injection material and/or an electron transport material, more preferably as a hole blocking material, an electron injection material and/or an electron transport material.

A preferred embodiment of the invention is organic electroluminescent devices. The organic electroluminescent device comprises cathode, anode and at least one emitting layer. Apart from these layers, it may comprise still further layers, for example in each case one or more hole injection layers, hole transport layers, hole blocking layers, electron transport layers, electron injection layers, exciton blocking layers, electron blocking layers, charge generation layers and/or organic or inorganic p/n junctions. At the same time, it is possible that one or more hole transport layers are p-doped, for example with metal oxides such as MoO₃ or WO₃ or with (per)fluorinated electron-deficient aromatic systems, and/or that one or more electron transport layers are n-doped. It is likewise possible for interlayers to be introduced between two emitting layers, these having, for example, an exciton-blocking function and/or controlling the charge balance in the electroluminescent device. However, it should be pointed out that not necessarily every one of these layers need be present.

In this case, it is possible for the organic electroluminescent device to contain an emitting layer, or for it to contain a plurality of emitting layers. If a plurality of emission layers are present, these preferably have several emission maxima between 380 nm and 750 nm overall, such that the overall result is white emission; in other words, various emitting compounds which may fluoresce or phosphoresce are used in the emitting layers. Especially preferred are three-layer systems where the three layers exhibit blue, green and orange or red emission (for the basic construction see, for example, WO 2005/011013), or systems having more than three emitting layers. The system may also be a hybrid system wherein one or more layers fluoresce and one or more other layers phosphoresce.

In a preferred embodiment of the invention, the organic electroluminescent device contains the compound of the invention comprising structures of Formula (A), (I) and/or (II) or the above-detailed preferred embodiments as matrix material, preferably as electron transport matrix material, in one or more emitting layers, preferably in combination with a further matrix material, preferably a hole transport matrix material. In a further preferred embodiment of the invention, the further matrix material is an electron-transporting compound. In yet a further preferred embodiment, the further matrix material is a compound having a large band gap which is not involved to a significant degree, if at all, in the hole and electron transport in the layer. An emitting layer comprises at least one emitting compound.

Suitable matrix materials which can be used in combination with the compounds of Formula (A), (I) and/or (II) or according to the preferred embodiments are aromatic ketones, aromatic phosphine oxides or aromatic sulphoxides or sulphones, for example according to WO 2004/013080, WO 2004/093207, WO 2006/005627 or WO 2010/006680, triarylamines, especially monoamines, for example according to WO 2014/015935, carbazole derivatives, e.g. CBP (N,N-biscarbazolylbiphenyl) or the carbazole derivatives disclosed in WO 2005/039246, US 2005/0069729, JP 2004/288381, EP 1205527 or WO 2008/086851, indolocarbazole derivatives, for example according to WO 2007/063754 or WO 2008/056746, indenocarbazole derivatives, for example according to WO 2010/136109 and WO 2011/000455, azacarbazole derivatives, for example according to EP 1617710, EP 1617711, EP 1731584, JP 2005/347160, bipolar matrix materials, for example according to WO 2007/137725, silanes, for example according to WO 005/111172, azaboroles or boronic esters, for example according to WO 2006/117052, triazine derivatives, for example according to WO 2010/015306, WO 2007/063754 or WO 2008/056746, zinc complexes, for example according to EP 652273 or WO 2009/062578, diazasilole or tetraazasilole derivatives, for example according to WO 2010/054729, diazaphosphole derivatives, for example according to WO 2010/054730, bridged carbazole derivatives, for example according to US 2009/0136779, WO 2010/050778, WO 2011/042107, WO 2011/088877 or WO 2012/143080, triphenylene derivatives, for example according to WO 2012/048781, lactams, for example according to WO 2011/116865, WO 2011/137951 or WO 2013/064206, or 4-spirocarbazole derivatives, for example according to WO 2014/094963 or the as yet unpublished application EP 14002104.9. It is likewise possible for a further phosphorescent emitter which emits at a shorter wavelength than the actual emitter to be present as co-host in the mixture.

Preferred co-host materials are triarylamine derivatives, especially monoamines, indenocarbazole derivatives, 4-spirocarbazole derivatives, lactams and carbazole derivatives.

It may also be preferable to use a plurality of different matrix materials as a mixture, especially at least one electron transport matrix material and at least one hole transport matrix material. Preference is likewise given to the use of a mixture of a charge-transporting matrix material and an electrically inert matrix material having no significant involvement, if any, in the charge transport, as described, for example, in WO 2010/108579.

It is further preferable to use a mixture of two or more triplet emitters together with a matrix. In this case, the triplet emitter having the shorter-wave emission spectrum serves as co-matrix for the triplet emitter having the longer-wave emission spectrum.

More preferably, a compound of the invention comprising structures of Formula (A), (I) and/or (II), in a preferred embodiment, can be used as matrix material in an emission layer of an organic electronic device, especially in an organic electroluminescent device, for example in an OLED or OLEC. In this case, the matrix material containing compound comprising structures of Formula (A), (I) and/or (II) or the preferred embodiments recited above and hereinafter is present in the electronic device in combination with one or more dopants, preferably phosphorescent dopants.

The proportion of the matrix material in the emitting layer in this case is between 50.0% and 99.9% by volume, preferably between 80.0% and 99.5% by volume, and more preferably between 92.0% and 99.5% by volume for fluorescent emitting layers and between 85.0% and 97.0% by volume for phosphorescent emitting layers.

Correspondingly, the proportion of the dopant is between 0.1% and 50.0% by volume, preferably between 0.5% and 20.0% by volume, and more preferably between 0.5% and 8.0% by volume for fluorescent emitting layers and between 3.0% and 15.0% by volume for phosphorescent emitting layers.

An emitting layer of an organic electroluminescent device may also comprise systems comprising a plurality of matrix materials (mixed matrix systems) and/or a plurality of dopants. In this case too, the dopants are generally those materials having the smaller proportion in the system and the matrix materials are those materials having the greater proportion in the system. In individual cases, however, the proportion of a single matrix material in the system may be less than the proportion of a single dopant.

In a further preferred embodiment of the invention, the compound comprising structures of Formula (A), (I) and/or (II) or the preferred embodiments recited above and hereinafter is used as a component of mixed matrix systems. The mixed matrix systems preferably comprise two or three different matrix materials, more preferably two different matrix materials. Preferably, in this case, one of the two materials is a material having hole-transporting properties and the other material is a material having electron-transporting properties. The desired electron-transporting and hole-transporting properties of the mixed matrix components may, however, also be combined mainly or entirely in a single mixed matrix component, in which case the further mixed matrix component(s) fulfill(s) other functions. The two different matrix materials may be present in a ratio of 1:50 to 1:1, preferably 1:20 to 1:1, more preferably 1:10 to 1:1 and most preferably 1:4 to 1:1. Preference is given to using mixed matrix systems in phosphorescent organic electroluminescent devices. One source of more detailed information about mixed matrix systems is the application WO 2010/108579.

The present invention further provides an electronic device, preferably an organic electroluminescent device, comprising one or more compounds of the invention and/or at least one oligomer, polymer or dendrimer of the invention in one or more electron transport layers, as electron transport compound.

The present invention further provides an electronic device, preferably an organic electroluminescent device, comprising one or more compounds of the invention and/or at least one oligomer, polymer or dendrimer of the invention in one or more hole-blocking layers, as hole-blocking compound.

The electronic device preferably comprises an electron transport region. The electron transport region may comprise one or more layers conducting electrons, such as hole-blocking layers, electron transport layers and/or electron injection layers. All of the layers of the electron transport region may comprise one or more compounds of the invention. In addition thereto, all of the layers of the electron transport region may consist of one or more compounds of the invention. In a further embodiment of the invention, one layer of the electron transport region may comprise one or more compounds of the invention and another layer of the electron transport region does not comprise a compound of the invention, preferably.

Additionally preferred is an electronic device, especially an organic electroluminescent device, which is characterized in that the device comprises at least one hole-blocking layer and at least one electron transport layer, wherein the hole-blocking layer is directly adjacent to an emitting layer and the at least one hole-blocking layer comprises a compound of the invention and the electron transport layer comprises at least one electron transport compound being different to a compound of the invention (a compound which does not comprise a group according to formula (R^{a}-1) to (R^{a}-33) as mentioned above and below). In a preferred embodiment, the device comprises a hole-blocking layer and at least one electron transport layer, wherein the hole-blocking layer is directly adjacent to an emitting layer and the hole-blocking consists of one or more compounds of the invention and the electron transport layer consists of one or more electron transport compounds being different to a compound of the invention (a compound which does not comprise a group according to formula (R^{a}-1) to (R^{a}-33) as mentioned above and below).

Preferred cathodes are metals having a low work function, metal alloys or multilayer structures composed of various metals, for example alkaline earth metals, alkali metals, main group metals or lanthanoids (e.g. Ca, Ba, Mg, Al, In, Mg, Yb, Sm, etc.). Additionally suitable are alloys composed of an alkali metal or alkaline earth metal and silver, for example an alloy composed of magnesium and silver. In the case of multilayer structures, in addition to the metals mentioned, it is also possible to use further metals having a relatively high work function, for example Ag, in which case combinations of the metals such as Mg/Ag, Ca/Ag or Ba/Ag, for example, are generally used. It may also be preferable to introduce a thin interlayer of a material having a high dielectric constant between a metallic cathode and the organic semiconductor. Examples of useful materials for this purpose are alkali metal or alkaline earth metal fluorides, but also the corresponding oxides or carbonates (e.g. LiF, Li₂O, BaF₂, MgO, NaF, CsF, Cs₂CO₃, etc.). Likewise useful for this purpose are organic alkali metal complexes, e.g. Liq (lithium quinolinate). The layer thickness of this layer is preferably between 0.5 and 5 nm.

Preferred anodes are materials having a high work function. Preferably, the anode has a work function of greater than 4.5 eV versus vacuum. Firstly, metals having a high redox potential are suitable for this purpose, for example Ag, Pt or Au. Secondly, metal/metal oxide electrodes (e.g. Al/Ni/NiOₓ, Al/PtOₓ) may also be preferred. For some applications, at least one of the electrodes has to be transparent or partly transparent in order to enable either the irradiation of the organic material (O-SC) or the emission of light (OLED/PLED, O-laser). Preferred anode materials here are conductive mixed metal oxides. Particular preference is given to indium tin oxide (ITO) or indium zinc oxide (IZO). Preference is further given to conductive doped organic materials, especially conductive doped polymers, for example PEDOT, PANI or derivatives of these polymers. It is further preferable when a p-doped hole transport material is applied to the anode as hole injection layer, in which case suitable p-dopants are metal oxides, for example MoO₃ or WO₃, or (per)fluorinated electron-deficient aromatic systems. Further suitable p-dopants are HAT-CN (hexacyanohexaazatriphenylene) or the compound NPD9 from Novaled.

Such a layer simplifies hole injection into materials having a low HOMO, i.e. a large HOMO in terms of magnitude.

In the further layers, it is generally possible to use any materials as used according to the prior art for the layers, and the person skilled in the art is able, without exercising inventive skill, to combine any of these materials with the materials of the invention in an electronic device.

The device is correspondingly (according to the application) structured, contact-connected and finally hermetically sealed, since the lifetime of such devices is severely shortened in the presence of water and/or air.

Additionally preferred is an electronic device, especially an organic electroluminescent device, which is characterized in that one or more layers are coated by a sublimation process. In this case, the materials are applied by vapour deposition in vacuum sublimation systems at an initial pressure of typically less than 10⁻⁵ mbar, preferably less than 10⁻⁶ mbar. It is also possible that the initial pressure is even lower or even higher, for example less than 10⁻⁷ mbar.

Preference is likewise given to an electronic device, especially an organic electroluminescent device, which is characterized in that one or more layers are coated by the OVPD (organic vapour phase deposition) method or with the aid of a carrier gas sublimation. In this case, the materials are applied at a pressure between 10⁻⁵ mbar and 1 bar. A special case of this method is the OVJP (organic vapour jet printing) method, in which the materials are applied directly by a nozzle and thus structured (for example, M. S. Arnold et al., Appl. Phys. Lett. 2008, 92, 053301).

Preference is additionally given to an electronic device, especially an organic electroluminescent device, which is characterized in that one or more layers are produced from solution, for example by spin-coating, or by any printing method, for example screen printing, flexographic printing, offset printing or nozzle printing, but more preferably LITI (light-induced thermal imaging, thermal transfer printing) or inkjet printing. For this purpose, soluble compounds are needed, which are obtained, for example, through suitable substitution.

The electronic device, especially the organic electroluminescent device can also be produced as a hybrid system by applying one or more layers from solution and applying one or more other layers by vapour deposition. For example, it is thus possible to apply an emitting layer comprising a compound of the invention comprising structures of formula (I) and/or formula (II) and a matrix material from solution, and to apply a hole blocking layer and/or an electron transport layer thereto by vapour deposition under reduced pressure.

These methods are known in general terms to those skilled in the art and can be applied without difficulty to electronic devices, especially organic electroluminescent devices comprising compounds of the invention comprising structures of Formula (A), (I) and/or (II) or the above-detailed preferred embodiments.

The electronic devices of the invention, especially organic electroluminescent devices, are notable for one or more of the following surprising advantages over the prior art:
1. The compounds according to the invention, employed in organic electroluminescent devices, provide increased external quantum efficiency (EQE).
2. Electronic devices, especially organic electroluminescent devices, comprising compounds, oligomers, polymers or dendrimers having structures of Formula (A), (I) and/or (II) or the preferred embodiments recited above and hereinafter, as electron transport materials have excellent efficiency. More particularly, efficiency is much higher compared to analogous compounds containing no structural unit of Formula (A), (I) and/or (II).
3. The compounds according to the invention, employed in organic electroluminescent devices, provide low refractive index (RI). Low refractive indices improve light outcoupling and hence improve efficiency.
4. The compounds according to the invention are very highly suitable for use in a hole-blocking or electron-transport layer in an organic electroluminescent device. They are also suitable, in particular, for use in a hole-blocking layer which is directly adjacent to a phosphorescent or fluorescent emitting layer, since the compounds according to the invention do not extinguish the luminescence.
5. The compounds according to the invention, employed as matrix material for fluorescent or phosphorescent emitters, result in very high efficiencies and long lifetimes. This applies, in particular, if the compounds are employed as matrix material together with a further matrix material and a phosphorescent emitter.
6. The compounds, oligomers, polymers or dendrimers of the invention having structures of Formula (A), (I) and/or (II) or the preferred embodiments detailed above and hereinafter exhibit a high glass transition temperature. A high glass transition temperature enables a processing of the compounds at a high temperature and improves the lifetime of electronic devices, especially organic electroluminescent devices.
7. The compounds according to the invention, employed in organic electroluminescent devices, result in high efficiencies and in steep current/voltage curves with low use and operating voltages.
8. Electronic devices, especially organic electroluminescent devices, comprising compounds, oligomers, polymers or dendrimers having structures of Formula (A), (I) and/or (II) or the preferred embodiments recited above and hereinafter, especially as electron transport materials, have a very good lifetime.
9. The compounds, oligomers, polymers or dendrimers of the invention having structures of Formula (A), (I) and/or (II) or the preferred embodiments detailed above and hereinafter exhibit very high stability and lead to compounds having a very long lifetime.
10. With compounds, oligomers, polymers or dendrimers having structures of Formula (A), (I) and/or (II) or the preferred embodiments recited above and hereinafter, it is possible to avoid the formation of optical loss channels in electronic devices, especially organic electroluminescent devices. As a result, these devices feature a high **PL** efficiency and hence high **EL** efficiency of emitters, and excellent energy transmission of the matrices to dopants.
11. The use of compounds, oligomers, polymers or dendrimers having structures of Formula (A), (I) and/or (II) or the preferred embodiments recited above and hereinafter in layers of electronic devices, especially organic electroluminescent devices, leads to high mobility of the electron transport structures.
12. Compounds, oligomers, polymers or dendrimers having structures of Formula (A), (I) and/or (II) or the preferred embodiments recited above and hereinafter feature excellent thermal stability, and compounds having a molar mass of less than about 1200 g/mol have good sublimability.
13. Compounds, oligomers, polymers or dendrimers having structures of Formula (A), (I) and/or (II) or the preferred embodiments recited above and hereinafter have excellent glass film formation properties.
14. Compounds, oligomers, polymers or dendrimers having structures of Formula (A), (I) and/or (II) or the preferred embodiments detailed above and hereinafter form very good films from solutions.
15. The compounds, oligomers, polymers or dendrimers comprising structures of Formula (A), (I) and/or (II) or the preferred embodiments recited above and hereinafter have a surprisingly high triplet level T₁, this being particularly true of compounds which are used as electron transport materials.

These abovementioned advantages are not accompanied by a deterioration in the further electronic properties.

The inventive compounds and mixtures are suitable for use in an electronic device. An electronic device is understood to mean a device containing at least one layer containing at least one organic compound. This component may also comprise inorganic materials or else layers formed entirely from inorganic materials.

The present invention therefore further provides for the use of the inventive compounds or mixtures in an electronic device, especially in an organic electroluminescent device.

The present invention still further provides for the use of a compound of the invention and/or an oligomer, polymer or dendrimer of the invention in an electronic device as hole blocking material, electron injection material and/or electron transport material.

The present invention still further provides an electronic device comprising at least one of the above-detailed inventive compounds or mixtures. In this case, the preferences detailed above for the compound also apply to the electronic devices.

In a further embodiment of the invention, the organic electroluminescent device of the invention does not contain any separate hole injection layer and/or hole transport layer and/or hole blocking layer and/or electron transport layer, meaning that the emitting layer directly adjoins the hole injection layer or the anode, and/or the emitting layer directly adjoins the electron transport layer or the electron injection layer or the cathode, as described, for example, in WO 2005/053051. It is additionally possible to use a metal complex identical or similar to the metal complex in the emitting layer as hole transport or hole injection material directly adjoining the emitting layer, as described, for example, in WO 2009/030981.

In addition, it is possible to use the compounds of the invention in a hole blocking or electron transport layer. This is especially true of compounds of the invention which do not have a carbazole structure. These may preferably also be substituted by one or more further electron-transporting groups, for example benzimidazole groups.

In the further layers of the organic electroluminescent device of the invention, it is possible to use any materials as typically used according to the prior art. The person skilled in the art is therefore able, without exercising inventive skill, to use any materials known for organic electroluminescent devices in combination with the inventive compounds of Formula (A), (I) and/or (II) or according to the preferred embodiments.

The compounds of the invention generally have very good properties on use in organic electroluminescent devices. Especially in the case of use of the compounds of the invention in organic electroluminescent devices, the lifetime is significantly better compared to similar compounds according to the prior art. At the same time, the further properties of the organic electroluminescent device, especially the efficiency and voltage, are likewise better or at least comparable.

It should be pointed out that variations of the embodiments described in the present invention are covered by the scope of this invention. Any feature disclosed in the present invention may, unless this is explicitly ruled out, be exchanged for alternative features which serve the same purpose or an equivalent or similar purpose. Thus, any feature disclosed in the present invention, unless stated otherwise, should be considered as an example of a generic series or as an equivalent or similar feature.

All features of the present invention may be combined with one another in any manner, unless particular features and/or steps are mutually exclusive. This is especially true of preferred features of the present invention. Equally, features of non-essential combinations may be used separately (and not in combination).

It should also be pointed out that many of the features, and especially those of the preferred embodiments of the present invention, are themselves inventive and should not be regarded merely as some of the embodiments of the present invention. For these features, independent protection may be sought in addition to or as an alternative to any currently claimed invention.

The technical teaching disclosed with the present invention may be abstracted and combined with other examples.

The invention is illustrated in detail by the examples which follow, without any intention of restricting it thereby.

The person skilled in the art will be able to use the details given, without exercising inventive skill, to produce further electronic devices of the invention and hence to execute the invention over the entire scope claimed.

### Examples

### A) Synthesis examples

The syntheses which follow, unless stated otherwise, are conducted under a protective gas atmosphere in dried solvents. The reactants can be purchased from commercial sources (n-butyl lithium, trimethyl borate, tetrakis(triphenylphosphine)palladium(0)). 2,4-Diphenyl-6-(9,9'-spirobi(9H-fluoren)-2-yl)-1,3,5-triazine (CAS 1207176-84-8) can be purchased from commercial sources as well. 2,4-Diphenyl-6-(9,9'-spirobi(9H-fluoren)-4-yl)-1,3,5-triazine (1561044-71-0) can be made according to WO 2014/023388 A1.

### a) (2,7-di-tert-butyl-9,9'-spirobi(fluoren)-4-yl)boronic acid

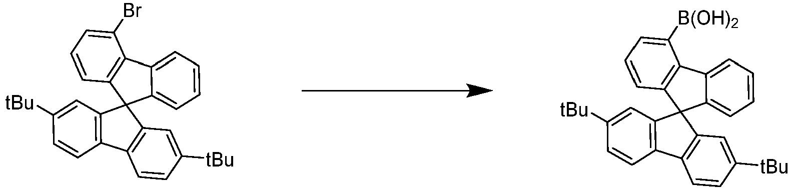

A solution of 5'-bromo-2,7-di-tert-butyl-9,9'-spirobi(fluorene) (50.0 g, 98.5 mmol) in THF (1.00 L) is cooled down to -75 °C and n-butyl lithium (47.3 mL, 118 mmol) is added dropwise. The reaction mixture is stirred for 2.5 h at -75 °C before dropwise addition of trimethylborate (14.6 mL, 128 mmol). The mixture is stirred overnight and allowed to come to room temperature. Dest. water (150 mL) is added slowly followed by the addition of HCl (2 N, 30 mL) and dilution of the mixture with ethyl acetate. After phase separation, the aqueous solution is extracted with ethyl acetate. The combined organic layers are washed with dest. water and sat. NaCl-solution and dried over Na₂SO₄, subsequently. The solvent is removed under reduced pressure and the residue is separated by chromatography (heptane:ethyl acetate = 4:1).

The yield is 23.1 g (48.9 mmol), corresponding to 50% of theory.

In an analogous manner, it is possible to obtain the following compounds:

**Table 1: Examples for Boronic Acid formation**

| | | |
|---|---|---|
| 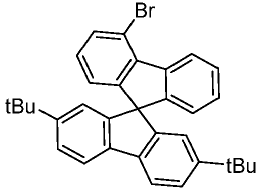 | 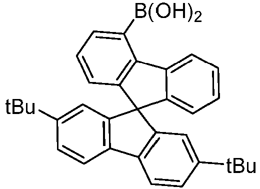 | 50% |
| 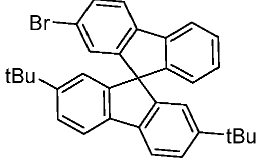 CAS 393841-81-1 | 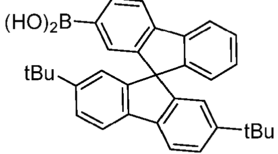 CAS 1241891-39-3 | 84% |
| 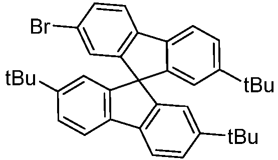 CAS 2529935-30-4 | 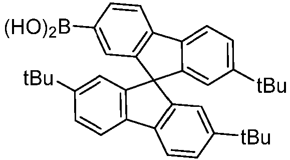 | 82% |
| 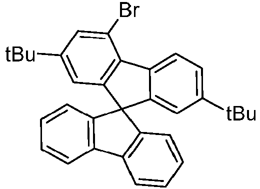 | 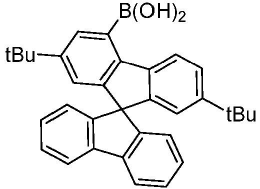 | 46% |
| 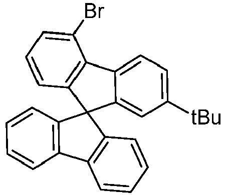 CAS 2102016-91-9 | 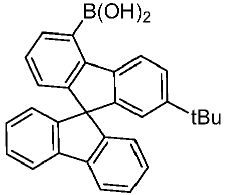 | 51% |
| 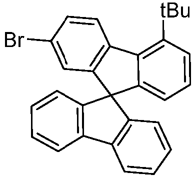 CAS 270741-67-5 | 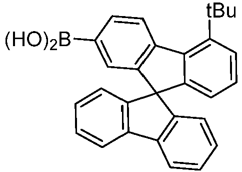 | 53% |
| 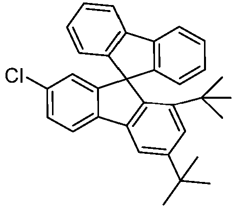 CAS 2768772-14-9 | 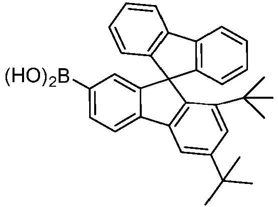 | 82% |
| 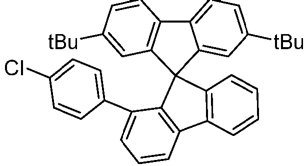 CAS 2260634-40-8 | 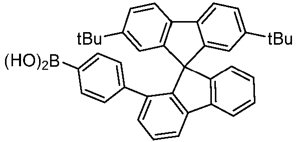 | 42% |
| 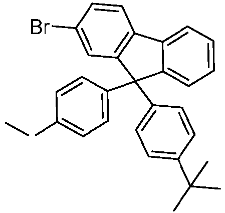 CAS 2413294-33-2 | 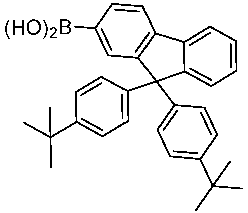 | 84% |
| 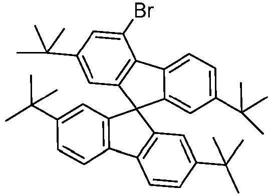 CAS 1469898-65-4 | 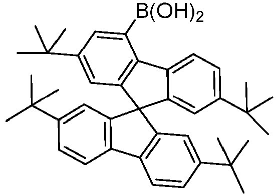 | 43% |

### b) 2-(2',7'-di-tert-butyl-9,9'-spirobi(fluoren)-2-yl)-4,6-diphenyl-1,3,5-triazine (2)

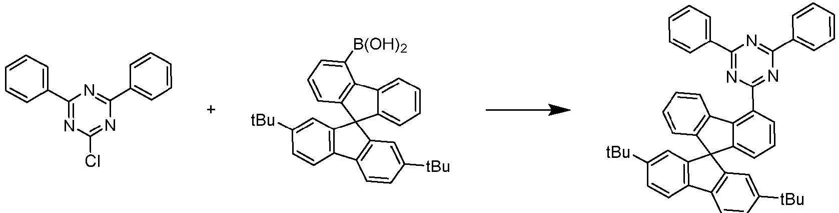

2-chloro-4,6-diphenyl-1,3,5-triazine (9.90 g, 37.0 mmol) and (2',7'-di-tert-butyl-9,9'-spiro(fluoren)4-yl)-boronic acid (19.2 g, 40.7 mmol) are solved in 1,4-dioxane (119 mL) and toluene (119 mL). The solution is warmed up to 40 °C and tetrakis(triphenylphosphine)palladium(0) (430 mg, 0.372 mmol) were added. Potassium carbonate (5.62 g, 41.0 mmol) is dissolved in dest. Water (44.2 mL) and added dropwise at 40 °C. The reaction mixture is heated to reflux for 2.5 h. After cooling down to room temperature, ethyl acetate and water are added. The aqueous layer is extracted with ethyl acetate. The combined organic layers are washed with sat. aq. NaCl-solution and dried over Na₂SO₄. The solvent is removed under reduced pressure and the residue is separated by chromatography (heptane:toluene = 1:1), followed by an additional recrystallization of the material from heptane.

The yield is 22.2 g (33.4 mmol), corresponding to 90% of theory.

In an analogous manner, it is possible to obtain the following compounds:

**Table 2: Examples for Triazine formation**

| | | | |
|---|---|---|---|
| 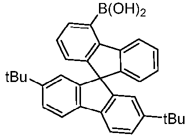 | 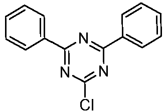 CAS 3842-55-5 | 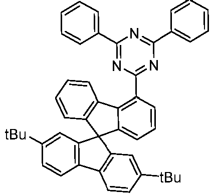 2 | 90% |
| 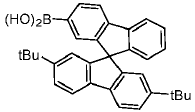 CAS 1241891-39-3 | 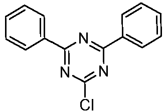 CAS 3842-55-5 | 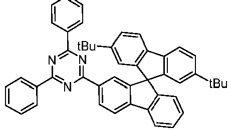 1 | 91% |
| 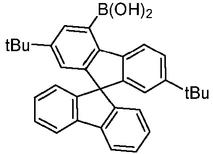 | 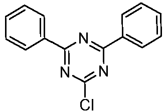 CAS 3842-55-5 | 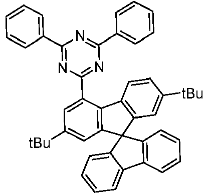 3 | 76% |
| 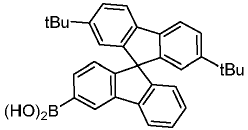 CAS 2641506-54-7 | 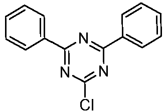 CAS 3842-55-5 | 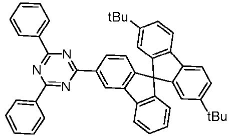 4 | 53% |
| 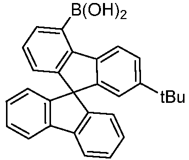 | 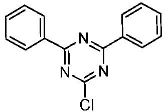 CAS 3842-55-5 | 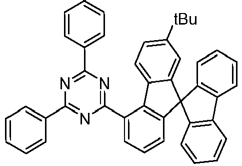 112 | 92% |
| 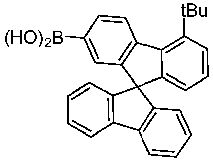 | 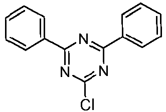 CAS 3842-55-5 | 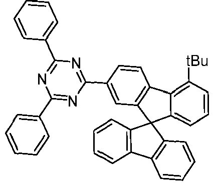 51 | 92% |
| 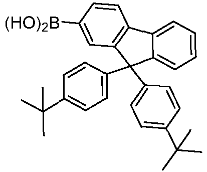 | 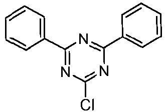 CAS 3842-55-5 | 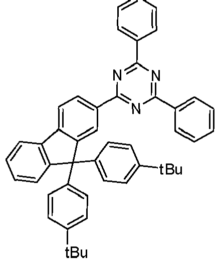 54 | 90% |
| 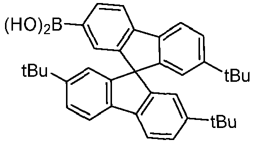 | 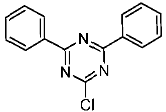 CAS 3842-55-5 | 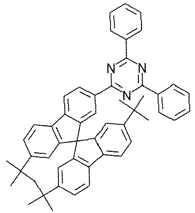 18 | 83% |
| 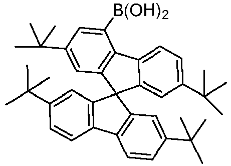 | 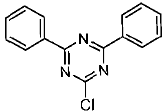 CAS 3842-55-5 | 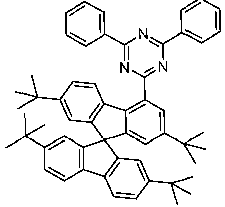 82 | 75% |
| 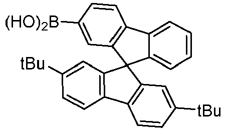 CAS 1241891-39-3 | 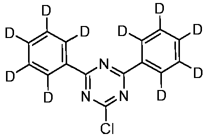 CAS 1300115-09-6 | 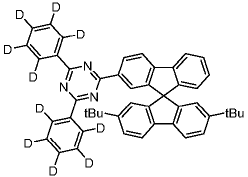 33 | 90% |
| 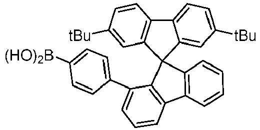 | 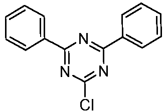 CAS 3842-55-5 | 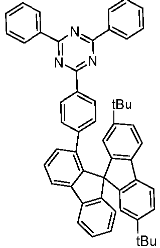 114 | 74% |
| 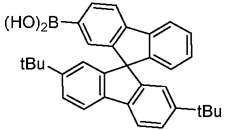 CAS 1241891-39-3 | 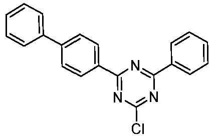 CAS 1472062-94-4 | 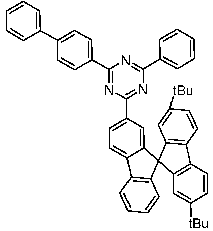 20 | 89% |
| 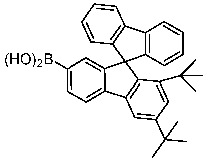 | 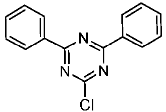 CAS 3842-55-5 | 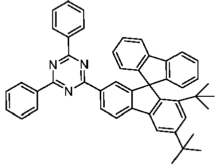 117 | 84% |
| 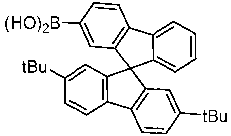 CAS 1241891-39-3 | 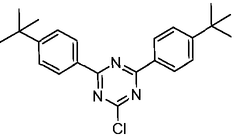 CAS 253158-13-3 | 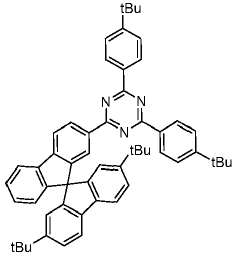 40 | 90% |
| 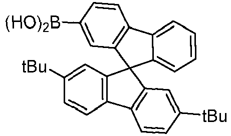 CAS 1241891-39-3 | 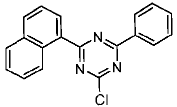 CAS 1472062-95-5 | 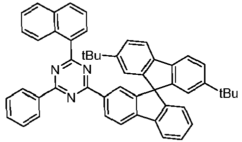 36 | 87% |
| 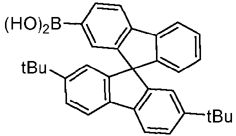 CAS 1241891-39-3 | 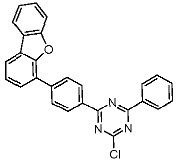 2074632-09-8 | 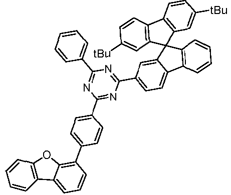 116 | 67% |
| 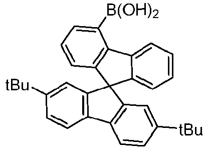 | 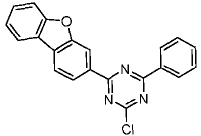 CAS 2142681-84-1 | 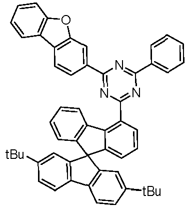 92 | 72% |
| 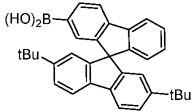 CAS 1241891-39-3 | 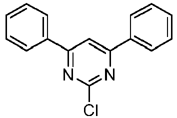 CAS 2915-16-4 | 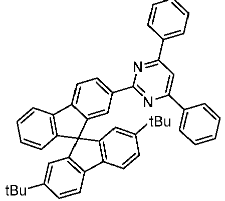 61 | 54% |

### B) Device examples

### 1) General production process for the OLEDs and characterization of the OLEDs

Glass plaques which have been coated with structured ITO (indium tin oxide) in a thickness of 50 nm form the substrates to which the OLEDs are applied.

The OLEDs basically have the following layer structure: substrate / optional interlayer (IL) / hole injection layer (HIL) / hole transport layer (HTL) / electron blocking layer (EBL) / emission layer (EML) / optional hole blocking layer (HBL) / electron transport layer (ETL) / optional electron injection layer (EIL) and finally a cathode. The cathode is formed by an aluminium layer of thickness 100 nm. The exact structure of the OLEDs can be found in the tables which follow. The structure of the OLEDs produced and the materials used for production of the OLEDs are shown in tables 3 and 4 below.

All materials are applied by thermal vapour deposition in a vacuum chamber. In this case, the emission layer consists of at least one matrix material (host material) and an emitting dopant which is added to the matrix material(s) in a particular proportion by volume by co-evaporation.

Details given in such a form as H:SEB (5 %) mean here that the material H is present in the layer in a proportion by volume of 95 % and SEB in a proportion of 5 %. Analogously, the electron transport layer may also consist of a mixture of two materials.

The OLEDs are characterized in a standard manner. For this purpose, the electroluminescence spectra, the current efficiency (CE, measured in cd/A) and the external quantum efficiency (EQE, measured in %) are determined as a function of luminance, calculated from current-voltage-luminance characteristics assuming Lambertian emission characteristics, as is the lifetime. The electroluminescence spectra are determined at a luminance of 1000 cd/m², and the CIE 1931 x and y colour coordinates are calculated therefrom. The parameter U1000 in table 5 refers to the voltage which is required for a luminance of 1000 cd/m². CE1000 and EQE1000 respectively denotes the current efficiency and external quantum efficiency that is attained at 1000 cd/m². Refractive indices are measured using a M-2000 spectroscopic ellipsometer from J. A, Woollam Co., Inc. using a Cauchy model.

### 2) Use and benefit of the inventive compounds in OLEDs

Improved efficiency in blue OLED devices is found when the recombination (emission) zone is narrow and confined close to the EML:EBL interface. Blue OLED typically make use of the cavity effect (especially in Top Emission devices) to more efficiently outcouple the generated light. Electron transport or hole blocking materials with low refractive indices can further improve light outcoupling and hence improve EQE. Electron transport or hole blocking materials with high glass transition temperature Tg allow an easier and more reliable processing with less formation of defect devices. The glass transition temperature Tg is determined in accordance with DIN EN ISO 11357-2 (2014).

### 2a) Use of compounds of the invention as electron transport material in the electron transport layer of OLEDs

When the inventive compounds ETM-3, ETM 4 and ETM-5 are used as electron transport material, a better efficiency (examples E1.1, E2.1 and E2.2) is achieved than with the substances ETM-1 and ETM-2 according to the prior art (examples C1 to C2). When the inventive compounds ETM-3 to ETM-6 are used as hole blocking material, significantly better efficiency and a better driving voltage are observed (examples, E4.1, E4.2 and E4.3) compared to ETM-2 according to prior art (example C4). Additional performance improvement can be observed when ETM-3 to ETM-6 are used as hole blocking material (examples E5 and E6) as better efficiency and better driving voltage are observed compared to ETM-1 and ETM-2 according to the prior art (examples C5 to C6).

Table 5 collates the results for the performance data of the OLEDs from examples. Additional material parameters can be found in Table 6.

**Table 3: Structure of the OLEDs**

| ***Ex.*** | ***HIL*** | ***HTL*** | ***EBL*** | ***EML*** | ***HBL*** | ***ETL*** | ***EIL*** |
|---|---|---|---|---|---|---|---|
| | Thickness / nm | Thickness / nm | Thickness / nm | Thickness / nm | Thickness / nm | Thickness / nm | Thickness / nm |
| C1 | HTM: p-Dopant (5%) | HTM | EBM | H:SEB(5%) | - | **ETM-1**:LiQ(50%) | LiQ |
| | 10 nm | 180 nm | 10 nm | 20 nm | | 30 nm | 1 nm |
| E1. 1 | HTM: p-Dopant (5%) | HTM | EBM | H:SEB(5%) | - | **ETM-3**:LiQ(50%) | LiQ |
| | 10 nm | 180 nm | 10 nm | 20 nm | | 30 nm | 1 nm |
| C2 | HTM: p-Dopant (5%) | HTM | EBM | H:SEB(5%) | - | **ETM-2**:LiQ(50%) | LiQ |
| | 10 nm | 180 nm | 10 nm | 20 nm | | 30 nm | 1 nm |
| E2. 1 | HTM: p-Dopant (5%) | HTM | EBM | H:SEB(5%) | - | **ETM-4**:LiQ(50%) | LiQ |
| | 10 nm | 180 nm | 10 nm | 20 nm | | 30 nm | 1 nm |
| E2. 2 | HTM: p-Dopant (5%) | HTM | EBM | H:SEB(5%) | - | **ETM-5**:LiQ(50%) | LiQ |
| | 10 nm | 180 nm | 10 nm | 20 nm | | 30 nm | 1 nm |
| C3 | HTM: p-Dopant (5%) | HTM | EBM | H:SEB(5%) | **ETM-1** | **ETM**:LiQ(50%) | LiQ |
| | 10 nm | 180 nm | 10 nm | 20 nm | 10 nm | 30 nm | 1 nm |
| E3. 1 | HTM: p-Dopant (5%) | HTM | EBM | H:SEB(5%) | **ETM-3** | **ETM**:LiQ(50%) | LiQ |
| | 10 nm | 180 nm | 10 nm | 20 nm | 10 nm | 30 nm | 1 nm |
| C4 | HTM: p-Dopant (5%) | HTM | EBM | H:SEB(5%) | **ETM-2** | **ETM**:LiQ(50%) | LiQ |
| | 10 nm | 180 nm | 10 nm | 20 nm | 10 nm | 30 nm | 1 nm |
| E4. 1 | HTM: p-Dopant (5%) | HTM | EBM | H:SEB(5%) | **ETM-4** | **ETM**:LiQ(50%) | LiQ |
| | 10 nm | 180 nm | 10 nm | 20 nm | 10 nm | 30 nm | 1 nm |
| E4. 2 | HTM: p-Dopant (5%) | HTM | EBM | H:SEB(5%) | **ETM-5** | **ETM**:LiQ(50%) | LiQ |
| | 10 nm | 180 nm | 10 nm | 20 nm | 10 nm | 30 nm | 1 nm |
| E4. 3 | HTM: p-Dopant (5%) | HTM | EBM | H:SEB(5%) | **ETM-6** | **ETM**:LiQ(50%) | LiQ |
| | 10 nm | 180 nm | 10 nm | 20 nm | 10 nm | 30 nm | 1 nm |
| C5 | HTM: p-Dopant (5%) | HTM | EBM | H:SEB(5%) | **ETM-1** | **ETM-1**:LiQ(50%) | LiQ |
| | 10 nm | 180 nm | 10 nm | 20 nm | 10 nm | 30 nm | 1 nm |
| E5. 1 | HTM: p-Dopant (5%) | HTM | EBM | H:SEB(5%) | **ETM-3** | **ETM-3**:LiQ(50%) | LiQ |
| | 10 nm | 180 nm | 10 nm | 20 nm | 10 nm | 30 nm | 1 nm |
| C6 | HTM: p-Dopant (5%) | HTM | EBM | H:SEB(5%) | **ETM-2** | **ETM-2**:LiQ(50%) | LiQ |
| | 10 nm | 180 nm | 10 nm | 20 nm | 10 nm | 30 nm | 1 nm |
| E6. 1 | HTM: p-Dopant (5%) | HTM | EBM | H:SEB(5%) | **ETM-4** | **ETM-4**:LiQ(50%) | LiQ |
| | 10 nm | 180 nm | 10 nm | 20 nm | 10 nm | 30 nm | 1 nm |
| E6. 2 | HTM: p-Dopant (5%) | HTM | EBM | H:SEB(5%) | **ETM-5** | **ETM-5**:LiQ(50%) | LiQ |
| | 10 nm | 180 nm | 10 nm | 20 nm | 10 nm | 30 nm | 1 nm |

In Table 3, C denotes a Comparative Example and E denotes an Example of the present invention.

**Table 4: Structural formulae of the materials for the OLEDs**

| | | |
|---|---|---|
| 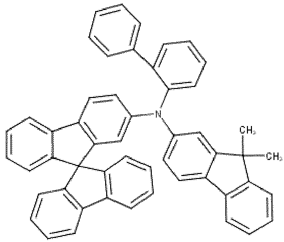 | 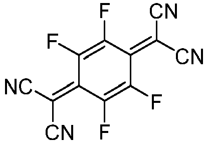 | 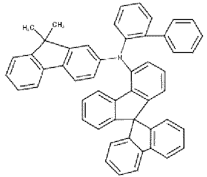 |
| HTM | P-dopant | EBM |
| 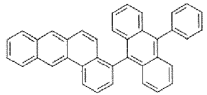 | 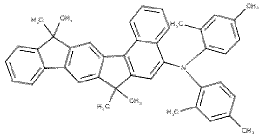 | 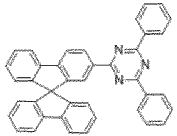 |
| H | SEB | ETM-1 (reference material) |
| 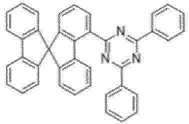 | 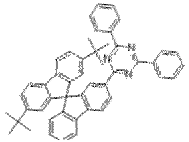 | 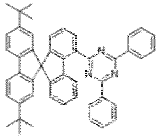 |
| ETM-2 (reference material) | ETM-3 (material of this invention) | ETM-4 (material of this invention) |
| 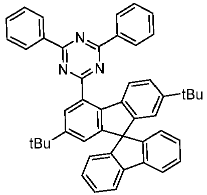 | 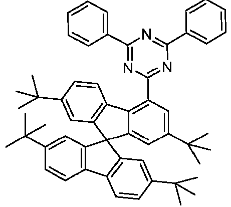 | 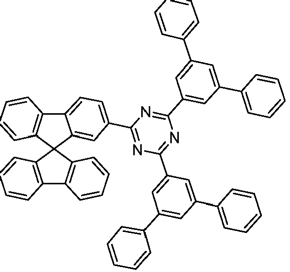 |
| ETM-5 (material of this invention) | ETM-6 (material of this invention) | ETM |
| 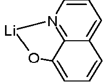 | | |
| LiQ | | |

**Table 5: Data of the OLEDs**

| **Examples** | **U1000 (V)** | **CE1000 (cd/A)** | **EQE 1000 (cd/m2)** | **CIE x/y at 1000 cd/m²** |
|---|---|---|---|---|
| C1 | 3.63 | 8.65 | 9.30 | 0.14/0.17 |
| E1.1 | 3.74 | 8.57 | 9.60 | 0.14/0.11 |
| C2 | 3.70 | 8.67 | 9.48 | 0.14/0.14 |
| E2.1 | 3.96 | 8.96 | 9.52 | 0.14/0.14 |
| E2.2 | 4.07 | 8.82 | 9.52 | 0.14/0.14 |
| C3 | 3.72 | 8.82 | 9.26 | 0.14/0.17 |
| E3.1 | 3.88 | 9.08 | 10.71 | 0.14/0.11 |
| C4 | 3.82 | 8.48 | 9.52 | 0.14/0.14 |
| E4.1 | 3.71 | 9.34 | 10.83 | 0.14/0.14 |
| E4.2 | 3.71 | 9.75 | 11.01 | 0.14/0.14 |
| E4.3 | 3.78 | 9.57 | 11.24 | 0.14/0.14 |
| C5 | 3.77 | 8.73 | 9.35 | 0.14/0.14 |
| E5.1 | 3.78 | 9.15 | 10.93 | 0.14/0.1 |
| C6 | 3.80 | 9.13 | 9.91 | 0.14/0.14 |
| E6.1 | 4.22 | 9.53 | 10.83 | 0.14/0.14 |
| E6.2 | 4.43 | 9.36 | 10.65 | 0.14/0.14 |

**Table 6: Data of the materials**

| **Ex.** | **RI (620 nm)** | **RI (550 nm)** | **RI (460 nm)** | **Tg (°C)** |
|---|---|---|---|---|
| ETM-1 | 1.76 | 1.79 | 1.83 | 134 °C |
| ETM-2 | 1.74 | 1.77 | 1.80 | 132 °C |
| ETM-3 | 1.68 | 1.71 | 1.74 | 145 °C |
| ETM-4 | 1.67 | 1.70 | 1.72 | 141 °C |
| ETM-5 | 1.68 | 1.70 | 1.73 | 145 °C |
| ETM-6 | 1.63 | 1.65 | 1.68 | 138 °C |

The present Examples and Comparative Examples show an unexpected lowering in refractive indices (RI) over the whole range of wavelength as shown in Table 6. In addition thereto, the efficiency and external quantum efficiency are improved. Furthermore, the Examples E4.1, E4.2 and E4.3 show that also an improvement in operating voltage can be achieved with the compounds of the present invention.

## Claims

1. Compound comprising structures of the Formula (A), preferably compound according to Formula (A), 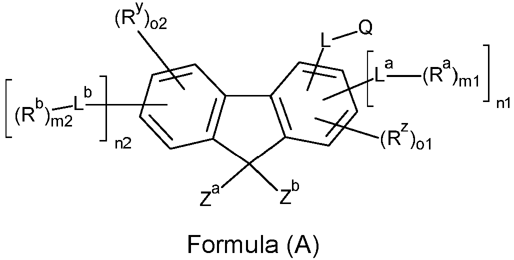 where the symbols used are as follows:
Z^{a} is Ar^{a} or R^{x}, wherein Ar^{a} is an aromatic or heteroaromatic ring system which has 5 to 40 aromatic ring atoms, each of which may be substituted by one or more R^{x} radicals, wherein the groups Z^{a} and Z^{b} may form a mono- or polycyclic aliphatic, heteroaliphatic, aromatic or heteroaromatic ring system with one another;
Z^{b} is Ar^{b} or R^{w}, wherein Ar^{b} is an aromatic or heteroaromatic ring system which has 5 to 40 aromatic ring atoms, each of which may be substituted by one or more R^{w} radicals, wherein the groups Z^{a} and Z^{b} may form a mono- or polycyclic aliphatic, heteroaliphatic, aromatic or heteroaromatic ring system with one another;
Q is an electron transport group;
L, L^{a}, L^{b} is the same or different at each instance and is a single bond, C(=O) or an aromatic or heteroaromatic ring system having 5 to 24 aromatic ring atoms, which may be substituted by one or more radicals R;
R, R^{w}, R^{x}, R^{y}, R^{z} is the same or different at each instance and is H, D, F, Cl, Br, I, CN, Si(R¹)₃, a straight-chain alkyl, alkoxy or thioalkyl group having 1 to 40 C atoms or a branched or cyclic alkyl, alkoxy or thio¬alkyl¬ group having 3 to 40 C atoms, each of which may be substituted by one or more radi¬cals R¹, where in each case one or more non-adjacent CH₂ groups may be replaced by -R¹C=CR¹-, -C≡C-, Si(R¹)₂, C=O, C=S, C=NR¹, - C(=O)O-, -C(=O)NR¹-, NR¹, P(=O)(R¹), -O-, -S-, SO or SO₂ and where one or more H atoms may be replaced by D, F, Cl, Br, I, CN or NO₂, or an aromatic or heteroaromatic ring system having 5 to 40 aromatic ring atoms, which may in each case be sub¬stituted by one or more radicals R¹, or an aryloxy or heteroaryloxy group having 5 to 40 aromatic ring atoms, which may be substituted by one or more radicals R¹, or an aralkyl group having 5 to 40 aromatic ring atoms, which may in each case be substituted by one or more radicals R¹, or a combination of these systems; at the same time two or more adjacent substituents R, R^{w}, R^{x}, R^{y}, R^{z} may also form a ring system, preferably a mono- or polycyclic, aliphatic or aromatic ring system with one another, which may be substituted by one or more radicals R¹;
R¹ is the same or different at each instance and is H, D, a straight-chain alkyl, alkoxy or thioalkoxy group having 1 to 40 carbon atoms or a branched or cyclic alkyl, alkoxy or thioalkoxy group having 3 to 40 carbon atoms, each of which may be substituted by one or more R² radicals, where one or more non-adjacent CH₂ groups may be replaced by -R²C=CR²-, -C=C-, Si(R²)₂, C=O, C=S, C=NR², -C(=O)O-, -C(=O)NR²-, NR², P(=O)(R²), -O-, -S-, SO or SO₂ and where one or more hydrogen atoms may be replaced by D, F, Cl, Br, I, CN or NO₂, or an aromatic or heteroaromatic ring system which has 5 to 40 aromatic ring atoms, each of which may be substituted by one or more R² radicals, or an aryloxy or heteroaryloxy group which has 5 to 40 aromatic ring atoms, each of which may be substituted by one or more R² radicals, or a combination of these systems; at the same time, two or more adjacent R¹ substituents may also form a ring system, preferably a mono- or polycyclic aliphatic or aromatic ring system with one another;
R² is the same or different at each instance and is H, D, F or an aliphatic hydrocarbyl radical having 1 to 20 carbon atoms, in which one or more hydrogen atoms may be replaced by D or F, or an aromatic or heteroaromatic ring system having 5 to 30 carbon atoms, in which one or more hydrogen atoms may be replaced by D or F; at the same time, two or more adjacent R² substituents together may also form a ring system, preferably a mono- or polycyclic, aliphatic or aromatic ring system;
R^{a},R^{b} is the same or different at each instance and selected from (R^{a}-1) to (R^{a}-33): 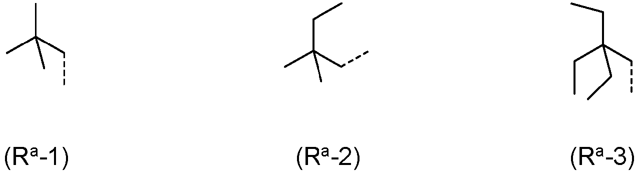 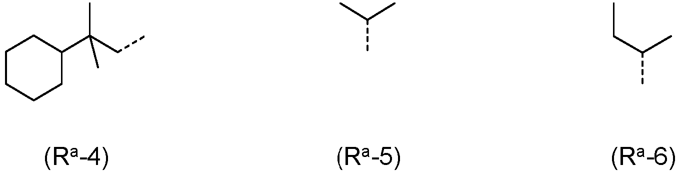 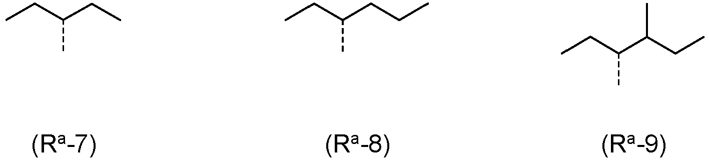 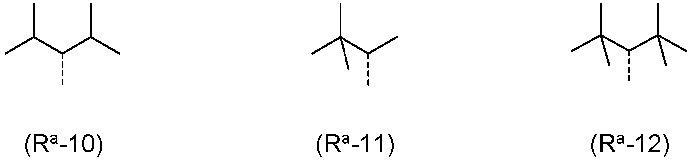 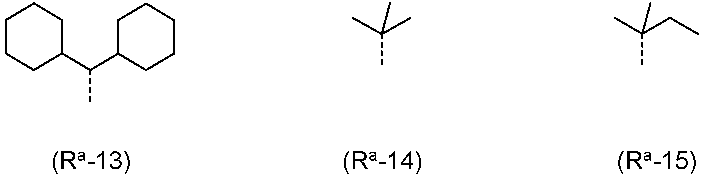 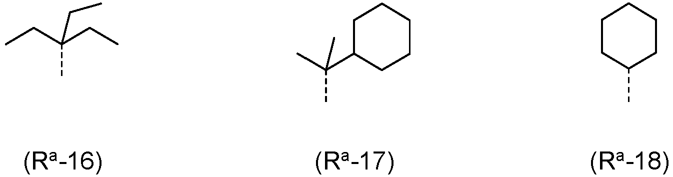 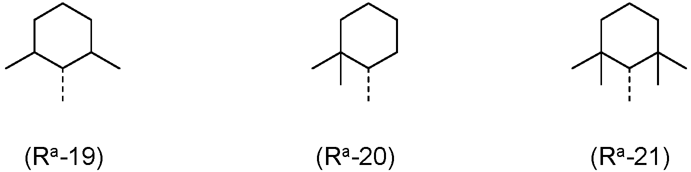 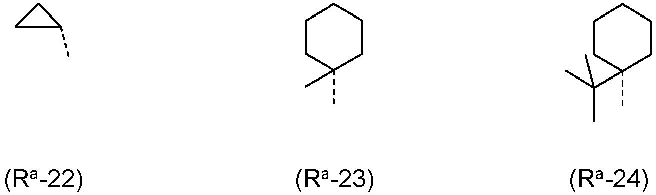 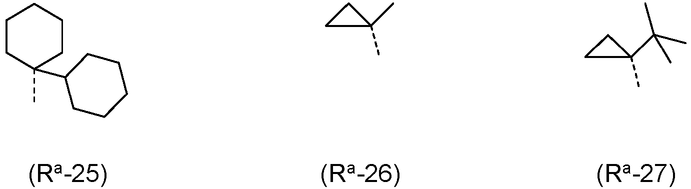 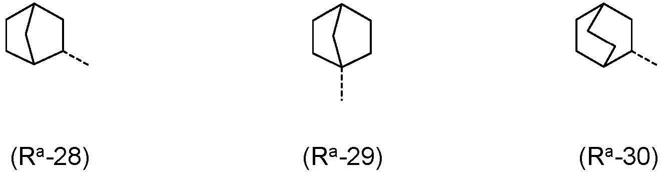 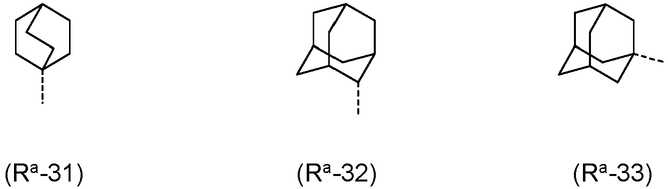 wherein one or more H atoms may be replaced by D and the dotted line denotes the bond to the corresponding group
m1, m2 is the same or different at each instance and is 0, 1, 2, 3 or 4, preferably 1, 2, 3 or 4, more preferably 1 or 2;
n2, o2 is the same or different at each instance and is 0, 1, 2, 3 or 4, preferably 0, 1 or 2;
n1, o1 is the same or different at each instance and is 0, 1, 2 or 3, preferably 0, 1 or 2;
the sum of n1 + o1 is 0, 1, 2 or 3, preferably 0, 1 or 2;
the sum of n2 + o2 is 0, 1, 2, 3 or 4, preferably 0, 1 or 2; and
**characterized in that**:
the compound comprises two, three or four structure elements of formulae (R^{a}-1) to (R^{a}-33).

2. Compound according to Claim 1, **characterized in that** the compound comprising structures of the Formula (I) or Formula (II), preferably compound according to Formula (I) or Formula (II), 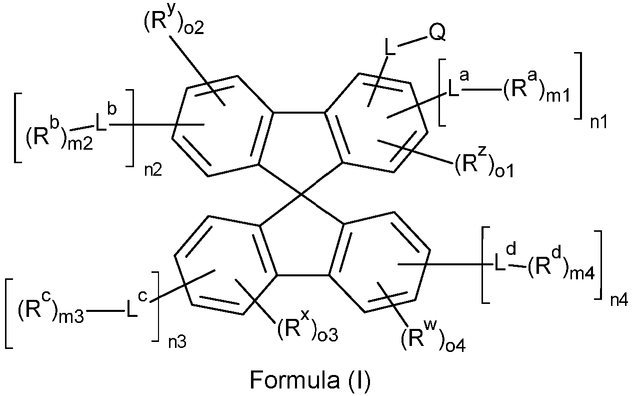 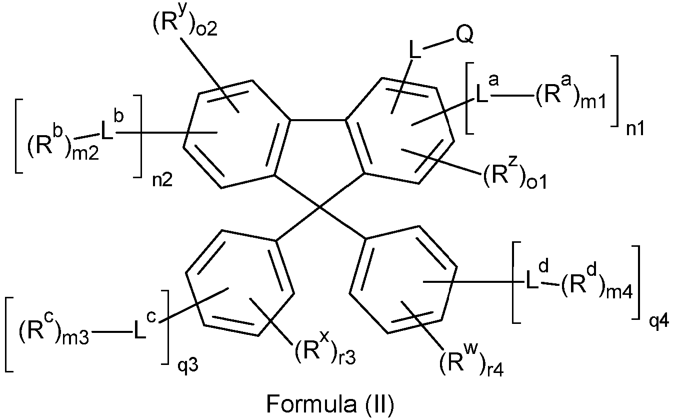 in which
the symbols Q, L, L^{a}, L^{b}, R, R^{w}, R^{x}, R^{y}, R^{z}, R^{a}, R^{b}, m1, m2, n1, n2, o1 and o2 have the definition given in Claim 1 and
L^{c}, L^{d} is the same or different at each instance and is a single bond, C(=O) or an aromatic or heteroaromatic ring system having 5 to 24 aromatic ring atoms, which may be substituted by one or more radicals R;
R^{c}, R^{d} is the same or different at each instance and selected from (R^{a}-1) to (R^{a}-33) as given in Claim 1;
m3, m4 is the same or different at each instance and is 0, 1, 2, 3 or 4, preferably 1, 2, 3 or 4, more preferably 1 or 2;
n3, n4, o3, o4 is the same or different at each instance and is 0, 1, 2, 3 or 4, preferably 0, 1 or 2;
q3, q4, r3, r4 is the same or different at each instance and is 0, 1, 2, 3, 4 or 5, preferably 0, 1 or 2;
the sum of n3 + o3 is 0, 1, 2, 3 or 4, preferably, 0, 1 or 2;
the sum of n4 + o4 is 0, 1, 2, 3 or 4, preferably, 0, 1 or 2;
the sum of q3 + r3 is 0, 1, 2, 3, 4 or 5, preferably, 0, 1 or 2;
the sum of q4 + r4 is 0, 1, 2, 3, 4 or 5, preferably, 0, 1 or 2; and
wherein the compound comprises two, three or four, structure elements of formulae (R^{a}-1) to (R^{a}-33).

3. Compound according to Claim 1 or 2, **characterized in that** the compound comprises at least one of the structures of the Formulae (Ia), (Ib), (Ic), (Id), (IIa), (IIb), (IIc) and/or (IId), preferably compound according to Formulae (Ia), (Ib), (Ic), (Id), (IIa), (IIb), (IIc) and/or (IId), 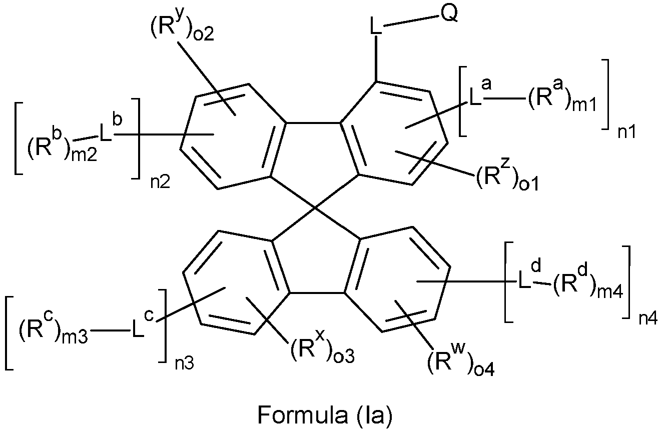 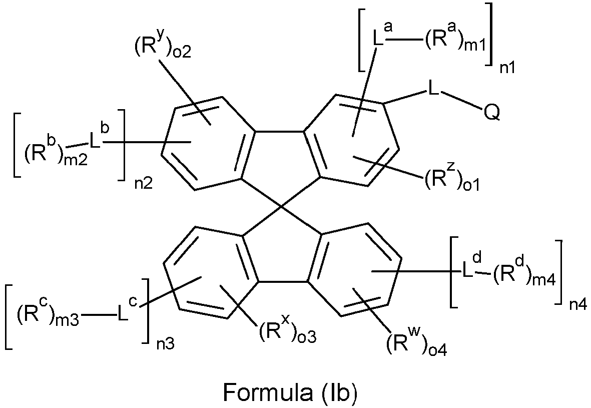 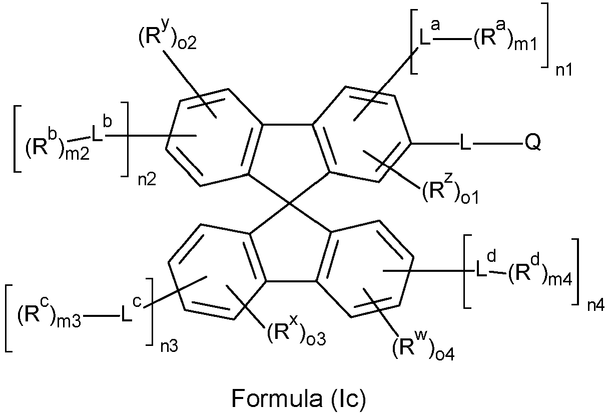 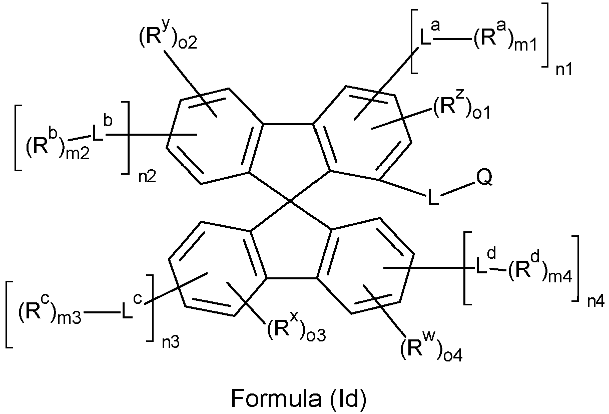 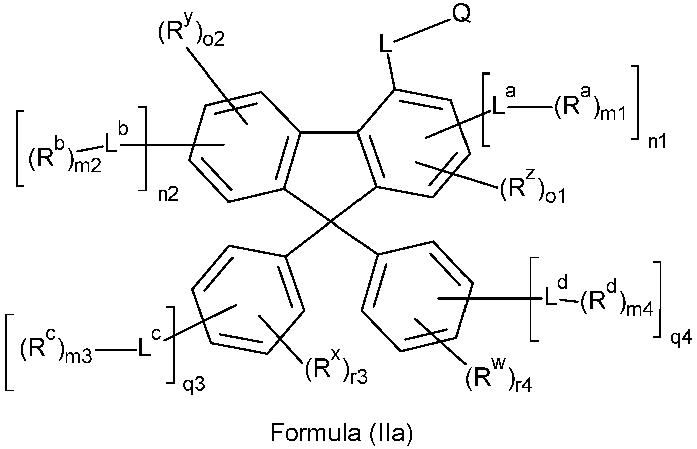 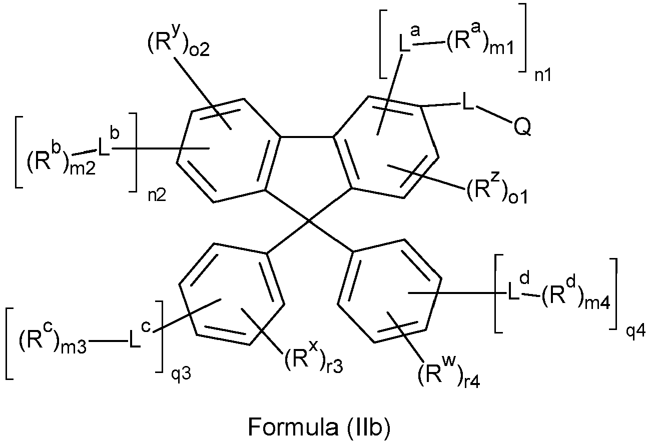 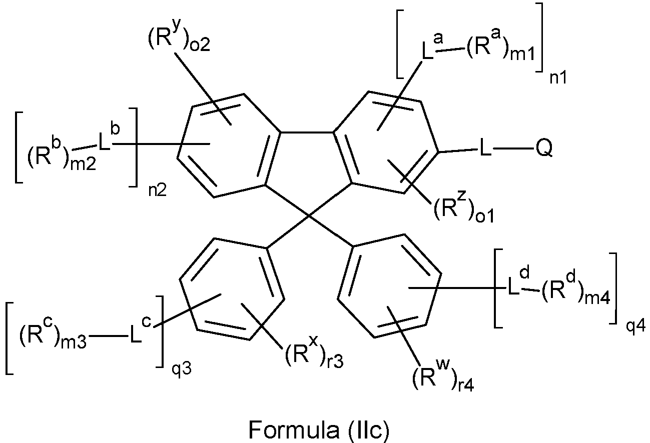 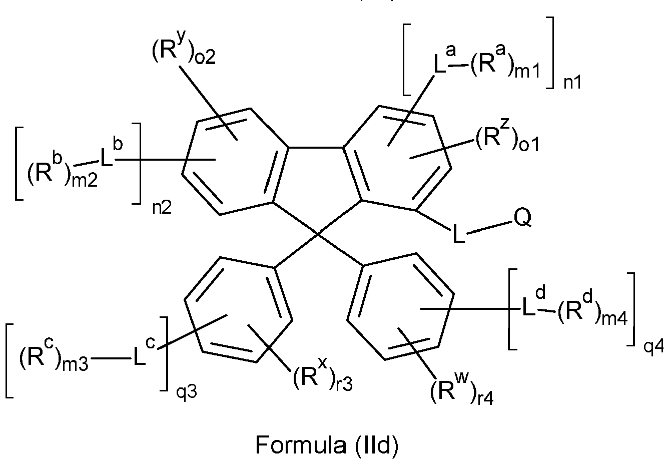 in which the symbols Q, L, L^{a}, L^{b}, L^{c}, L^{d}, R^{w}, R^{x}, R^{y}, R^{z}, R^{a}, R^{b}, R^{c}, R^{d}, m1, m2, m3, m4, n1, n2, n3, n4, o1, o2, o3, o4, q3, q4, r3 and r4 have the definition set out in Claim 1 or 2, wherein the Structures (la) and (Ic) are preferred.

4. Compound according to one or more of Claims 2 or 3, **characterized in that** the sum of the indices m1, m2, m3 and m4 is in the range of 1 to 6, preferably 1 to 4, more preferably 2 or 3.

5. Compound according to one or more of Claims 1 to 4, **characterized in that** the compound comprises at least one of the structures of the Formulae (1-1), (I-2), (I-3), (I-4), (I-5), (I-6), (II-1), (II-2), (II-3), (II-4), (II-5) and/or (II-6), preferably compound according to Formulae (1-1), (I-2), (I-3), (I-4), (I-5), (I-6), (II-1), (II-2), (II-3), (II-4), (II-5) and/or (II-6), 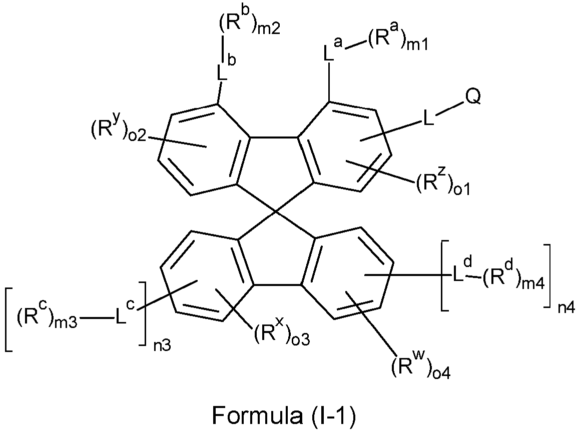 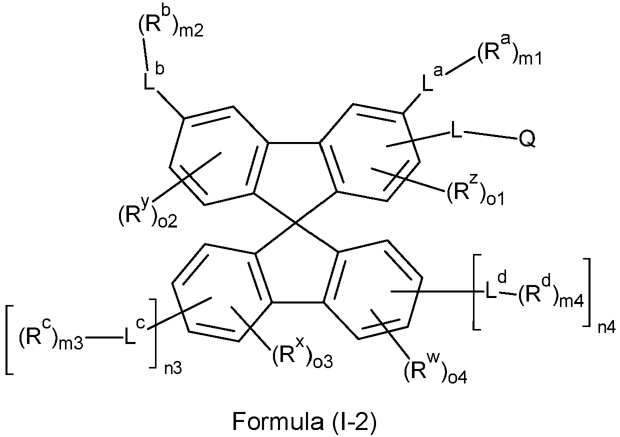 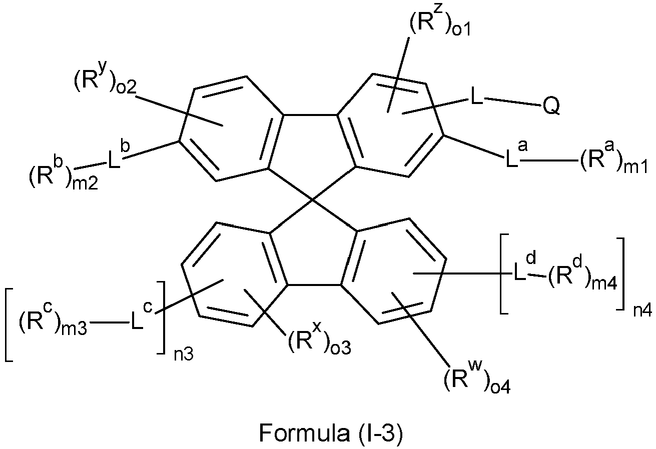 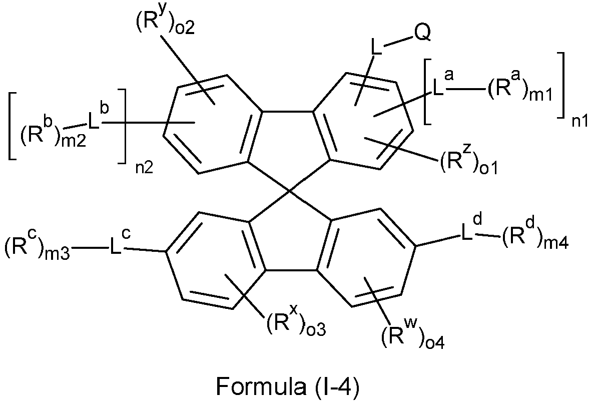 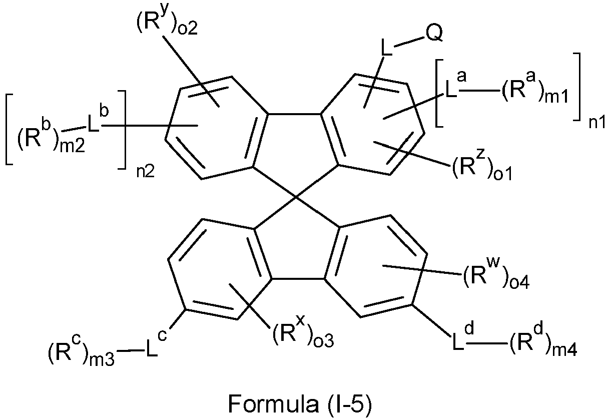 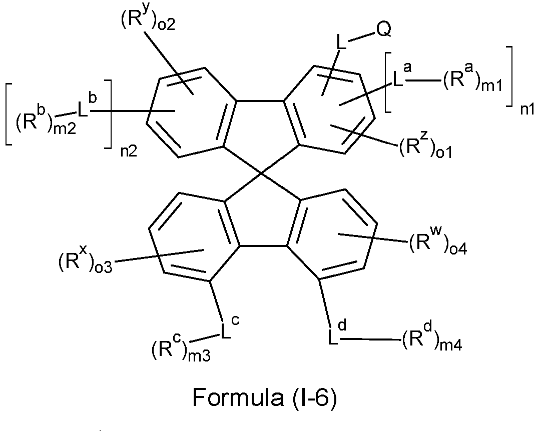 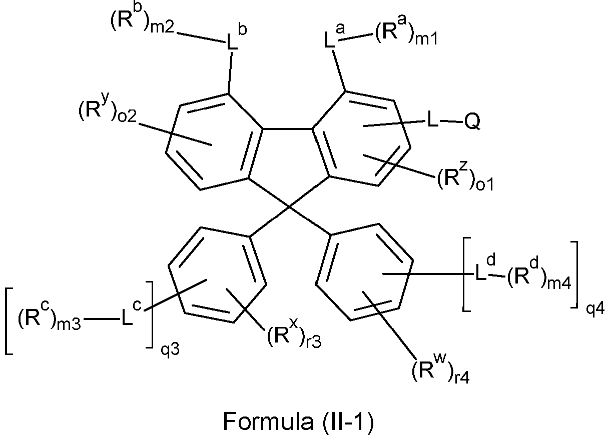 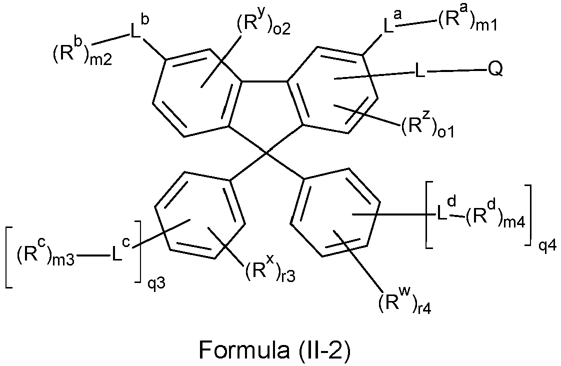 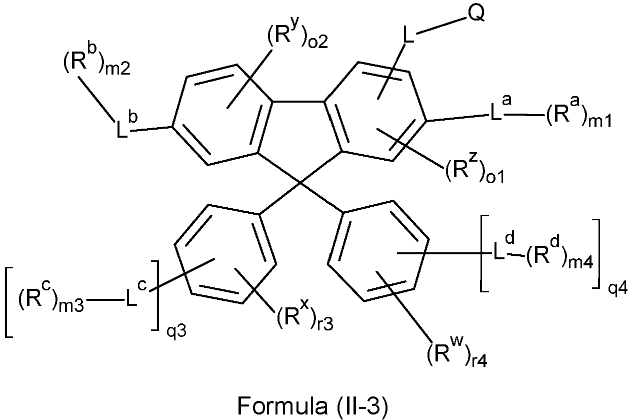 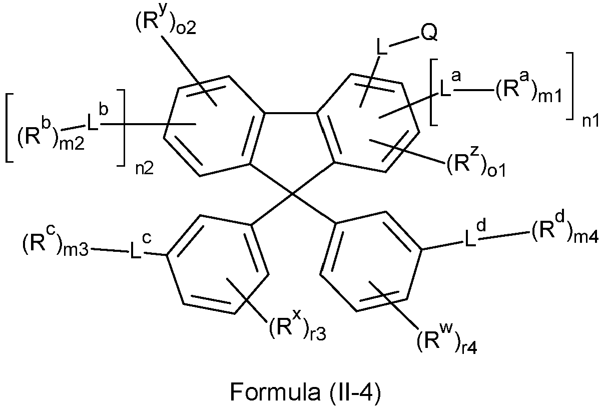 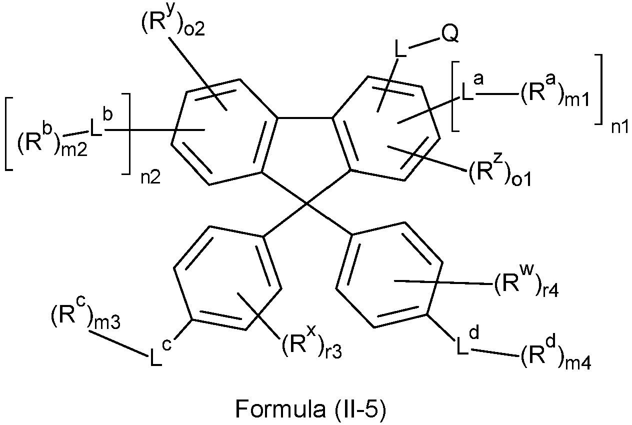 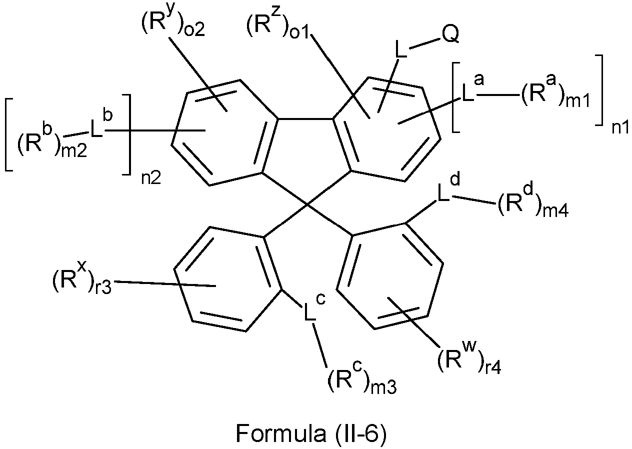 in which the symbols Q, L, L^{a}, L^{b}, L^{c}, L^{d}, R^{w}, R^{x}, R^{y}, R^{z}, R^{a}, R^{b}, R^{c}, R^{d}, m1, m2, m3, m4, n1, n2, n3, n4, o1, o2, o3, o4, q3, q4, r3 and r4 have the definition set out in Claim 1 or 2, wherein the Structures (I-3) and (I-4) are preferred.

6. Compound according to one or more of Claims 1 to 5, **characterized in that** the compound comprises at least one of the structures of the Formulae (la-3), (la-4), (Ib-4), (Ic-4) and/or (Id-4), preferably compound according to Formulae (la-3), (la-4), (Ib-4), (Ic-4) and/or (Id-4), 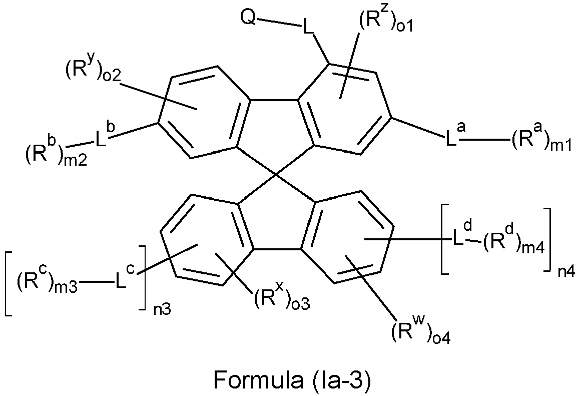 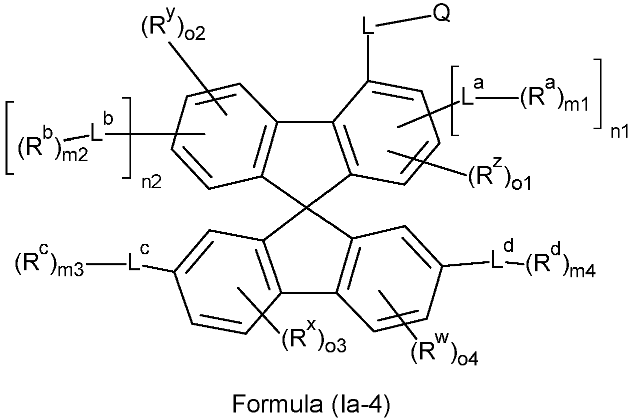 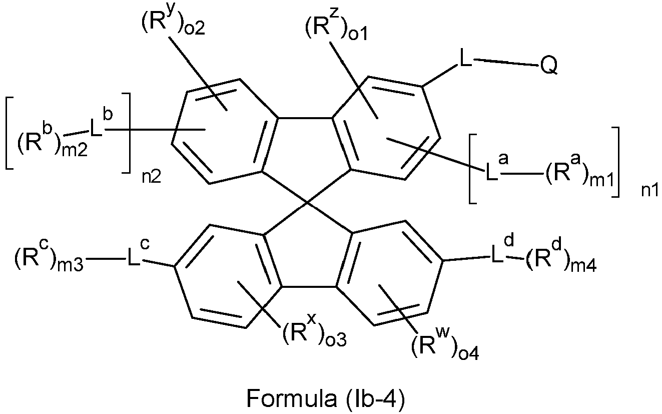 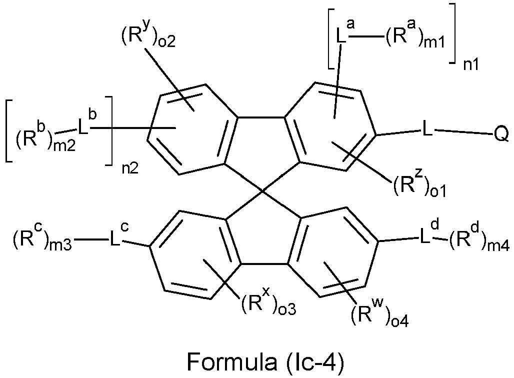 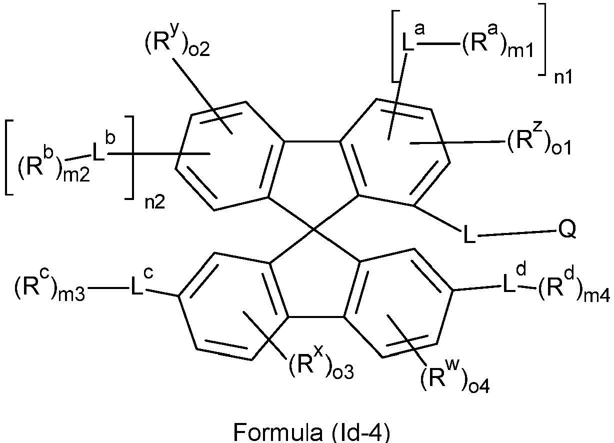 in which the symbols Q, L, L^{a}, L^{b}, L^{c}, L^{d}, R^{w}, R^{x}, R^{y}, R^{z}, R^{a}, R^{b}, R^{c}, R^{d}, m1, m2, m3, m4, n1, n2, n3, n4, o1, o2, o3 and o4 have the definition set out in Claim 1 or 2.

7. Compound according to one or more of Claims 1 to 6, **characterized in that** the Q group is a pyridine group, a pyrimidine group, or a triazine group, more preferably a triazine group, which may be sub¬stituted by one or more radicals R.

8. Compound according to Claim 7, **characterized in that** the Q group is selected from the structures of the Formulae (Q-1), (Q-2), (Q-3), (Q-4), (Q-5), (Q-6), (Q-7) and/or (Q-8) 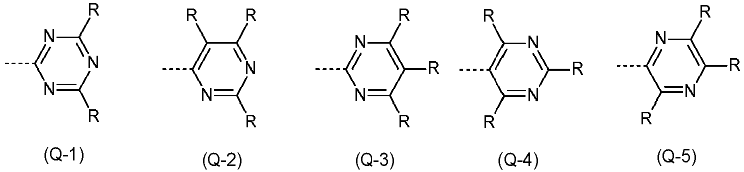 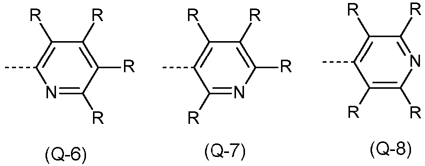 where the symbol R has the definition given above in Claim 1, the dotted bond marks the attachment position, wherein the structures (Q-1) to (Q-5) are preferred and the structure (Q-1) is especially preferred.

9. Compound according to Claim 8, **characterized in that** the Q group is selected from the structures of the Formulae (Q-1a), (Q-1b), (Q-1c), (Q-1d), (Q-1e), (Q-1f), (Q-1g), (Q-1h), (Q-1i) and/or (Q-1j) 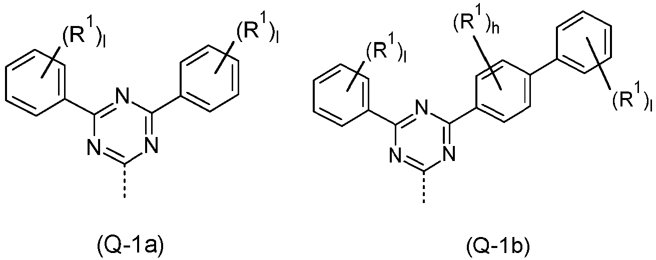 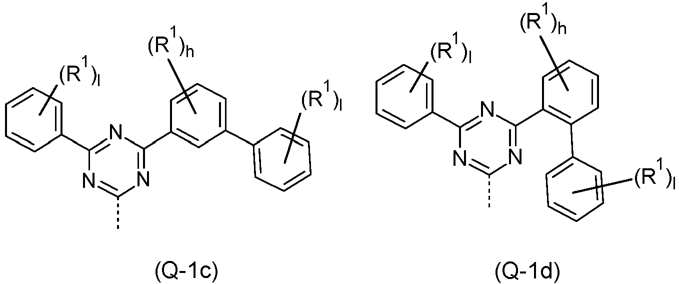 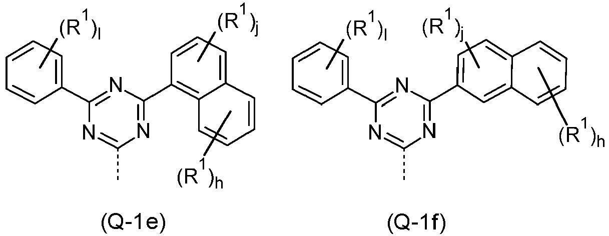 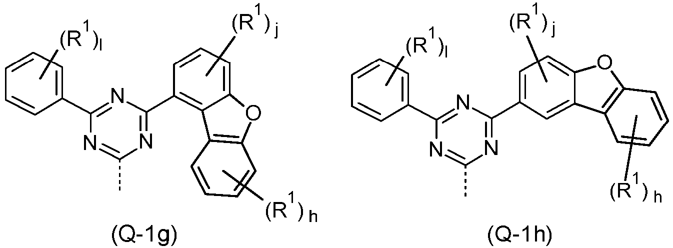 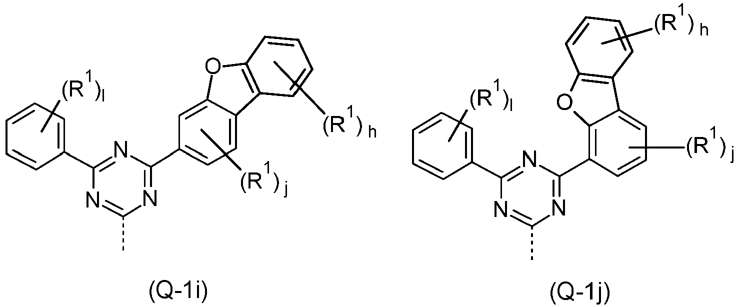 where the symbol R¹ has the definition given above in Claim 1, the dotted bond marks the attachment position and the index I is the same or different at each instance and is 0, 1, 2, 3, 4 or 5, preferably 0, 1, 2 or 3, more preferably 0 or 1, the index h is the same or different at each instance and is 0, 1, 2, 3 or 4, preferably 0, 1 or 2, the index j is the same or different at each instance and is 0, 1, 2 or 3, preferably 0, 1 or 2; wherein the structure (Q-1a) is preferred.

10. Compound according to one or more of Claims 1 to 9, **characterized in that** the symbol L, L^{a}, L^{b}, L^{c}, L^{d}, is a bond or an aryl group having 6 to 12 aromatic ring atoms, which may be substituted by one or more radicals R, preferably a phenylene group, a biphenylene group or a naphthylene group, more preferably a phenylene group or a naphthylene group, which may be sub¬stituted by one or more radicals R.

11. Compound according to Claim 10, **characterized in that** the symbol L, L^{a}, L^{b}, L^{c}, L^{d}, is a bond or is selected from the structures of the Formulae (L-1), (L-5), (L-6) and/or (L-7) 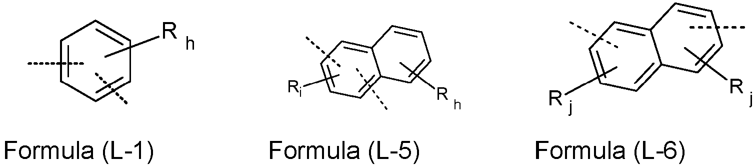 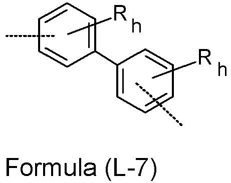 where the symbol R has the definition given above in Claim 1, the dotted bond marks the attachment positions, the index h is the same or different at each instance and is 0, 1, 2, 3 or 4, preferably 0, 1 or 2, the index j is the same or different at each instance and is 0, 1, 2 or 3, preferably 0, 1 or 2; the index i is the same or different at each instance and 0, 1 or 2, preferably 0 or 1; wherein the structure (L-1) is preferred.

12. Compound according to one or more of Claims 1 to 11, **characterized in that** the compound comprises at least one of the structures of the Formulae (la-3a), (la-4a), (Ib-4a) and/or (Ic-4a), preferably compound according to Formulae (la-3a), (la-4a), (Ib-4a) and/or (Ic-4a), 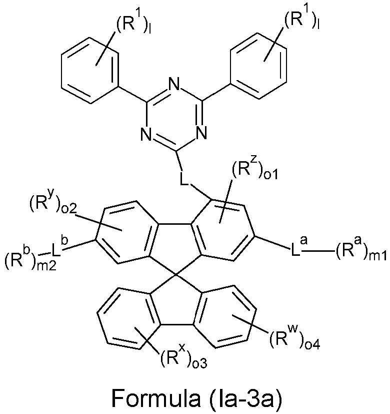 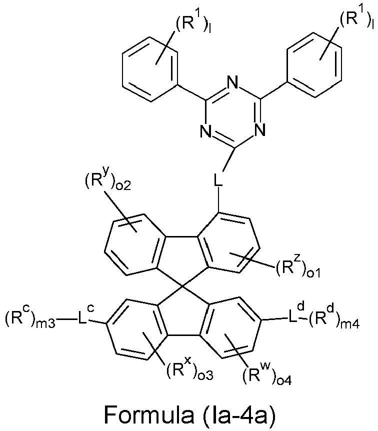 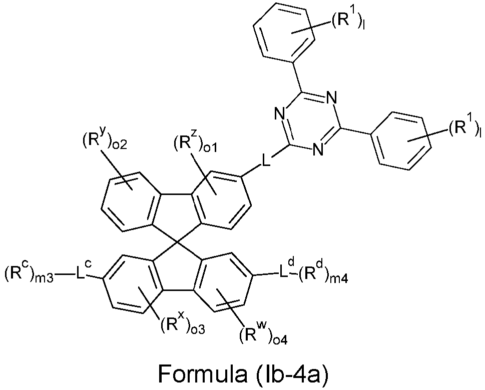 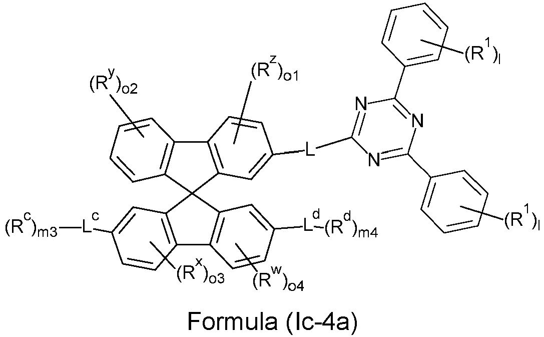 in which the symbols L, L^{a}, L^{b}, L^{c}, L^{d}, R¹, R^{w}, R^{x}, R^{y}, R^{z}, R^{a}, R^{b}, R^{c}, R^{d}, l, m1, m2, m3, m4, o1, o2, o3 and o4 have the definition set out in Claim 1, 2 or 13.

13. Compound according to one or more of Claims 1 to 12, **characterized in that** the compound comprises exactly one triazine group, more preferably exactly one electron transport group.

14. Oligomer, polymer or dendrimer containing one or more compounds according to one or more of Claims 1 to 13, wherein one or more bonds of the compound to the polymer, oligomer or dendrimer are present.

15. Composition comprising at least one compound according to one or more of Claims 1 to 13 and/or an oligomer, polymer or dendrimer according to Claim 14 and at least one further compound selected from the group consisting of fluorescent emitters, phosphorescent emitters, TADF emitters, host materials, matrix materials, electron transport materials, electron injection materials, hole conductor materials, hole injection materials, electron blocking materials and hole blocking materials.

16. Formulation comprising at least one compound according to one or more of Claims 1 to 13, an oligomer, polymer or dendrimer according to Claim 14 and/or at least one composition according to Claim 15 and at least one solvent.

17. Process for preparing a compound according to one or more of Claims 1 to 13 or an oligomer, polymer or dendrimer according to Claim 14, **characterized in that**, in a coupling reaction, a compound comprising at least one electron-transporting group is reacted with a compound comprising at least one fluorene or spirobifluorene radical.

18. Use of a compound according to one or more of Claims 1 to 13, of an oligomer, polymer or dendrimer according to Claim 14 or of a composition according to Claim 15 in an electronic device as a hole blocking material, electron injection material and/or electron transport material.

19. Electronic device comprising at least one compound according to one or more of Claims 1 to 13, an oligomer, polymer or dendrimer according to Claim 14 or a composition according to Claim 15, wherein the electronic device is preferably selected from the group consisting of organic electroluminescent devices, organic integrated circuits, organic field-effect transistors, organic thin-film transistors, organic light-emitting transistors, organic solar cells, organic optical detectors, organic photoreceptors, organic field quench devices, light-emitting electrochemical cells and organic laser diodes.

20. Electronic device according to Claim 19, **characterized in that** the device comprises at least one hole-blocking layer and at least one electron transport layer, wherein the hole-blocking layer is directly adjacent to an emitting layer and the at least one hole-blocking layer comprises a compound according to one or more of Claims 1 to 13 and/or an oligomer, polymer or dendrimer according to Claim 14 and the electron transport layer comprises at least one electron transport compound being different to a compound according to one or more of Claims 1 to 13 and/or an oligomer, polymer or dendrimer according to Claim 14.

## Patentansprüche

1. Verbindung umfassend Strukturen der Formel (A), bevorzugt Verbindung gemäß Formel (A), 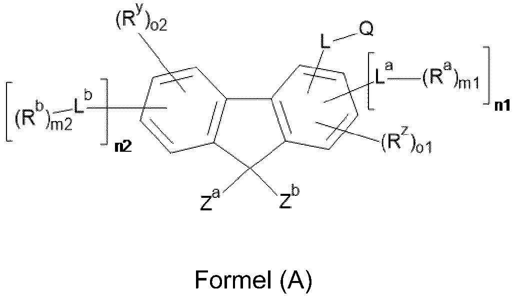 wobei die verwendeten Symbole wie folgt definiert sind:
Z^{a} ist Ar^{a} oder R^{x}, wobei Ar^{a} ein aromatisches oder heteroaromatisches Ringsystem ist, das 5 bis 40 aromatische Ringatome aufweist, von denen jedes durch ein oder mehrere R^{x}-Radikale substituiert sein kann, wobei die Gruppen Z^{a} und Z^{b} miteinander ein mono- oder polycyclisches aliphatisches, heteroaliphatisches, aromatisches oder heteroaromatisches Ringsystem bilden können;
Z^{b} ist Ar^{b} oder R^{w}, wobei Ar^{b} ein aromatisches oder heteroaromatisches Ringsystem ist, das 5 bis 40 aromatische Ringatome aufweist, von denen jedes durch ein oder mehrere R^{w}-Radikale substituiert sein kann, wobei die Gruppen Z^{a} und Z^{b} miteinander ein mono- oder polycyclisches aliphatisches, heteroaliphatisches, aromatisches oder heteroaromatisches Ringsystem bilden können;
Q ist eine Elektronentransportgruppe;
L, L^{a}, L^{b} ist in jedem Fall gleich oder verschieden und ist eine Einfachbindung, C(=O) oder ein aromatisches oder heteroaromatisches Ringsystem mit 5 bis 24 aromatischen Ringatomen, das durch ein oder mehrere Radikale R substituiert sein kann;
R, R^{w}, R^{x}, R^{y}, R^{z} ist in jedem Fall gleich oder verschieden und ist H, D, F, Cl, Br, I, CN, Si(R¹)₃, eine unverzweigte Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, von denen jede durch ein oder mehrere Radikale R¹ substituiert sein kann, wobei jeweils eine oder mehrere nicht benachbarte CH₂-Gruppen durch -R¹C=CR¹-, -C≡C-, Si(R¹)₂, C=O, C=S, C=NR¹, -C(=O)O-, -C(=O)NR¹-, NR¹, P(=O)(R¹), -O-, -S-, SO oder SO₂ ersetzt werden können und wobei ein oder mehrere H-Atome durch D, F, Cl, Br, I, CN oder NO₂ ersetzt werden können, oder ein aromatisches oder heteroaromatisches Ringsystem mit 5 bis 40 aromatischen Ringatomen, das jeweils durch ein oder mehrere Radikale R¹ substituiert sein kann, oder eine Aryloxy- oder Heteroaryloxygruppe mit 5 bis 40 aromatischen Ringatomen, die durch ein oder mehrere Radikale R¹ substituiert sein kann, oder eine Aralkylgruppe mit 5 bis 40 aromatischen Ringatomen, die jeweils durch ein oder mehrere Radikale R¹ substituiert sein kann, oder eine Kombination dieser Systeme; gleichzeitig können auch zwei oder mehr benachbarte Substituenten R, R^{w}, R^{x}, R^{y}, R^{z} miteinander ein Ringsystem, bevorzugt ein mono- oder polycyclisches, aliphatisches oder aromatisches Ringsystem, bilden, das durch ein oder mehrere Radikale R¹ substituiert sein kann;
R¹ ist in jedem Fall gleich oder verschieden und ist H, D, eine unverzweigte Alkyl-, Alkoxy- oder Thioalkoxygruppe mit 1 bis 40 Kohlenstoffatomen oder eine verzweigte oder cyclische Alkyl-, Alkoxy- oder Thioalkoxygruppe mit 3 bis 40 Kohlenstoffatomen, von denen jede durch ein oder mehrere R²-Radikale substituiert sein kann, wobei eine oder mehrere nicht benachbarte CH₂- Gruppen durch -R²C=CR²-, -C≡C-, Si(R²)₂, C=O, C=S, C=NR², - C(=O)O-, -C(=O)NR²-, NR², P(=O)(R²), -O-, -S-, SO oder SO₂ ersetzt werden können und wobei ein oder mehrere Wasserstoffatome durch D, F, Cl, Br, I, CN oder NO₂ ersetzt werden können, oder ein aromatisches oder heteroaromatisches Ringsystem, das 5 bis 40 aromatische Ringatome aufweist, von denen jedes durch ein oder mehrere R²-Radikale substituiert sein kann, oder eine Aryloxy- oder Heteroaryloxygruppe, die 5 bis 40 aromatische Ringatome aufweist, von denen jedes durch ein oder mehrere R²-Radikale substituiert sein kann, oder eine Kombination dieser Systeme; gleichzeitig können auch zwei oder mehr benachbarte R¹-Substituenten miteinander ein Ringsystem, bevorzugt ein mono- oder polycyclisches aliphatisches oder aromatisches Ringsystem, bilden;
-R² ist in jedem Fall gleich oder verschieden und ist H, D, F oder ein aliphatisches Kohlenwasserstoffradikal mit 1 bis 20 Kohlenstoffatomen, in dem ein oder mehrere Wasserstoffatome durch D oder F ersetzt werden können, oder ein aromatisches oder heteroaromatisches Ringsystem mit 5 bis 30 Kohlenstoffatomen, in dem ein oder mehrere Wasserstoffatome durch D oder F ersetzt werden können; gleichzeitig können auch zwei oder mehr benachbarte R²-Substituenten zusammen ein Ringsystem, bevorzugt ein mono- oder polycyclisches, aliphatisches oder aromatisches Ringsystem, bilden;
R^{a}, R^{b} ist in jedem Fall gleich oder verschieden und ausgewählt aus
(R^{a}-1) bis (R^{a}-33): 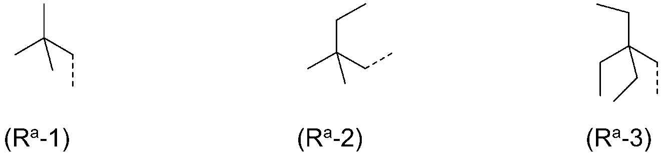 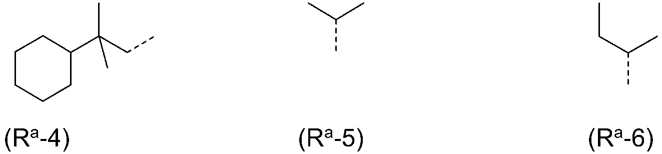 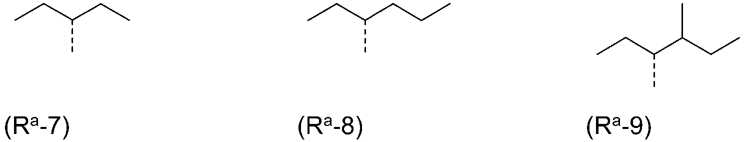 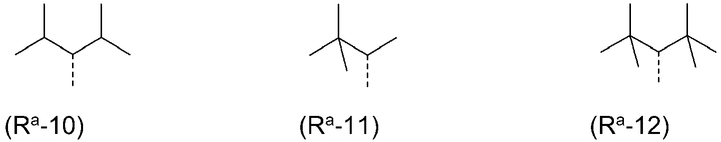 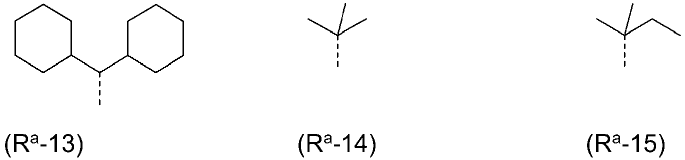 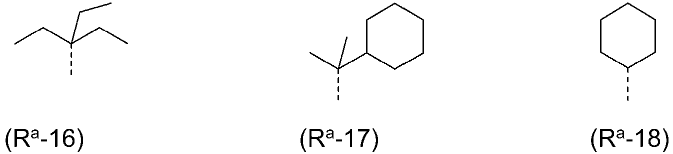 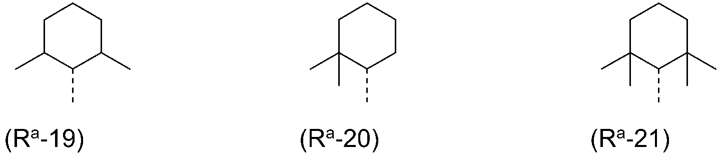 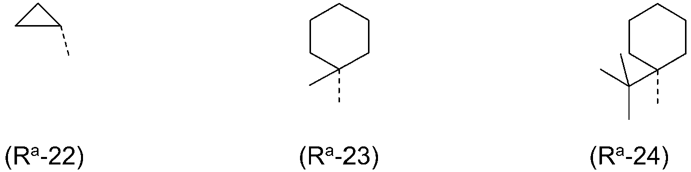 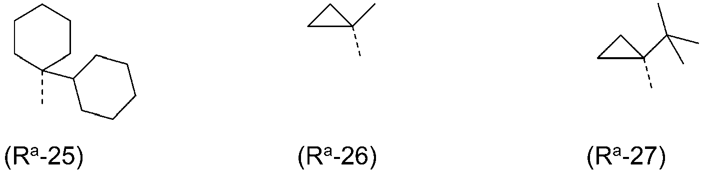 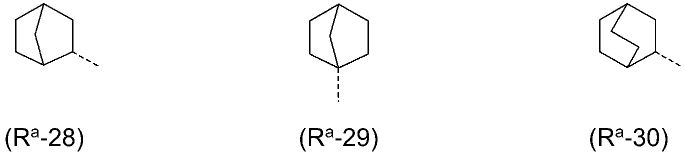 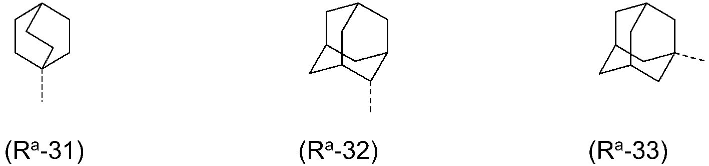 wobei ein oder mehrere H-Atome durch D ersetzt werden können und die gestrichelte Linie die Bindung zur entsprechenden Gruppe bezeichnet;
m1, m2 ist in jedem Fall gleich oder verschieden und ist 0, 1, 2, 3 oder 4, bevorzugt 1, 2, 3 oder 4, stärker bevorzugt 1 oder 2;
n2, o2 ist in jedem Fall gleich oder verschieden und ist 0, 1, 2, 3 oder 4, bevorzugt 0, 1 oder 2;
n1, o1 ist in jedem Fall gleich oder verschieden und ist 0, 1, 2 oder 3, bevorzugt 0, 1 oder 2;
die Summe aus n1 + o1 ist 0, 1, 2 oder 3, bevorzugt 0, 1 oder 2;
die Summe aus n2 + o2 ist 0, 1, 2, 3 oder 4, bevorzugt 0, 1 oder 2; und
**dadurch gekennzeichnet, dass** die Verbindung zwei, drei oder vier Strukturelemente der Formeln (R^{a}-1) bis (R^{a}-33) umfasst.

2. Verbindung gemäß Anspruch 1, **dadurch gekennzeichnet, dass** die Verbindung Strukturen der Formel (I) oder Formel (II) umfasst, bevorzugt Verbindung gemäß Formel (I) oder Formel (II), 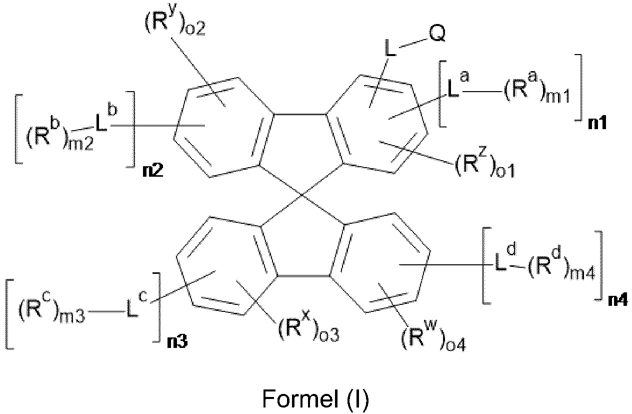 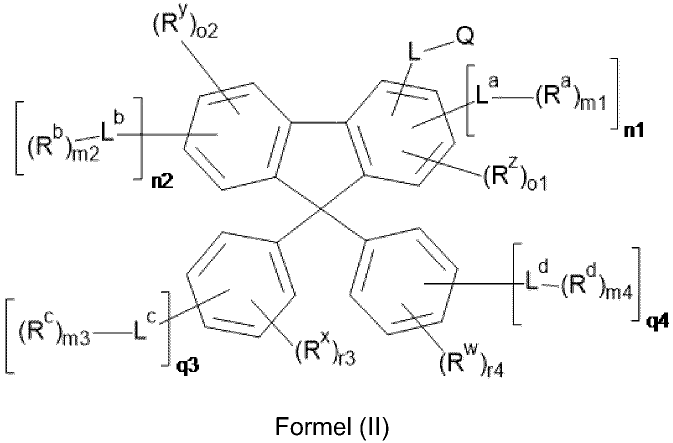 in denen
die Symbole Q, L, L^{a}, L^{b}, R, R^{w}, R^{x}, R^{y}, R^{z}, R^{a}, R^{b}, m1, m2, n1, n2, o1 und o2 die in Anspruch 1 angegebene Definition haben und
L^{c}, L^{d} ist in jedem Fall gleich oder verschieden ist und eine Einfachbindung, C(=O) oder ein aromatisches oder heteroaromatisches Ringsystem mit 5 bis 24 aromatischen Ringatomen, das durch ein oder mehrere Radikale R substituiert sein kann, ist;
R^{c}, R^{d} ist in jedem Fall gleich oder verschieden und ist ausgewählt aus (R^{a}-1) bis (R^{a}-33) wie in Anspruch 1 angegeben;
m3, m4 ist in jedem Fall gleich oder verschieden und ist 0, 1, 2, 3 oder 4, bevorzugt 1, 2, 3 oder 4, bevorzugter 1 oder 2;
n3, n4, o3, o4 ist in jedem Fall gleich oder verschieden und ist 0, 1, 2, 3 oder 4, bevorzugt 0, 1 oder 2;
q3, q4, r3, r4 ist in jedem Fall gleich oder verschieden und ist 0, 1, 2, 3, 4 oder 5, bevorzugt 0, 1 oder 2;
die Summe aus n3 + o3 ist 0, 1, 2, 3 oder 4, bevorzugt 0, 1 oder 2;
die Summe aus n4 + o4 ist 0, 1, 2, 3 oder 4, bevorzugt 0, 1 oder 2;
die Summe aus q3 + r3 ist 0, 1, 2, 3, 4 oder 5, bevorzugt 0, 1 oder 2;
die Summe aus q4 + r4 ist 0, 1, 2, 3, 4 oder 5, bevorzugt 0, 1 oder 2; und
wobei die Verbindung zwei, drei oder vier Strukturelemente der Formeln (R^{a}-1) bis (R^{a}-33) umfasst.

3. Verbindung gemäß Anspruch 1 oder 2, **dadurch gekennzeichnet, dass** die Verbindung mindestens eine der Strukturen der Formeln (la), (Ib), (Ic), (Id), (IIa), (IIb), (IIc) und/oder (IId) umfasst, bevorzugt Verbindung gemäß den Formeln (la), (Ib), (Ic), (Id), (IIa), (IIb), (IIc) und/oder (IId), 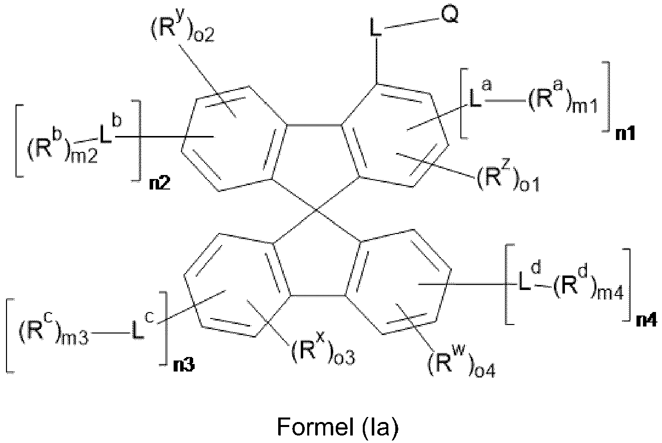 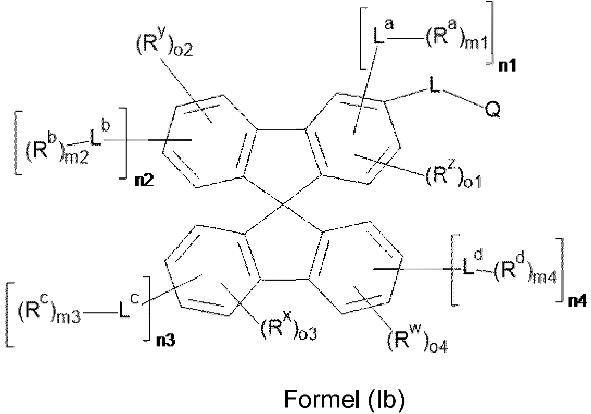 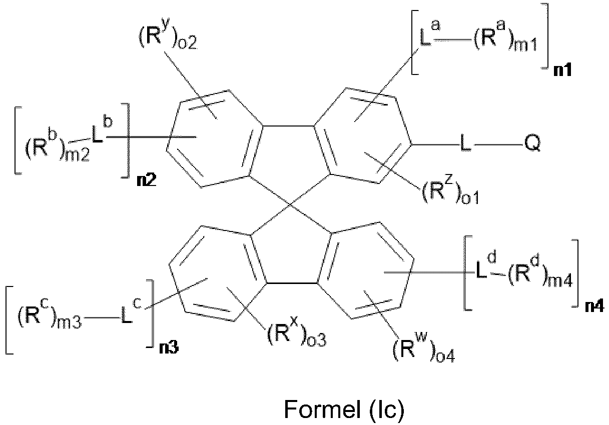 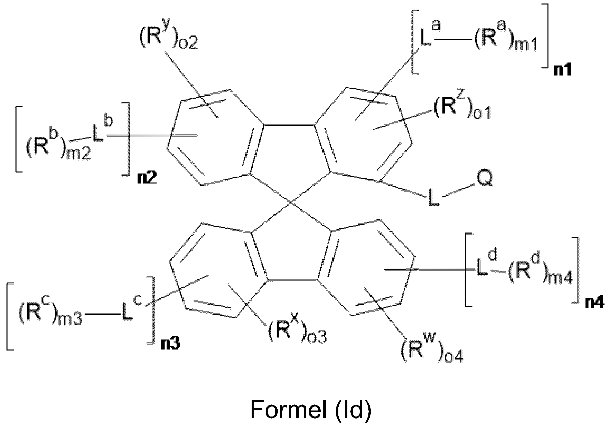 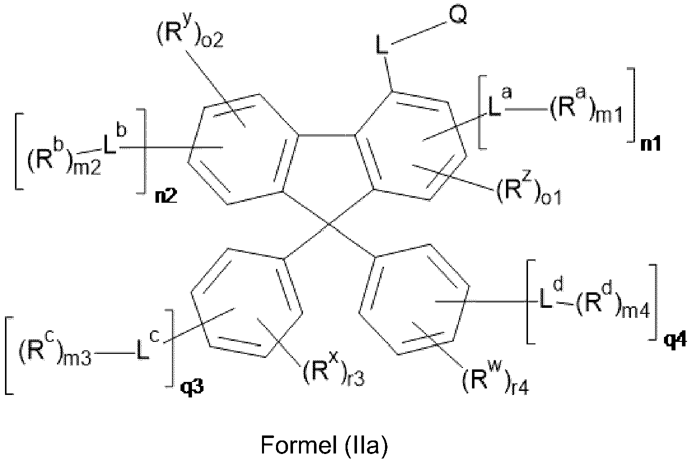 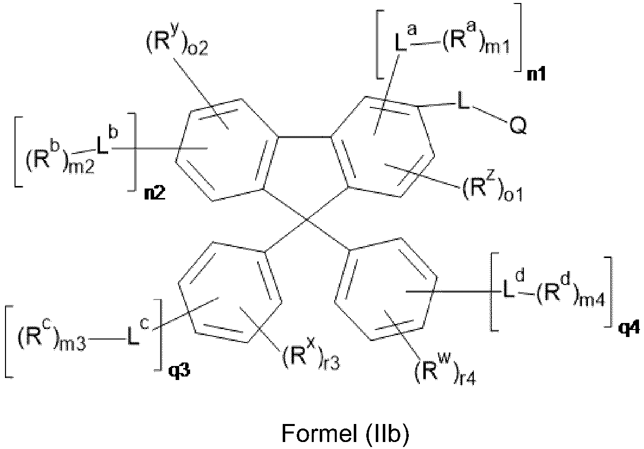 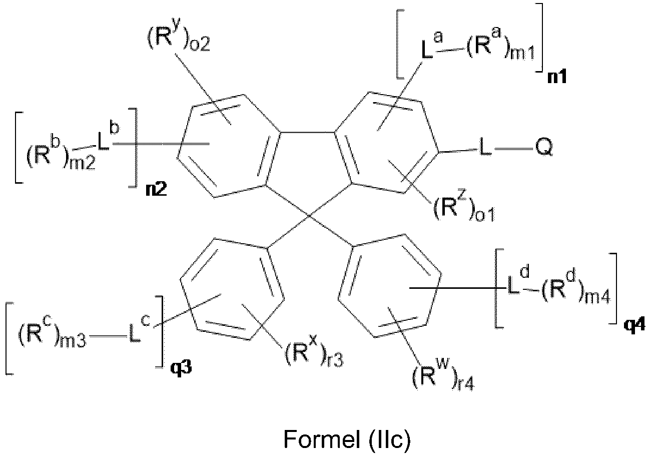 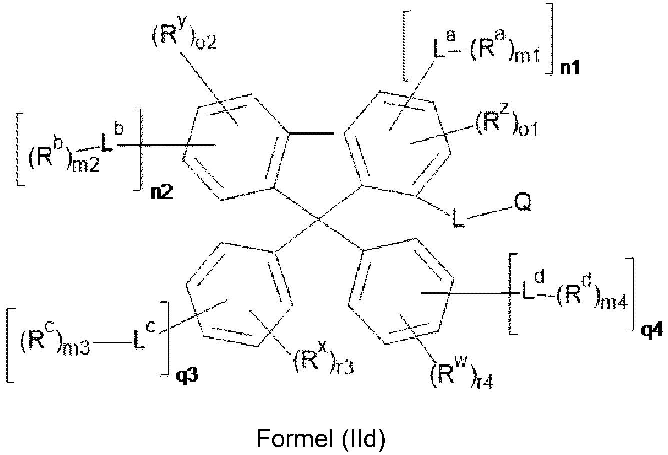 in denen die Symbole Q, L, L^{a}, L^{b}, L^{c}, L^{d}, R^{w}, R^{x}, R^{y}, R^{z}, R^{a}, R^{b}, R^{c}, R^{d}, m1, m2, m3, m4, n1, n2, n3, n4, o1, o2, o3, o4, q3, q4, r3 und r4 die in Anspruch 1 oder 2 angegebene Definition haben, wobei die Strukturen (la) und (Ic) bevorzugt sind.

4. Verbindung gemäß einem oder mehreren der Ansprüche 2 oder 3, **dadurch gekennzeichnet, dass** die Summe der Indizes m1, m2, m3 und m4 im Bereich von 1 bis 6 liegt, bevorzugt 1 bis 4, bevorzugter 2 oder 3.

5. Verbindung gemäß einem oder mehreren der Ansprüche 1 bis 4, **dadurch gekennzeichnet, dass** die Verbindung mindestens eine der Strukturen der Formeln (I-1), (I-2), (I-3), (I-4), (I-5), (I-6), (II-1), (II-2), (II-3), (II-4), (II-5) und/oder (II-6) umfasst, bevorzugt Verbindung gemäß den Formeln (I-1), (I-2), (I-3), (I-4), (I-5), (I-6), (II-1), (II-2), (II-3), (II-4), (II-5) und/oder (II-6), 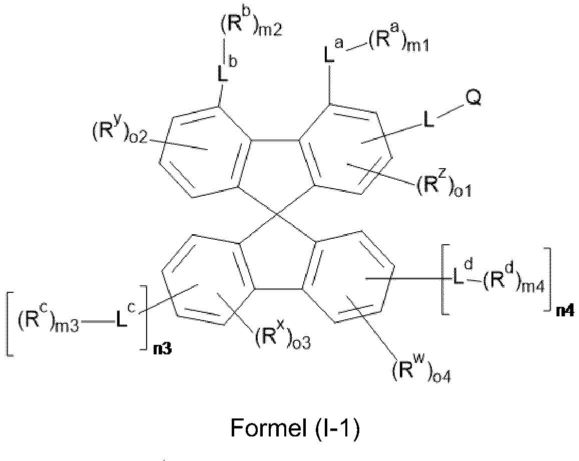 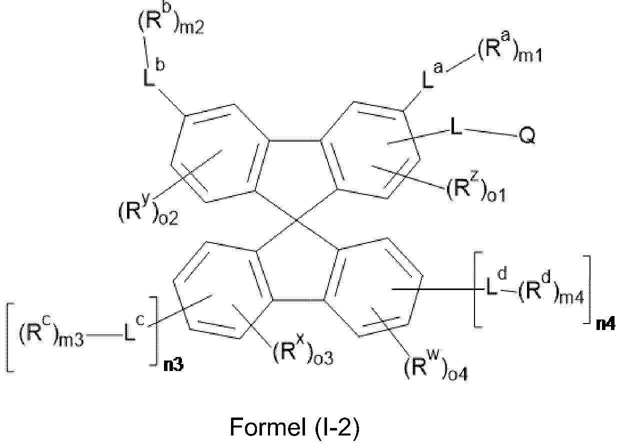 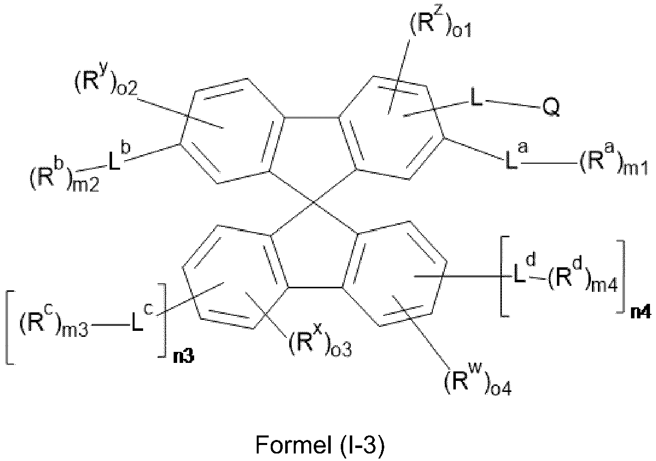 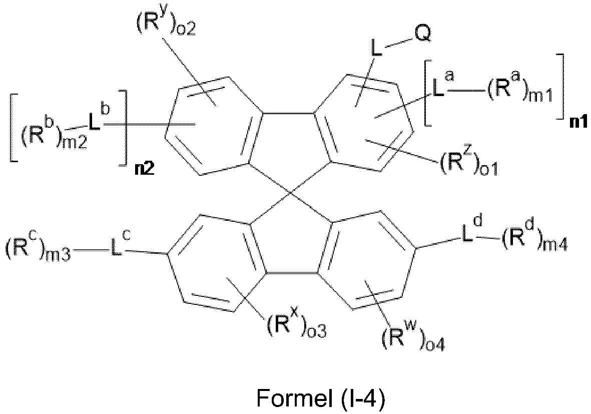 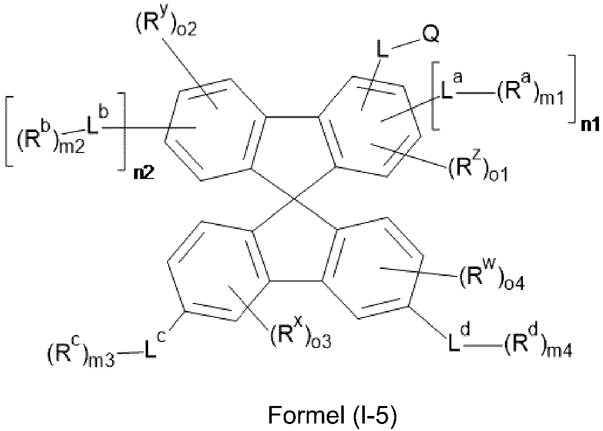 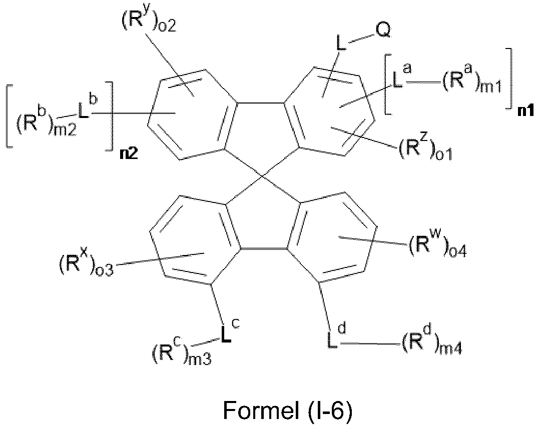 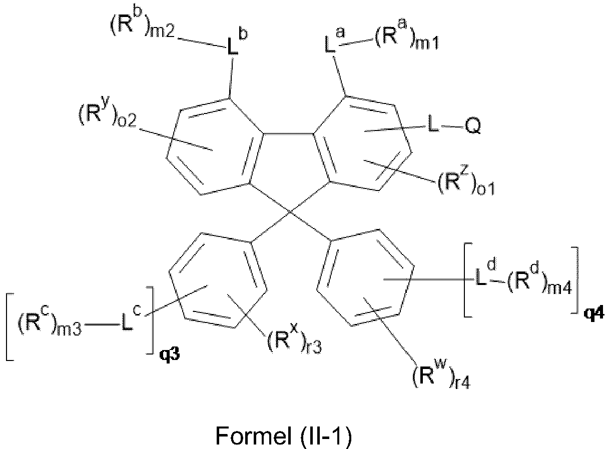 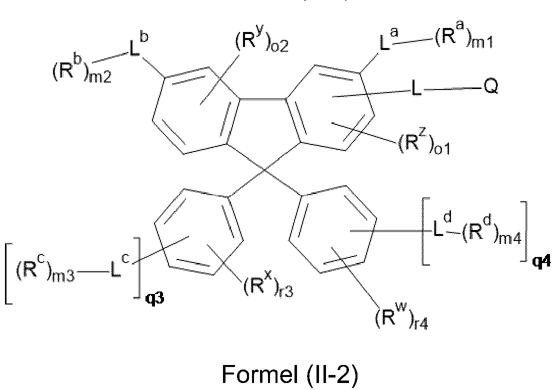 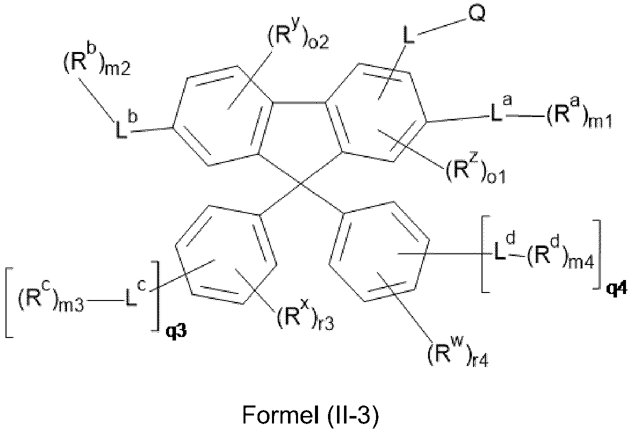 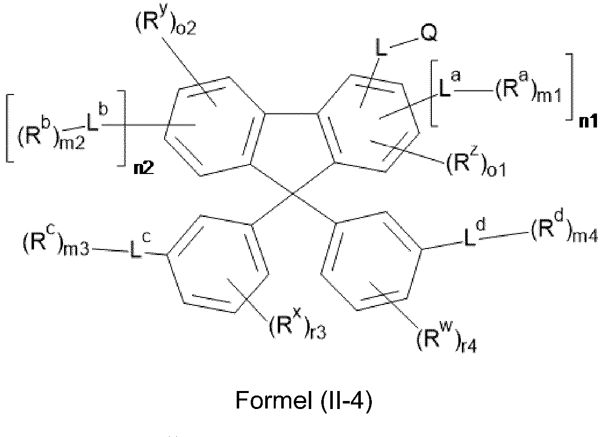 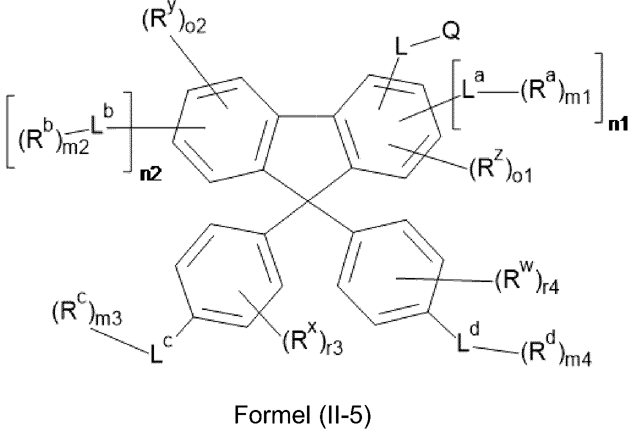 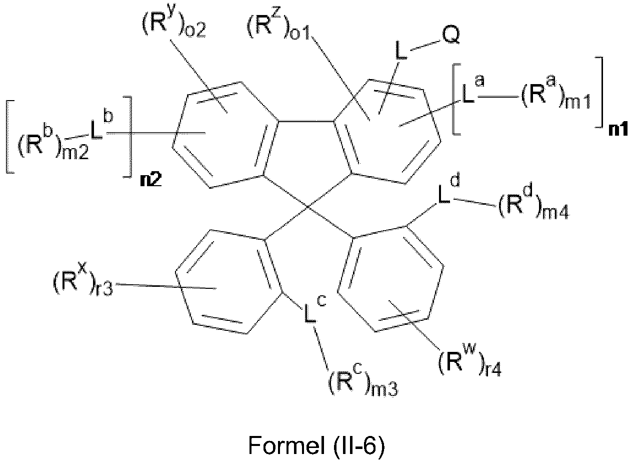 in denen die Symbole Q, L, L^{a}, L^{b}, L^{c}, L^{d}, R^{w}, R^{x}, R^{y}, R^{z}, R^{a}, R^{b}, R^{c}, R^{d}, m1, m2, m3, m4, n1, n2, n3, n4, o1, o2, o3, o4, q3, q4, r3 und r4 die in Anspruch 1 oder 2 angegebene Definition haben, wobei die Strukturen (I-3) und (I-4) bevorzugt sind.

6. Verbindung gemäß einem oder mehreren der Ansprüche 1 bis 5, **dadurch gekennzeichnet, dass** die Verbindung mindestens eine der Strukturen der Formeln (la-3), (la-4), (Ib-4), (Ic-4) und/oder (Id-4) umfasst, bevorzugt Verbindung gemäß den Formeln (la-3), (la-4), (Ib-4), (Ic-4) und/oder (Id-4), 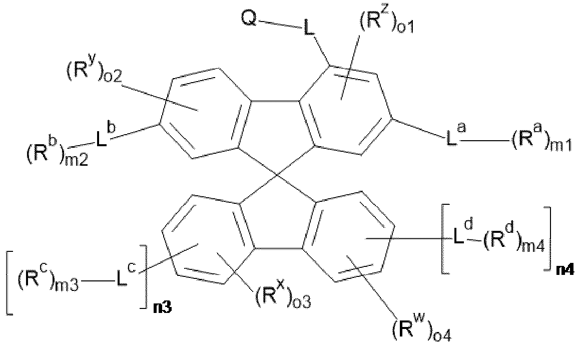 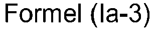 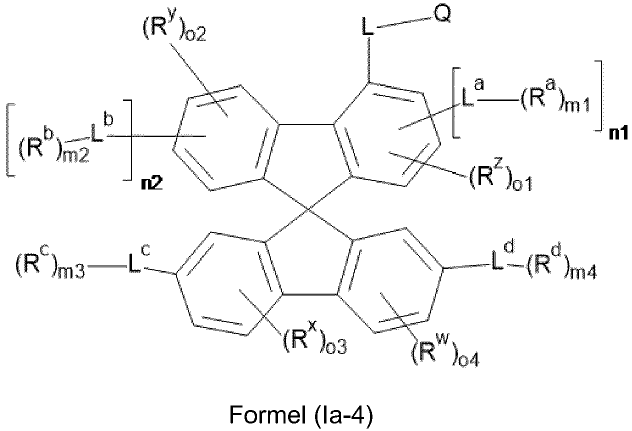 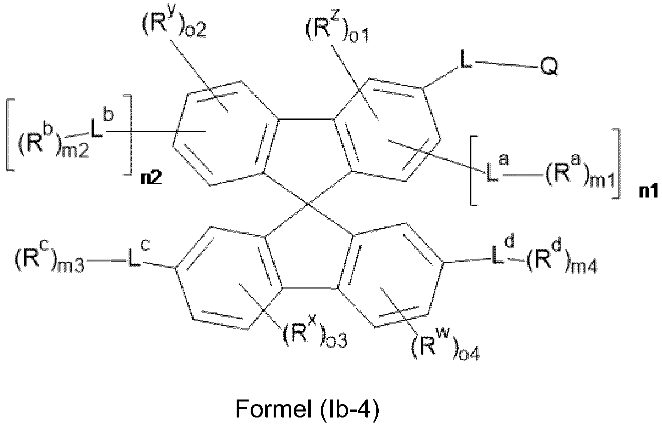 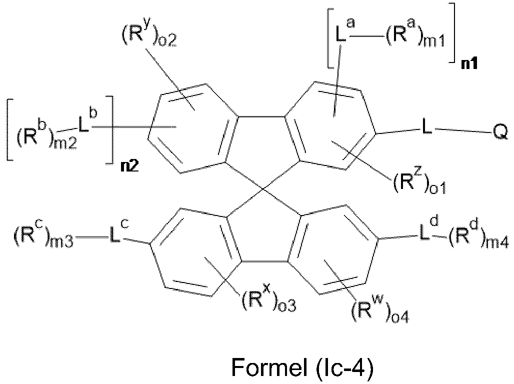 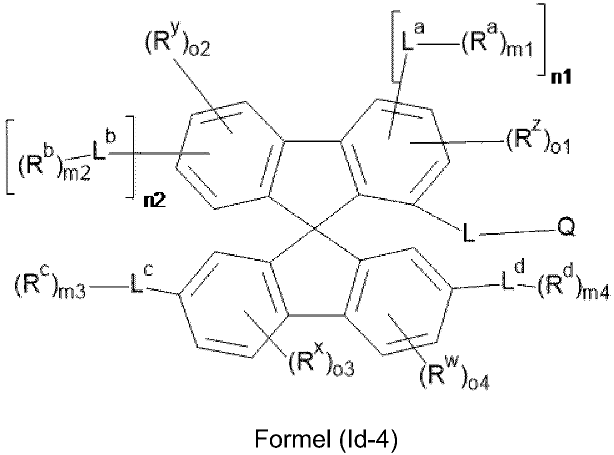 in denen die Symbole Q, L, L^{a}, L^{b}, L^{c}, L^{d}, R^{w}, R^{x}, R^{y}, R^{z}, R^{a}, R^{b}, R^{c}, R^{d}, m1, m2, m3, m4, n1, n2, n3, n4, o1, o2, o3 und o4 die in Anspruch 1 oder 2 angegebene Definition haben.

7. Verbindung gemäß einem oder mehreren der Ansprüche 1 bis 6, **dadurch gekennzeichnet, dass** die Q-Gruppe eine Pyridingruppe, eine Pyrimidin-Gruppe oder eine Triazin-Gruppe ist, bevorzugter eine Triazin-Gruppe, die durch ein oder mehrere Radikale R substituiert sein kann.

8. Verbindung gemäß Anspruch 7, **dadurch gekennzeichnet, dass** die Q-Gruppe aus den Strukturen der Formeln (Q-1), (Q-2), (Q-3), (Q-4), (Q-5), (Q-6), (Q-7) und/oder (Q-8) ausgewählt ist 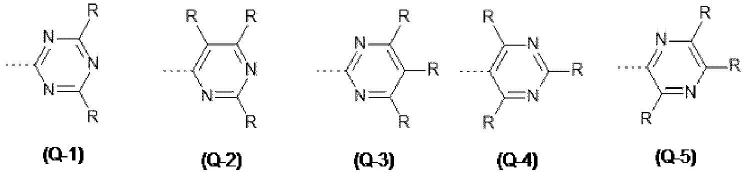 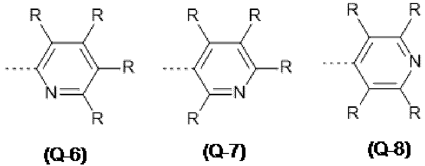 wobei das Symbol R die oben in Anspruch 1 angegebene Definition hat, die gestrichelte Bindung die Anknüpfungsposition kennzeichnet, wobei die Strukturen (Q-1) bis (Q-5) bevorzugt sind und die Struktur (Q-1) besonders bevorzugt ist.

9. Verbindung gemäß Anspruch 8, **dadurch gekennzeichnet, dass** die Q-Gruppe aus den Strukturen der Formeln (Q-1a), (Q-1b), (Q-1c), (Q-1d), (Q-1e), (Q-1f), (Q-1g), (Q-1h), (Q-1i) und/oder (Q-1j) ausgewählt ist 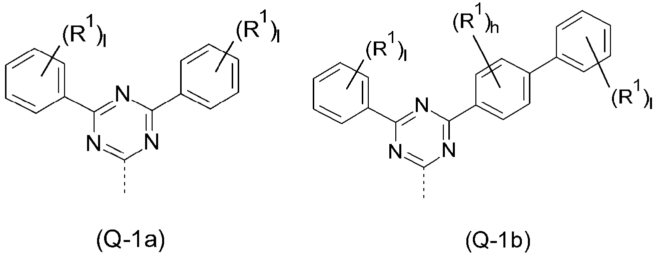 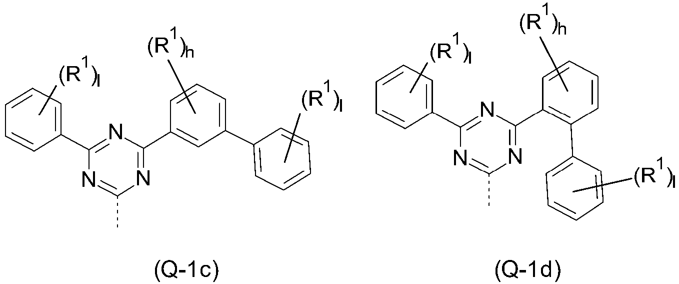 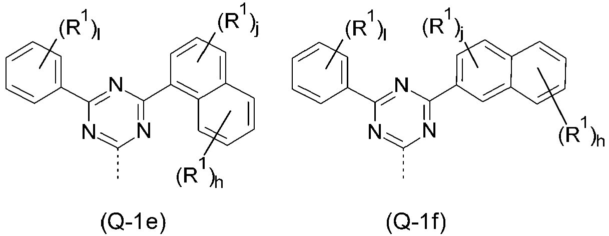 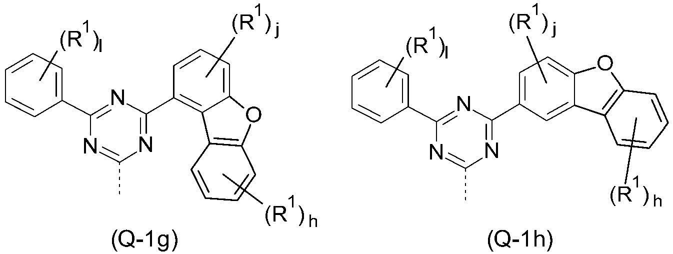 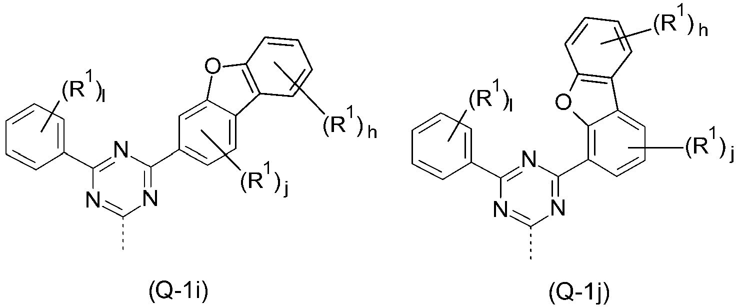 wobei das Symbol R¹ die oben in Anspruch 1 angegebene Definition hat, die gestrichelte Bindung die Anknüpfungsposition kennzeichnet und der Index I in jedem Fall gleich oder verschieden ist und 0, 1, 2, 3, 4 oder 5 ist, bevorzugt 0, 1, 2 oder 3, bevorzugter 0 oder 1, der Index h in jedem Fall gleich oder verschieden ist und 0, 1, 2, 3 oder 4 ist, bevorzugt 0, 1 oder 2, der Index j in jedem Fall gleich oder verschieden ist und 0, 1, 2 oder 3 ist, bevorzugt 0, 1 oder 2; wobei die Struktur (Q-1a) bevorzugt ist.

10. Verbindung gemäß einem oder mehreren der Ansprüche 1 bis 9, **dadurch gekennzeichnet, dass** das Symbol **L,** L^{a}, L^{b}, L^{c}, L^{d} eine Bindung oder eine Arylgruppe mit 6 bis 12 aromatischen Ringatomen ist, die durch ein oder mehrere Radikale R substituiert sein kann, bevorzugt eine Phenylen-Gruppe, eine Biphenylen-Gruppe oder eine Naphthylen-Gruppe, bevorzugter eine Phenylen-Gruppe oder eine Naphthylen-Gruppe, die durch ein oder mehrere Radikale R substituiert sein kann.

11. Verbindung gemäß Anspruch 10, **dadurch gekennzeichnet, dass** das Symbol L, L^{a}, L^{b}, L^{c}, L^{d} eine Bindung ist oder aus den Strukturen der Formeln (L-1), (L-5), (L-6) und/oder (L-7) ausgewählt ist 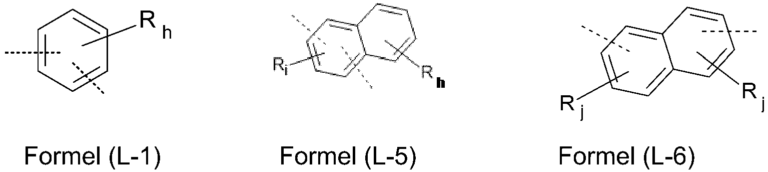 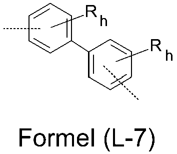 wobei das Symbol R die oben in Anspruch 1 angegebene Definition hat, die gestrichelte Bindung die Anknüpfungspositionen kennzeichnet, der Index h in jedem Fall gleich oder verschieden ist und 0, 1, 2, 3 oder 4 ist, bevorzugt 0, 1 oder 2, der Index j in jedem Fall gleich oder verschieden ist und 0, 1, 2 oder 3 ist, bevorzugt 0, 1 oder 2; der Index i in jedem Fall gleich oder verschieden ist und 0, 1 oder 2 ist, bevorzugt 0 oder 1; wobei die Struktur (L-1) bevorzugt ist.

12. Verbindung gemäß einem oder mehreren der Ansprüche 1 bis 11, **dadurch gekennzeichnet, dass** die Verbindung mindestens eine der Strukturen der Formeln (la-3a), (la-4a), (Ib-4a) und/oder (Ic-4a) umfasst, bevorzugt Verbindung gemäß den Formeln (la-3a), (la-4a), (Ib-4a) und/oder (Ic-4a), 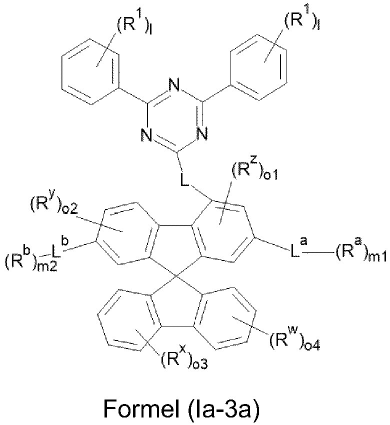 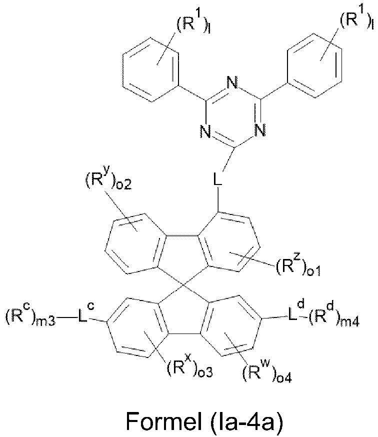 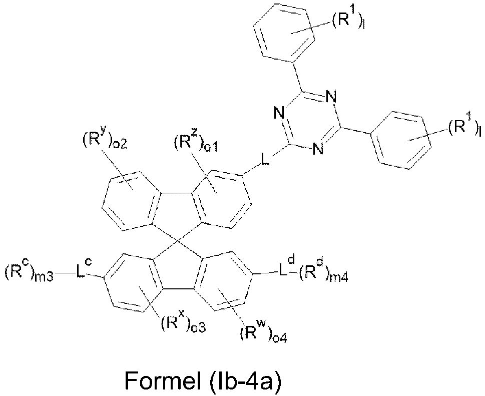 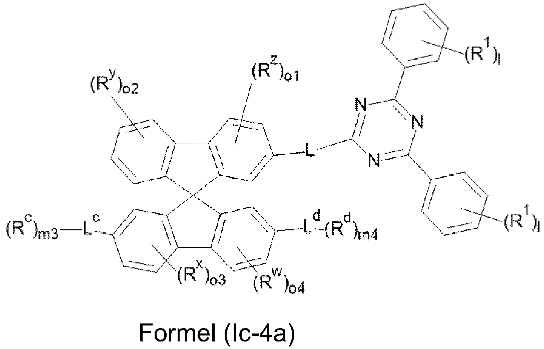 in denen die Symbole L, L^{a}, L^{b}, L^{c}, L^{d}, R¹, R^{w}, R^{x}, R^{y}, R^{z}, R^{a}, R^{b}, R^{c}, R^{d}, l, m1, m2, m3, m4, o1, o2, o3 und o4 die in Anspruch 1, 2 oder 13 angegebene Definition haben.

13. Verbindung gemäß einem oder mehreren der Ansprüche 1 bis 12, **dadurch gekennzeichnet, dass** die Verbindung genau eine Triazin-Gruppe umfasst, bevorzugter genau eine Elektronentransportgruppe.

14. Oligomer, Polymer oder Dendrimer enthaltend eine oder mehrere Verbindungen gemäß einem oder mehreren der Ansprüche 1 bis 13, wobei eine oder mehrere Bindungen der Verbindung an das Polymer, Oligomer oder Dendrimer vorhanden sind.

15. Zusammensetzung umfassend mindestens eine Verbindung gemäß einem oder mehreren der Ansprüche 1 bis 13 und/oder ein Oligomer, Polymer oder Dendrimer gemäß Anspruch 14 sowie mindestens eine weitere Verbindung ausgewählt aus der Gruppe bestehend aus fluoreszierenden Emittern, phosphoreszierenden Emittern, TADF-Emittern, Wirtsmaterialien, Matrixmaterialien, Elektronentransportmaterialien, Elektroneninjektionsmaterialien, Lochleitermaterialien, Lochinjektionsmaterialien, Elektronenblockiermaterialien und Lochblockiermaterialien.

16. Formulierung umfassend mindestens eine Verbindung gemäß einem oder mehreren der Ansprüche 1 bis 13, ein Oligomer, Polymer oder Dendrimer gemäß Anspruch 14 und/oder mindestens eine Zusammensetzung gemäß Anspruch 15 sowie mindestens ein Lösungsmittel.

17. Verfahren zur Herstellung einer Verbindung gemäß einem oder mehreren der Ansprüche 1 bis 13 oder eines Oligomers, Polymers oder Dendrimers gemäß Anspruch 14, **dadurch gekennzeichnet, dass** in einer Kupplungsreaktion eine Verbindung, die mindestens eine Elektronentransportgruppe umfasst, mit einer Verbindung umgesetzt wird, die mindestens ein Fluoren- oder Spirobifluoren-Radikal umfasst.

18. Verwendung einer Verbindung gemäß einem oder mehreren der Ansprüche 1 bis 13, eines Oligomers, Polymers oder Dendrimers gemäß Anspruch 14 oder einer Zusammensetzung gemäß Anspruch 15 in einer elektronischen Vorrichtung als Lochblockiermaterial, Elektroneninjektionsmaterial und/oder Elektronentransportmaterial.

19. Elektronische Vorrichtung umfassend mindestens eine Verbindung gemäß einem oder mehreren der Ansprüche 1 bis 13, ein Oligomer, Polymer oder Dendrimer gemäß Anspruch 14 oder eine Zusammensetzung gemäß Anspruch 15, wobei die elektronische Vorrichtung bevorzugt aus der Gruppe bestehend aus organischen elektrolumineszierenden Vorrichtungen, organischen integrierten Schaltungen, organischen Feldeffekttransistoren, organischen Dünnschichttransistoren, organischen lichtemittierenden Transistoren, organischen Solarzellen, organischen optischen Detektoren, organischen Photorezeptoren, organischen Feld-Quench-Vorrichtungen, lichtemittierenden elektrochemischen Zellen und organischen Laser-Dioden ausgewählt ist.

20. Elektronische Vorrichtung gemäß Anspruch 19, **dadurch gekennzeichnet, dass** die Vorrichtung mindestens eine Lochblockierschicht und mindestens eine Elektronentransportschicht umfasst, wobei die Lochblockierschicht direkt an eine Emitterschicht angrenzt und die mindestens eine Lochblockierschicht eine Verbindung gemäß einem oder mehreren der Ansprüche 1 bis 13 und/oder ein Oligomer, Polymer oder Dendrimer gemäß Anspruch 14 umfasst und die Elektronentransportschicht mindestens eine Elektronentransportverbindung umfassend, die sich von einer Verbindung gemäß einem oder mehreren der Ansprüche 1 bis 13 und/oder einem Oligomer, Polymer oder Dendrimer gemäß Anspruch 14 unterscheidet.

## Revendications

1. Composé comprenant des structures de la formule (A), de préférence composé selon la formule (A), 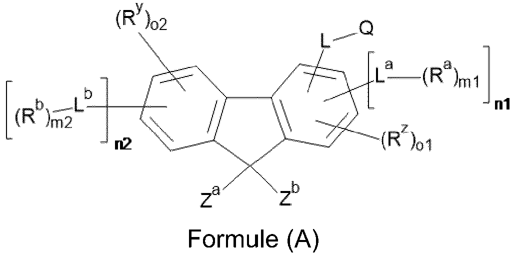 dans lesquels les symboles utilisés sont les suivants :
Z^{a} est Ar^{a} ou R^{x}, dans lequel Ar^{a} est un système cyclique aromatique ou hétéroaromatique qui comporte de 5 à 40 atomes de cycle aromatique, chacun pouvant être substitué par un ou plusieurs radicaux R^{x}, dans lequel les groupes Z^{a} et Z^{b} peuvent former ensemble un système cyclique aliphatique, hétéroaliphatique, aromatique ou hétéroaromatique mono- ou polycyclique ;
Z^{b} est Ar^{b} ou R^{w}, dans lequel Ar^{b} est un système cyclique aromatique ou hétéroaromatique qui comporte de 5 à 40 atomes de cycle aromatique, chacun pouvant être substitué par un ou plusieurs radicaux R^{w}, dans lequel les groupes Z^{a} et Z^{b} peuvent former ensemble un système cyclique aliphatique, hétéroaliphatique, aromatique ou hétéroaromatique mono- ou polycyclique ;
Q est un groupe de transport d'électrons ;
L, L^{a}, L^{b} est identique ou différent à chaque occurrence et est une liaison simple, C(=O) ou un système cyclique aromatique ou hétéroaromatique comportant de 5 à 24 atomes de cycle aromatique, pouvant être substitué par un ou plusieurs radicaux R ;
R, R^{w}, R^{x}, R^{y}, R^{z} est identique ou différent à chaque occurrence et est H, D, F, CI, Br, I, CN, Si(R¹)₃, un groupe alkyle, alkoxy ou thioalkyle linéaire comportant de 1 à 40 atomes de C ou un groupe alkyle, alkoxy ou thioalkyle ramifié ou cyclique comportant de 3 à 40 atomes de C, chacun pouvant être substitué par un ou plusieurs radicaux R¹, où, dans chaque cas, un ou plusieurs groupes CH₂ non adjacents peuvent être remplacés par -R¹C=CR¹-, -C≡C-, Si(R¹)₂, C=O, C=S, C=NR¹, -C(=O)O-, -C(=O)NR¹-, NR¹, P(=O)(R¹), -O-, -S-, SO ou SO₂ et où un ou plusieurs atomes H peuvent être remplacés par D, F, CI, Br, I, CN ou NO₂, ou un système cyclique aromatique ou hétéroaromatique comportant de 5 à 40 atomes de cycle aromatique, pouvant, dans chaque cas, être substitué par un ou plusieurs radicaux R¹, ou un groupe aryloxy ou hétéroaryloxy comportant de 5 à 40 atomes de cycle aromatique, pouvant être substitué par un ou plusieurs radicaux R¹, ou un groupe aralkyle comportant de 5 à 40 atomes de cycle aromatique, pouvant, dans chaque cas, être substitué par un ou plusieurs radicaux R¹, ou une combinaison de ces systèmes ; en même temps, deux ou plusieurs substituants adjacents R, R^{w}, R^{x}, R^{y}, R^{z} peuvent également former ensemble un système cyclique, de préférence un système cyclique aliphatique ou aromatique mono- ou polycyclique, pouvant être substitué par un ou plusieurs radicaux R¹ ;
R¹ est identique ou différent à chaque occurrence et est H, D, un groupe alkyle, alkoxy ou thioalkoxy linéaire comportant de 1 à 40 atomes de carbone ou un groupe alkyle, alkoxy ou thioalkoxy ramifié ou cyclique comportant de 3 à 40 atomes de carbone, chacun pouvant être substitué par un ou plusieurs radicaux R², où un ou plusieurs groupes CH₂ non adjacents peuvent être remplacés par -R²C=CR²-, -C≡C-, Si(R²)₂, C=O, C=S, C=NR², - C(=O)O-, -C(=O)NR²-, NR², P(=O)(R²), -O-, -S-, SO ou SO₂ et où un ou plusieurs atomes d'hydrogène peuvent être remplacés par D, F, CI, Br, I, CN ou NO₂, ou un système cyclique aromatique ou hétéroaromatique qui comporte de 5 à 40 atomes de cycle aromatique, chacun pouvant être substitué par un ou plusieurs radicaux R², ou un groupe aryloxy ou hétéroaryloxy qui comporte de 5 à 40 atomes de cycle aromatique, chacun pouvant être substitué par un ou plusieurs radicaux R², ou une combinaison de ces systèmes ; en même temps, deux ou plusieurs substituants R¹ adjacents peuvent également former ensemble un système cyclique, de préférence un système cyclique aliphatique ou aromatique mono- ou polycyclique ;
R² est identique ou différent à chaque occurrence et est H, D, F ou un radical hydrocarbylé aliphatique comportant de 1 à 20 atomes de carbone, dans lequel un ou plusieurs atomes d'hydrogène peuvent être remplacés par D ou F, ou un système cyclique aromatique ou hétéroaromatique comportant de 5 à 30 atomes de carbone, dans lequel un ou plusieurs atomes d'hydrogène peuvent être remplacés par D ou F ; en même temps, deux ou plusieurs substituants R² adjacents ensemble peuvent également former un système cyclique, de préférence un système cyclique aliphatique ou aromatique mono- ou polycyclique ;
R^{a}, R^{b} est identique ou différent à chaque occurrence et choisi parmi (R^{a}-1) à (R^{a}-33) : 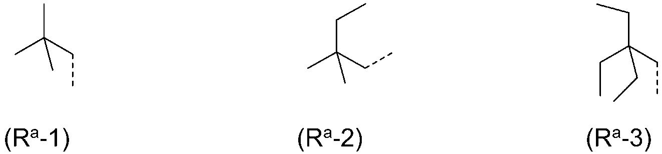 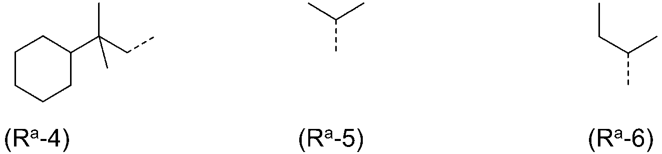 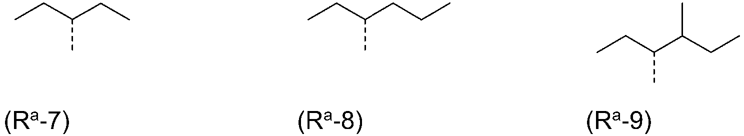 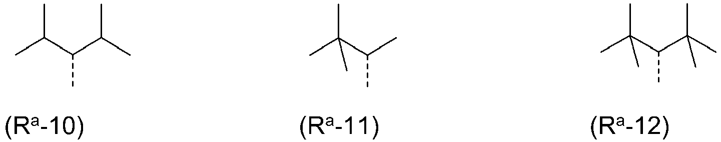 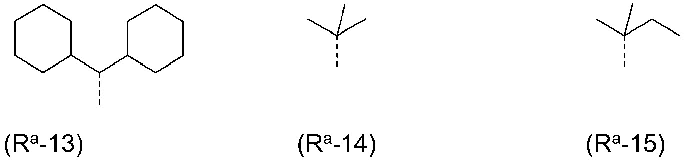 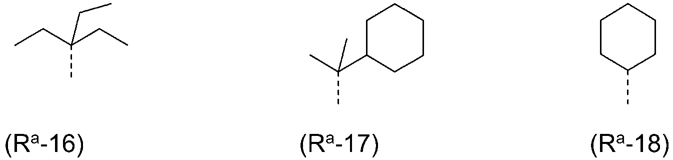 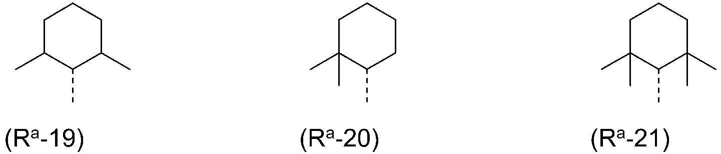 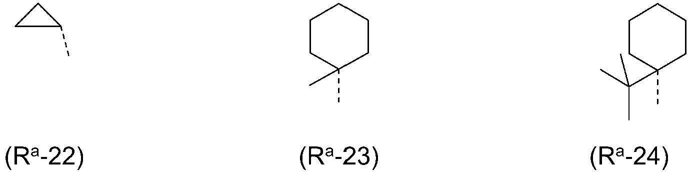 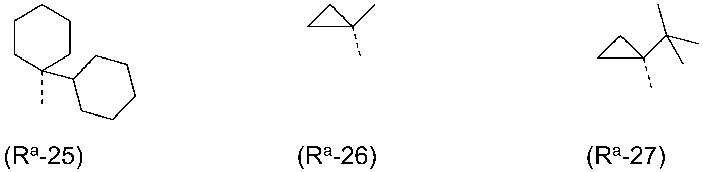 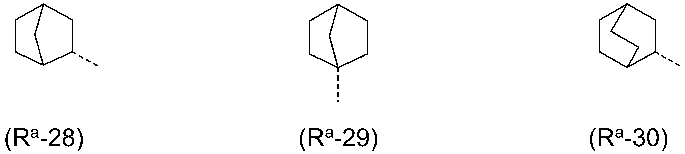 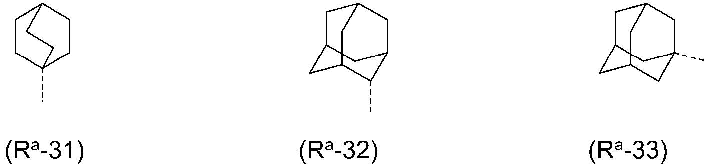 dans lequel un ou plusieurs atomes H peuvent être remplacés par D et la ligne pointillée indique la liaison avec le groupe correspondant
m1, m2 est identique ou différent à chaque occurrence et vaut 0, 1, 2, 3 ou 4, de préférence 1, 2, 3 ou 4, plus préférablement 1 ou 2 ;
n2, o2 est identique ou différent à chaque occurrence et vaut 0, 1, 2, 3 ou 4, de préférence 0, 1 ou 2 ;
n1, o1 est identique ou différent à chaque occurrence et vaut 0, 1, 2 ou 3, de préférence 0, 1 ou 2 ;
la somme de n1 + o1 vaut 0, 1, 2 ou 3, de préférence 0, 1 ou 2 ;
la somme de n2 + o2 vaut 0, 1, 2, 3 ou 4, de préférence 0, 1 ou 2 ;
et **caractérisé en ce que** le composé comprend deux, trois ou quatre éléments structuraux des formules (R^{a}-1) à (R^{a}-33).

2. Composé selon la revendication 1, **caractérisé en ce que** le composé comprend des structures de la formule (I) ou de la formule (II), de préférence composé selon la formule (I) ou la formule (II), 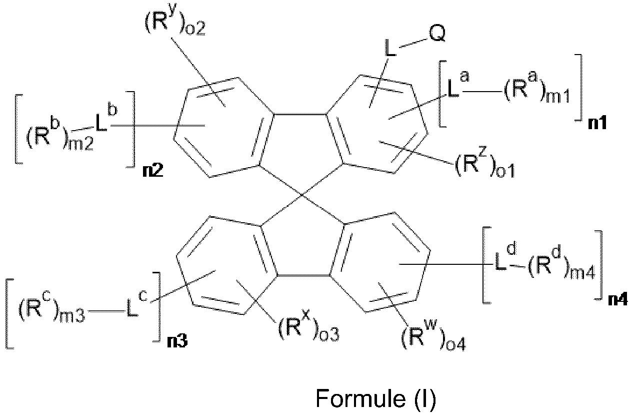 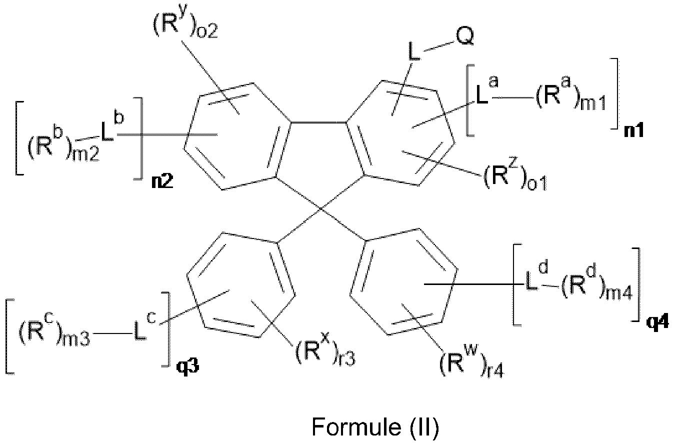
dans lesquelles les symboles Q, L, L^{a}, L^{b}, R, R^{w}, R^{x}, R^{y}, R^{z}, R^{a}, R^{b}, m1, m2, n1, n2, o1 et o2 ont la définition donnée dans la revendication 1 et
L^{c}, L^{d} est identique ou différent à chaque occurrence et est une liaison simple, C(=O) ou un système cyclique aromatique ou hétéroaromatique comportant de 5 à 24 atomes de cycle aromatique, pouvant être substitué par un ou plusieurs radicaux R ;
R^{c}, R^{d} est identique ou différent à chaque occurrence et choisi parmi (R^{a}-1) à (R^{a}-33) tels que donnés dans la revendication 1 ;
m3, m4 est identique ou différent à chaque occurrence et vaut 0, 1, 2, 3 ou 4, de préférence 1, 2, 3 ou 4, plus préférablement 1 ou 2 ;
n3, n4, o3, o4 est identique ou différent à chaque occurrence et vaut 0, 1, 2, 3 ou 4, de préférence 0, 1 ou 2 ;
q3, q4, r3, r4 est identique ou différent à chaque occurrence et vaut 0, 1, 2, 3, 4 ou 5, de préférence 0, 1 ou 2 ;
la somme de n3 + o3 vaut 0, 1, 2, 3 ou 4, de préférence 0, 1 ou 2 ;
la somme de n4 + o4 vaut 0, 1, 2, 3 ou 4, de préférence 0, 1 ou 2 ;
la somme de q3 + r3 vaut 0, 1, 2, 3, 4 ou 5, de préférence 0, 1 ou 2 ;
la somme de q4 + r4 vaut 0, 1, 2, 3, 4 ou 5, de préférence 0, 1 ou 2 ;
etdans lequel le composé comprend deux, trois ou quatre éléments structuraux des formules (R^{a}-1) à (R^{a}-33).

3. Composé selon la revendication 1 ou 2, **caractérisé en ce que** le composé comprend au moins une des structures des formules (la), (Ib), (Ic), (Id), (IIa), (IIb), (IIc) et/ou (IId), de préférence composé selon les formules (la), (Ib), (Ic), (Id), (IIa), (IIb), (IIc) et/ou (IId), 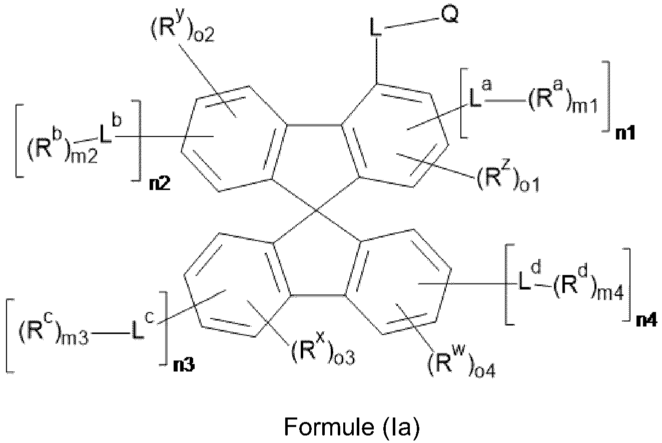 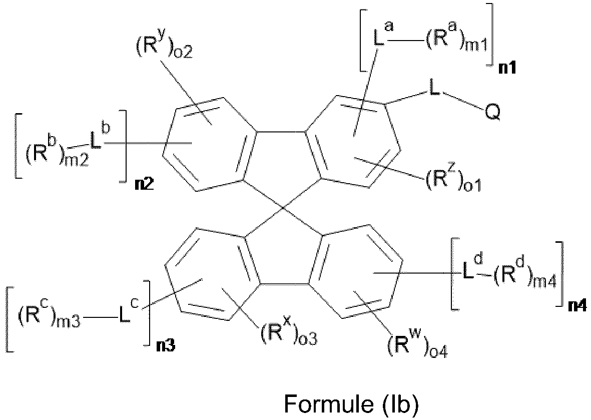 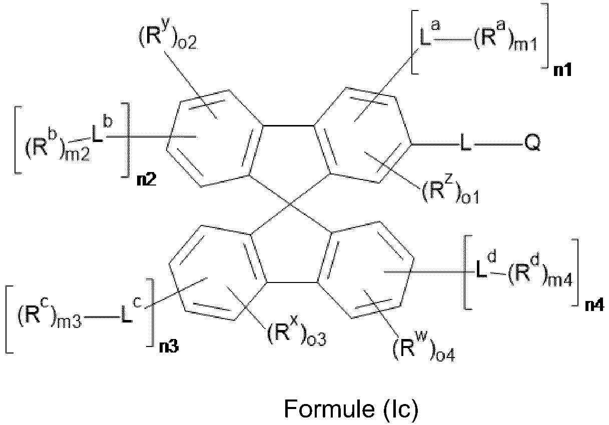 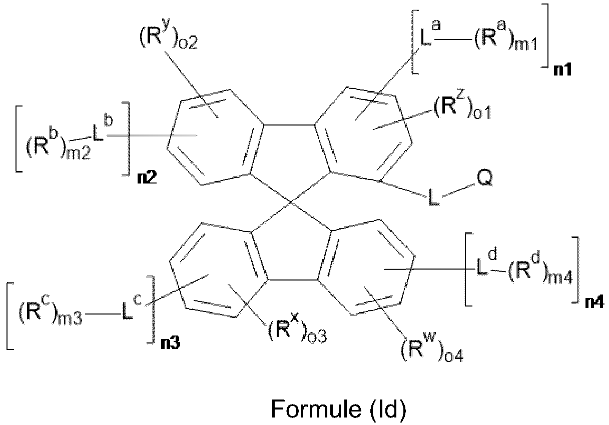 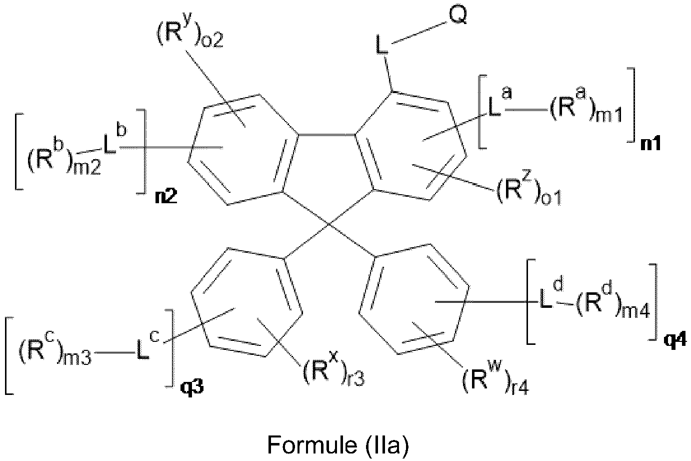 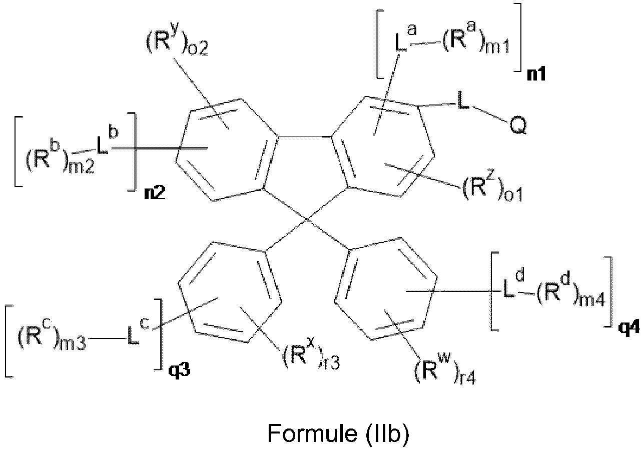 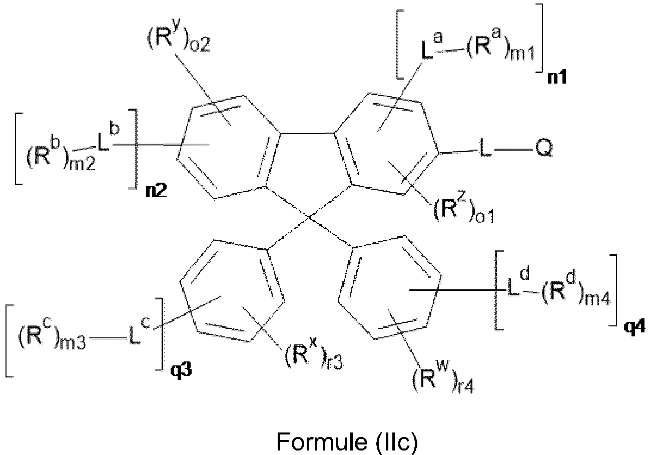 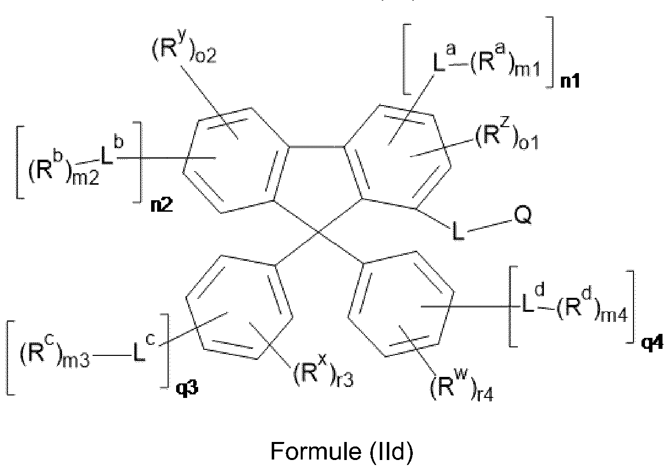 dans lesquelles les symboles Q, L, L^{a}, L^{b}, L^{c}, L^{d}, R^{w}, R^{x}, R^{y}, R^{z}, R^{a}, R^{b}, R^{c}, R^{d}, m1, m2, m3, m4, n1, n2, n3, n4, o1, o2, o3, o4, q3, q4, r3 et r4 ont la définition énoncée dans la revendication 1 ou 2, dans lesquelles les structures (la) et (Ic) sont préférées.

4. Composé selon une ou plusieurs des revendications 2 ou 3, **caractérisé en ce que** la somme des indices m1, m2, m3 et m4 est dans l'intervalle de 1 à 6, de préférence 1 à 4, plus préférablement 2 ou 3.

5. Composé selon une ou plusieurs des revendications 1 à 4, **caractérisé en ce que** le composé comprend au moins une des structures des formules (I-1), (I-2), (I-3), (I-4), (I-5), (I-6), (II-1), (II-2), (II-3), (II-4), (II-5) et/ou (II-6), de préférence composé selon les formules (I-1), (I-2), (I-3), (I-4), (I-5), (I-6), (II-1), (II-2), (II-3), (II-4), (II-5) et/ou (II-6), 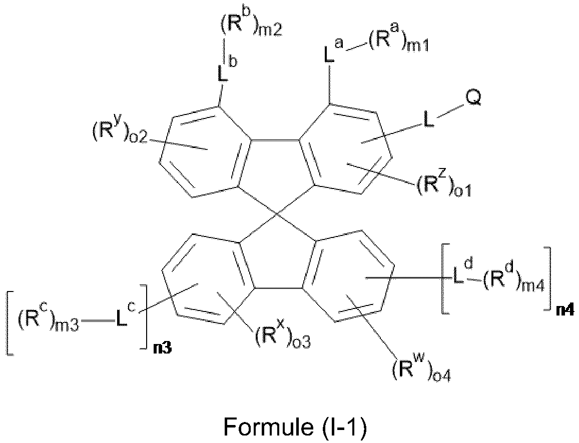 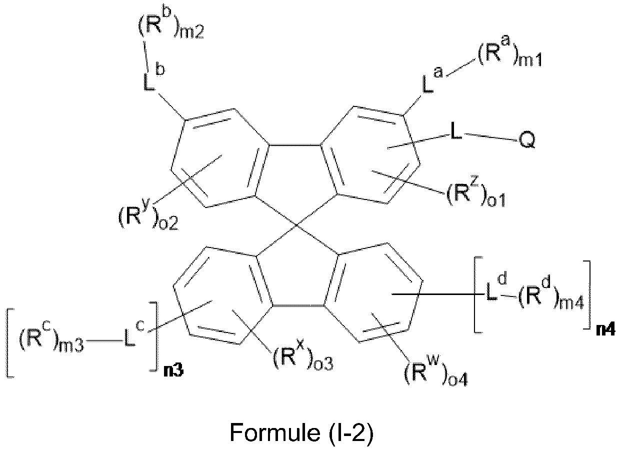 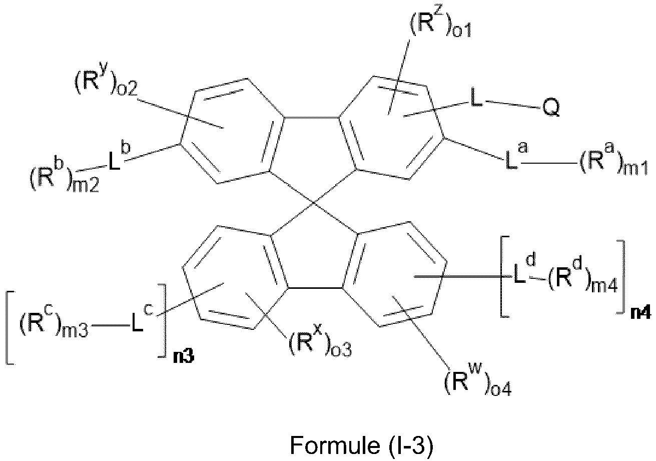 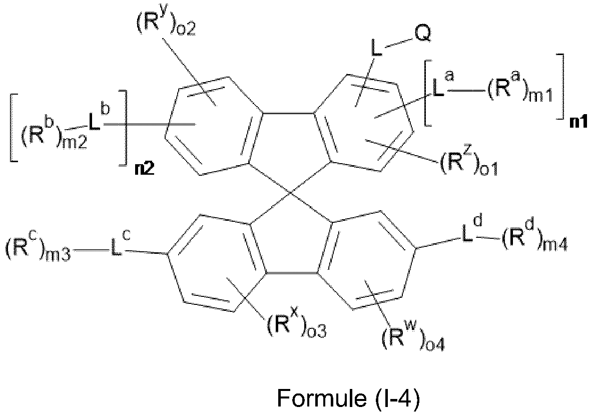 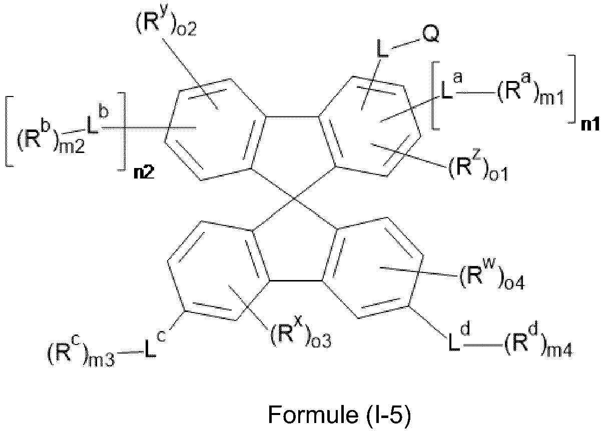 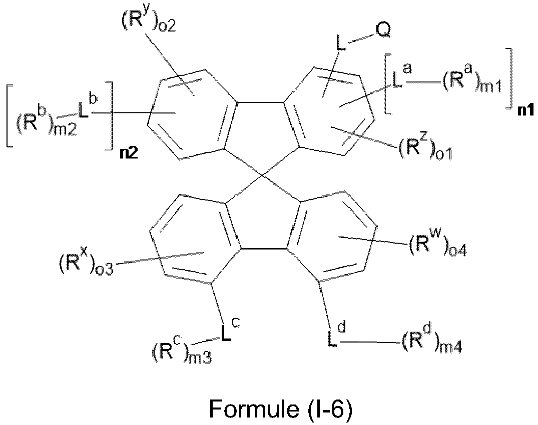 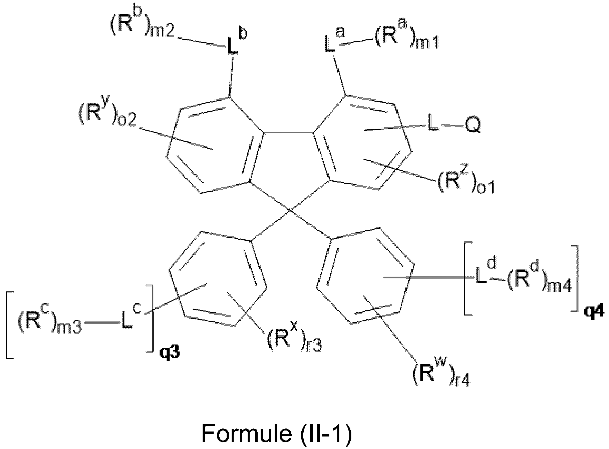 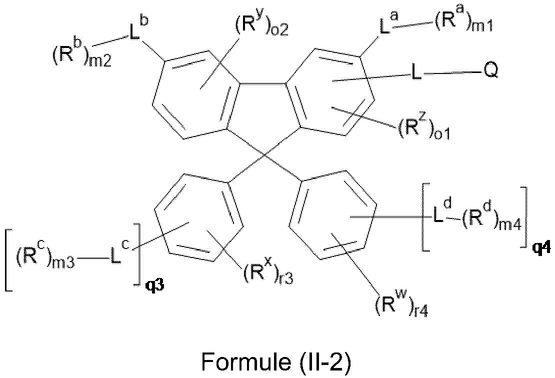 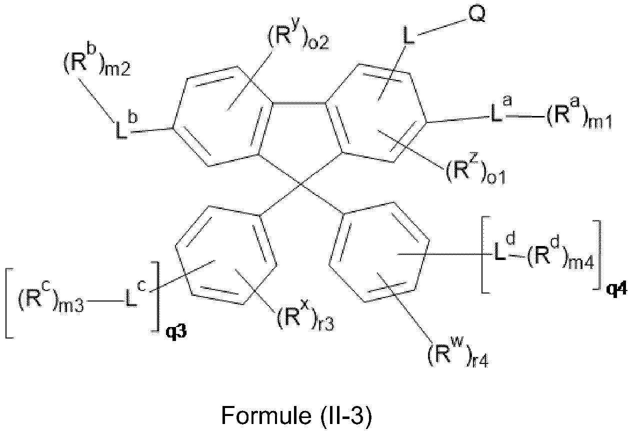 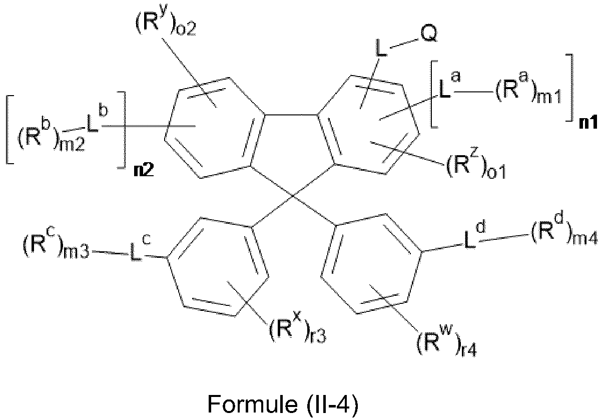 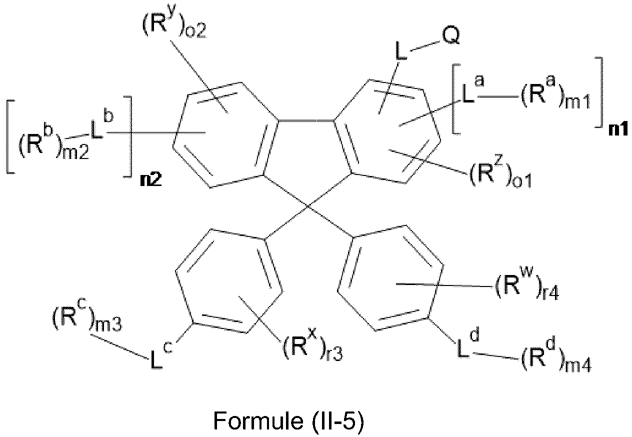 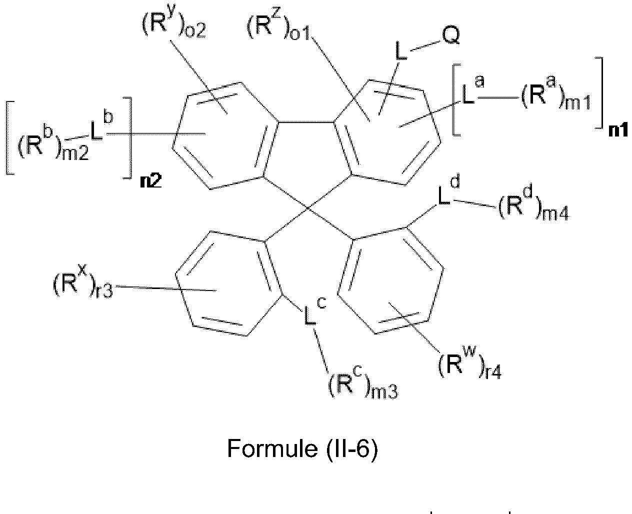 dans lesquelles les symboles Q, L, L^{a}, L^{b}, L^{c}, L^{a}, R^{w}, R^{x}, R^{y}, R^{z}, R^{a}, R^{b}, R^{c}, R^{d}, m1, m2, m3, m4, n1, n2, n3, n4, o1, o2, o3, o4, q3, q4, r3 et r4 ont la définition énoncée dans la revendication 1 ou 2, dans lesquelles les structures (I-3) et (I-4) sont préférées.

6. Composé selon une ou plusieurs des revendications 1 à 5, **caractérisé en ce que** le composé comprend au moins une des structures des formules (Ia-3), (Ia-4), (Ib-4), (Ic-4) et/ou (Id-4), de préférence composé selon les formules (la-3), (la-4), (Ib-4), (Ic-4) et/ou (Id-4), 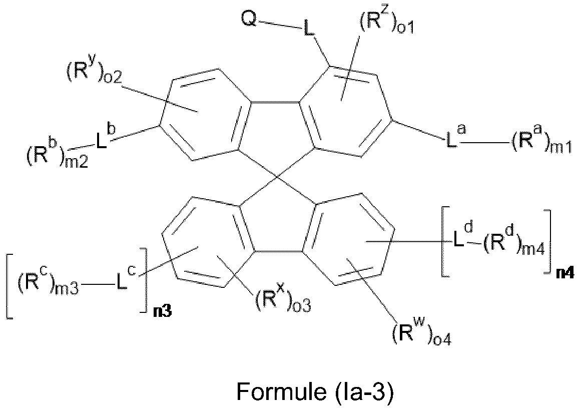 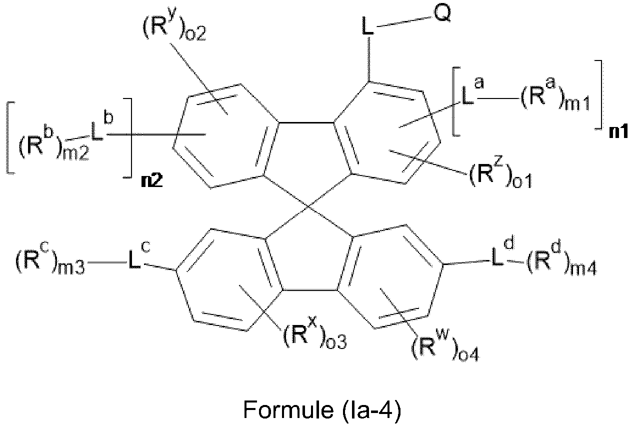 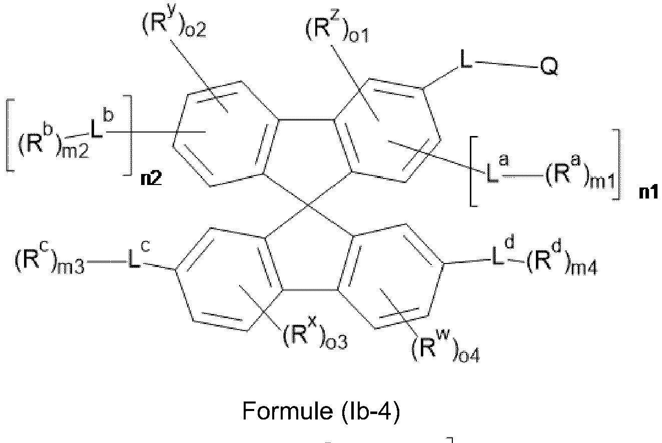 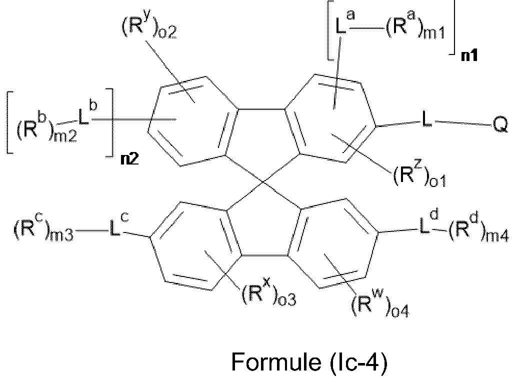 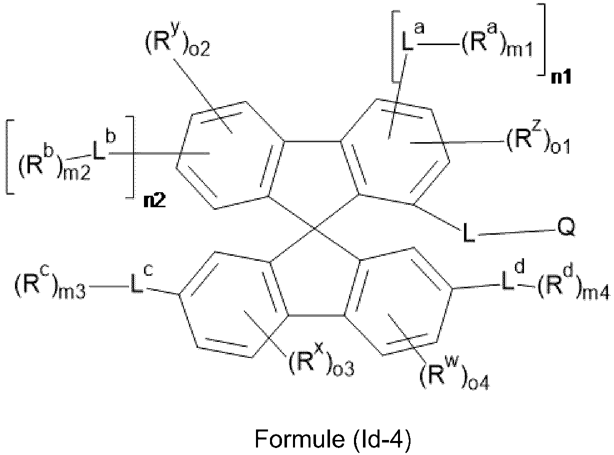 dans lesquelles les symboles Q, L, L^{a}, L^{b}, L^{c}, L ^{d}, R^{w}, R^{x}, R^{y}, R^{z}, R^{a}, R^{b}, R^{c}, R^{d}, m1, m2, m3, m4, n1, n2, n3, n4, o1, o2, o3 et o4 ont la définition énoncée dans la revendication 1 ou 2.

7. Composé selon une ou plusieurs des revendications 1 à 6, **caractérisé en ce que** le groupe Q est un groupe pyridine, un groupe pyrimidine, ou un groupe triazine, plus préférablement un groupe triazine, pouvant être substitué par un ou plusieurs radicaux R.

8. Composé selon la revendication 7, **caractérisé en ce que** le groupe Q est choisi parmi les structures des formules (Q-1), (Q-2), (Q-3), (Q-4), (Q-5), (Q-6), (Q-7) et/ou (Q-8) 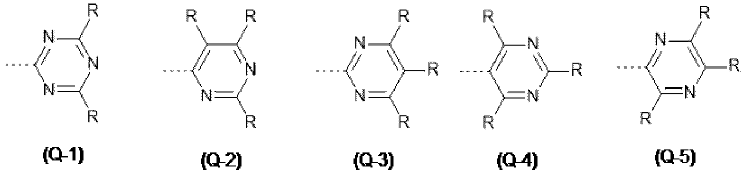 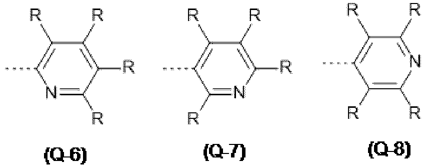 dans lequel le symbole R a la définition donnée ci-dessus dans la revendication 1, la liaison pointillée marque la position de fixation, dans lesquelles les structures (Q-1) à (Q-5) sont préférées et la structure (Q-1) est particulièrement préférée.

9. Composé selon la revendication 8, **caractérisé en ce que** le groupe Q est choisi parmi les structures des formules (Q-1a), (Q-1b), (Q-1c), (Q-1d), (Q-1e), (Q-1f), (Q-1g), (Q-1h), (Q-1i) et/ou (Q-1j) 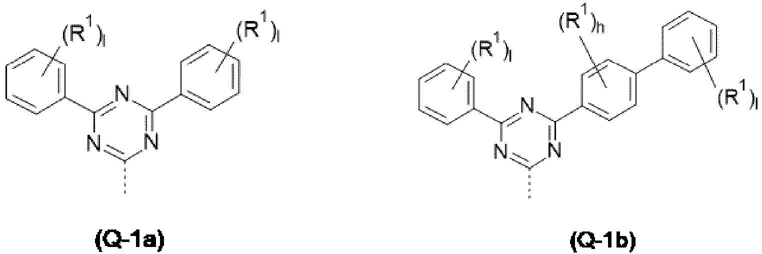 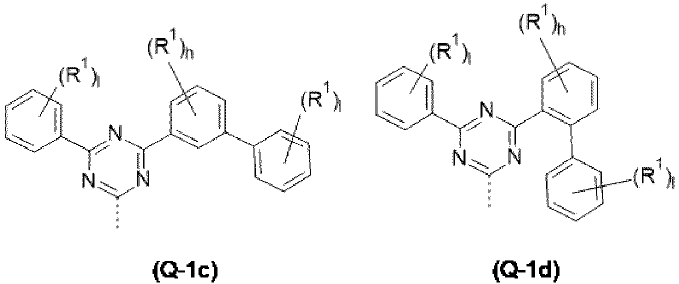 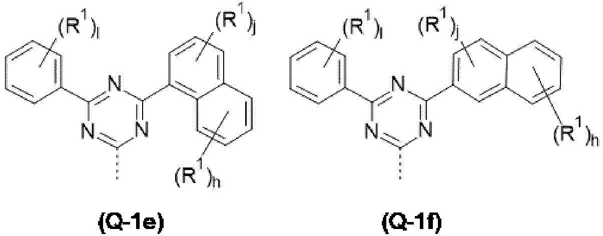 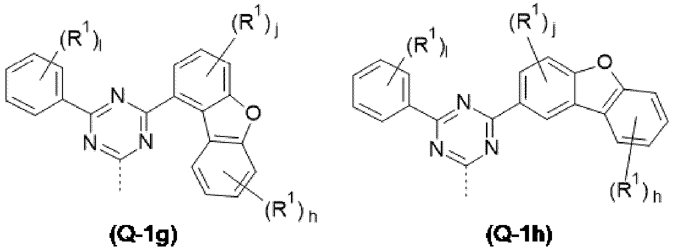 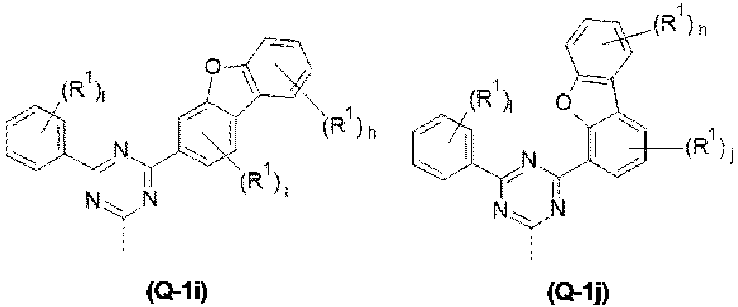 dans lequel le symbole R¹ a la définition donnée ci-dessus dans la revendication 1, la liaison pointillée marque la position de fixation et l'indice I est identique ou différent à chaque occurrence et vaut 0, 1, 2, 3, 4 ou 5, de préférence 0, 1, 2 ou 3, plus préférablement 0 ou 1, l'indice h est identique ou différent à chaque occurrence et vaut 0, 1, 2, 3 ou 4, de préférence 0, 1 ou 2, l'indice j est identique ou différent à chaque occurrence et vaut 0, 1, 2 ou 3, de préférence 0, 1 ou 2 ; dans lesquelles la structure (Q-1a) est préférée.

10. Composé selon une ou plusieurs des revendications 1 à 9, **caractérisé en ce que** le symbole L, L^{a}, L^{b}, L^{c}, L^{d}, est une liaison ou un groupe aryle comportant de 6 à 12 atomes de cycle aromatique, pouvant être substitué par un ou plusieurs radicaux R, de préférence un groupe phénylène, un groupe biphénylène ou un groupe naphtylène, plus préférablement un groupe phénylène ou un groupe naphtylène, pouvant être substitué par un ou plusieurs radicaux R.

11. Composé selon la revendication 10, **caractérisé en ce que** le symbole L, L^{a}, L^{b}, L^{c}, L^{d}, est une liaison ou est choisi parmi les structures des formules (L-1), (L-5), (L-6) et/ou (L-7) 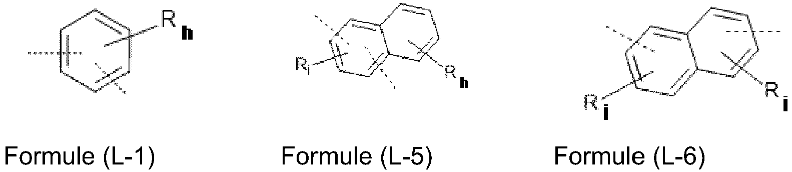 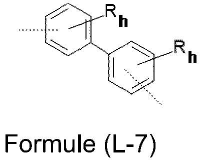
dans lequel le symbole R a la définition donnée ci-dessus dans la revendication 1, la liaison pointillée marque les positions de fixation, l'indice h est identique ou différent à chaque occurrence et vaut 0, 1, 2, 3 ou 4, de préférence 0, 1 ou 2, l'indice j est identique ou différent à chaque occurrence et vaut 0, 1, 2 ou 3, de préférence 0, 1 ou 2 ;
l'indice i est identique ou différent à chaque occurrence et vaut 0, 1 ou 2, de préférence 0 ou 1 ; dans lesquelles la structure (L-1) est préférée.

12. Composé selon une ou plusieurs des revendications 1 à 11, **caractérisé en ce que** le composé comprend au moins une des structures des formules (la-3a), (la-4a), (Ib-4a) et/ou (Ic-4a), de préférence composé selon les formules (la-3a), (la-4a), (Ib-4a) et/ou (Ic-4a), 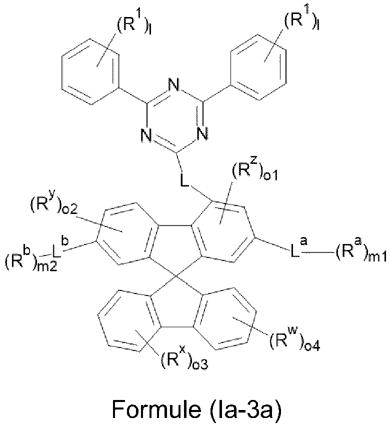 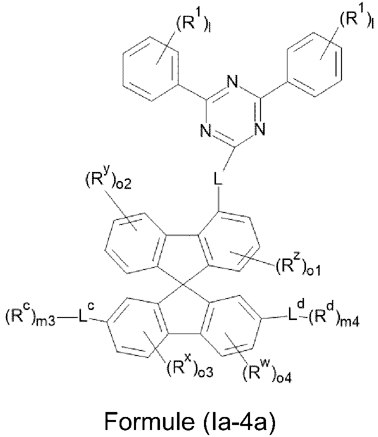 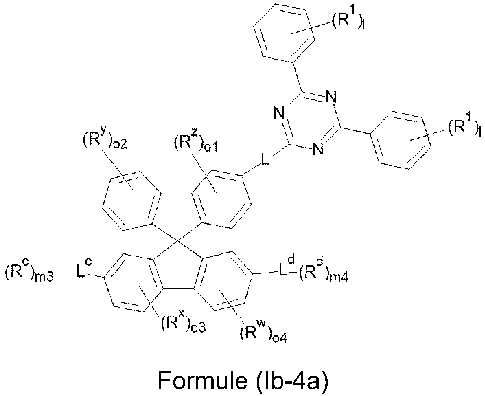 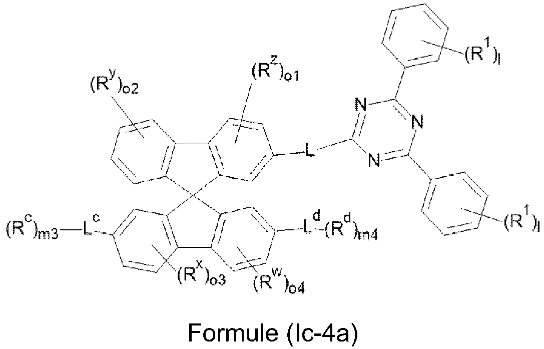 dans lesquelles les symboles L, L^{a}, L^{b}, L^{c}, L^{d}, R¹, R^{w}, R^{x}, R^{y}, R^{z}, R^{a}, R^{b}, R^{c}, R^{d}, l, m1, m2, m3, m4, o1, o2, o3 et o4 ont la définition énoncée dans la revendication 1, 2 ou 13.

13. Composé selon une ou plusieurs des revendications 1 à 12, **caractérisé en ce que** le composé comprend exactement un groupe triazine, plus préférablement exactement un groupe de transport d'électrons.

14. Oligomère, polymère ou dendrimère contenant un ou plusieurs composés selon une ou plusieurs des revendications 1 à 13, dans lequel une ou plusieurs liaisons du composé au polymère, oligomère ou dendrimère sont présentes.

15. Composition comprenant au moins un composé selon une ou plusieurs des revendications 1 à 13 et/ou un oligomère, polymère ou dendrimère selon la revendication 14 et au moins un autre composé choisi dans le groupe constitué d'émetteurs fluorescents, d'émetteurs phosphorescents, d'émetteurs TADF, de matériaux hôtes, de matériaux de matrice, de matériaux de transport d'électrons, de matériaux d'injection d'électrons, de matériaux conducteurs de trous, de matériaux d'injection de trous, de matériaux de blocage d'électrons et de matériaux de blocage de trous.

16. Formulation comprenant au moins un composé selon une ou plusieurs des revendications 1 à 13, un oligomère, polymère ou dendrimère selon la revendication 14 et/ou au moins une composition selon la revendication 15 et au moins un solvant.

17. Procédé de préparation d'un composé selon une ou plusieurs des revendications 1 à 13 ou d'un oligomère, polymère ou dendrimère selon la revendication 14, **caractérisé en ce que**, dans une réaction de couplage, un composé comprenant au moins un groupe transporteur d'électrons est mis à réagir avec un composé comprenant au moins un radical fluorène ou spirobifluorène.

18. Utilisation d'un composé selon une ou plusieurs des revendications 1 à 13, d'un oligomère, polymère ou dendrimère selon la revendication 14 ou d'une composition selon la revendication 15 dans un dispositif électronique en tant que matériau de blocage de trous, matériau d'injection d'électrons et/ou matériau de transport d'électrons.

19. Dispositif électronique comprenant au moins un composé selon une ou plusieurs des revendications 1 à 13, un oligomère, polymère ou dendrimère selon la revendication 14 ou une composition selon la revendication 15, dans lequel le dispositif électronique est de préférence choisi dans le groupe constitué de dispositifs électroluminescents organiques, circuits intégrés organiques, transistors à effet de champ organiques, transistors organiques à couche mince, transistors émetteurs de lumière organiques, cellules solaires organiques, détecteurs optiques organiques, photorécepteurs organiques, dispositifs organiques d'extinction de champ, cellules électrochimiques émettrices de lumière et diodes laser organiques.

20. Dispositif électronique selon la revendication 19, **caractérisé en ce que** le dispositif comprend au moins une couche de blocage de trous et au moins une couche de transport d'électrons, dans lequel la couche de blocage de trous est directement adjacente à une couche émissive et la au moins une couche de blocage de trous comprend un composé selon une ou plusieurs des revendications 1 à 13 et/ou un oligomère, polymère ou dendrimère selon la revendication 14 et la couche de transport d'électrons comprend au moins un composé de transport d'électrons différent d'un composé selon une ou plusieurs des revendications 1 à 13 et/ou d'un oligomère, polymère ou dendrimère selon la revendication 14.
